# EUROPÄISCHE PATENTSCHRIFT

(11) **EP 1 846 402 B1**
(45) Veröffentlichungstag und Bekanntmachung des Hinweises auf die Patenterteilung: **26.02.2014**
(21) Anmeldenummer: 05821901.5
(22) Anmeldetag: 12.12.2005
(51) Int. Cl.: C07D 471/04, A61K 31/437, A61P 35/00, A61P 9/10, A61P 25/28

(54) **3-AMINO-PYRAZOLO[3,4B]PYRIDINE ALS INHIBITOREN VON PROTEINTYROSINKINASEN ZUR BEHANDLUNG VON ANGIOGENEN, HYPERPROLIFERATIVEN ODER NEURODEGENERATIVEN ERKRANKUNGEN**
3-AMINO-PYRAZOLO[3,4B]PYRIDINES USED AS INHIBITORS OF PROTEIN TYROSINE KINASES FOR TREATING ANGIOGENIC, HYPERPROLIFERATIVE OR NEURODEGENERATIVE DISEASES
3-AMINO-PYRAZOLO[3,4B]PYRIDINES EN TANT QU'INHIBITEURS DE PROTEINES TYROSINE KINASES POUR TRAITER DES MALADIES ANGIOGENES, HYPERPROLIFERATIVES OU NEURODEGENERATIVES

(30) Priorität: 14.12.2004 DE 102004061288; 17.12.2004 US 636690 P
(43) Veröffentlichungstag der Anmeldung: 24.10.2007
(73) Patentinhaber: Bayer Intellectual Property GmbH, 40789 Monheim (DE)
(72) Erfinder: SCHWEDE, Wolfgang, 16548 Berlin (DE); KÜNZER, Hermann, 13348 Berlin (DE); TER LAAK, Antonius, 12159 Berlin (DE); BADER, Benjamin, 13467 Berlin (DE); HILLIG, Roman, 10825 Berlin (DE); MÖNNING, Ursula, 15569 Woltersdorf (DE); SCHMITZ, Arndt, 12167 Berlin (DE); ZOPF, Dieter, 14167 Berlin (DE)
(74) Vertreter: BIP Patents
(86) Internationale Anmeldenummer: PCT/EP2005/013417
(87) Internationale Veröffentlichungsnummer: WO 2006/063805

(56) Entgegenhaltungen:
- EP-A- 1 308 441
- WO-A-01/19828
- DE-A1- 2 238 400
- DE-A1- 2 643 753
- DE-A1- 3 001 498
- US-A- 6 046 216
- US-A1- 2004 014 784
- DATABASE REGISTRY 10. Juli 2002 (2002-07-10), XP002373925 Database accession no. RN: 438020-37-2
- MURATA ET AL: "Discovery of novel and selective IKK-? serine-threonine protein kinase inhibitors. Part 1" BIOORGANIC & MEDICINAL CHEMISTRY LETTERS, Bd. 13, Nr. 5, 2003, Seiten 913-918, XP002373853
- DATABASE CHEMCATS 18. Januar 2005 (2005-01-18), INTERCHIM INTERMEDIATES: XP002373926 Database accession no. 2005:1815941 CHEMCATS

## Beschreibung

Die Erfindung betrifft Verbindungen der allgemeinen Formel (I), Herstellung und Verwendung als Inhibitoren von Proteintyrosinkinasen, insbesondere Eph (*e*rythropoetin-*p*roducing *h*epatoma amplified sequence) -Rezeptoren zur Behandlung verschiedener Erkrankungen.

Proteintyrosinkinasen katalysieren die Phosphorylierung spezifischer Tyrosinreste in verschiedenen Proteinen. Solche Phosphorylierungsreaktionen spielen bei einer Vielzahl von zellulären Prozessen eine Rolle, die in der Regulation des Wachstums und der Differenzierung von Zellen involviert sind. Proteintyrosinkinasen werden in Rezeptor- und Nicht-Rezeptortyrosinkinasen eingeteilt. Die Familie der Rezeptortyrosinkinasen (RTKs) besteht aus 58 Kinasen (Manning G. et al. 2002, Science 298, 1912-1934). RTKs besitzen eine extrazelluläre Ligandenbindungsdomäne, eine Transmembrandomäne und eine intrazelluläre Domäne, die in der Regel die Tyrosinkinaseaktivität enthält. RTKs vermitteln die Signalweiterleitung von extrazellulären Stimulatoren wie z.B. Wachstumsfaktoren. Die Ligandenbindung führt zur Dimerisierung der RTKs und der wechselseitigen Autophosphorylierung ihrer intrazellulären Domänen. In Abhängigkeit vom Zelltyp werden dadurch spezifische intrazelluläre Bindungsproteine rekrutiert (u.a. Nicht-Rezeptortyrosinkinasen), über die eine Signalverarbeitung in der Zelle erfolgt (Schlessinger J. 2000, Cell 103, 211-225). Zu diesen zählen Rezeptorfamilien von Wachstumsfaktoren wie EGF (epidermal growth factor), VEGF (vascular endothelial growth factor), FGF (fibroblast growth factor), PDGF (platelet derived growth factor) und NGF (nerve growth factor), sowie der Insulinrezeptoren und die große Familie der Ephrinrezeptoren und andere.

Die größte Familie innerhalb der RTKs nehmen die Ephrin (Eph)-Rezeptoren ein. Sie teilen sich entsprechend ihrer sequenziellen Verwandtschaft und ihrer Ligandenspezifität in die Gruppe der EphA-Rezeptoren (9 Mitglieder) und der EphB-Rezeptoren (6 Mitglieder) auf (Kullander K. and Klein R. 2002, Nat.Rev.Mol.Cell Biol. 3, 475-486; Cheng N. et al. 2002, Cyt. and growth factor Rev. 13, 75-85.). Eph-Rezeptoren werden von membranständigen Liganden der EphrinA- bzw. EphrinB-Familie aktiviert. EphrinAs sind über Glykolipide (GPI) in der Zellmembran verankert, während EphrinBs eine Transmembranregion und eine intrazelluläre Domäne besitzen. Die Interaktion zwischen Ephrinen und den Eph-Rezeptoren führt zu einer bidirektionellen Signalübertragung in den ephrinexprimierenden und in den Eph-Rezeptor tragenden Zellen. Ephrine und Eph-Rezeptoren spielen in einer Vielzahl von morphogenetischen Prozessen in der Embryonalentwicklung und im adulten Organismus eine Rolle. Sie sind involviert in der embryonalen Musterbildung, in der Entwicklung des Blutgefäßsystems (Gerety S.S: et al 1999, Mol. Cell 4, 403-414) und in der Etablierung von neuronalen Verschaltungen (Flanagan, J.G. and Vanderhaeghen, P., 1998, Annu.Rev.Neurosci. 21, 306-354). Im adulten Organismus sind sie bei Neovascularisierungsprozesses z.B. bei der Tumorentstehung und bei der Endometriose, sowie bei der Morphogenese des Darmepithels beteiligt (Batlle E. et al. 2002, Cell 111:251-63). Auf zellulärer Ebene vermitteln sie Migration, Adhäsion und juxtacrine Zellkontakte. Erhöhte Expression von Eph-Rezeptoren, wie z.B. EphB2 und EphB4 wurde auch in verschiedenen Tumorgeweben, wie z.B. Brust- und Darmtumoren beobachtet (Nakamoto M. and Bergemann A.D. 2002, Mic.Res.Tech. 59, 58-67). Knock-out Mäuse von EphB2, EphB3 und EphB4 zeigen Defekte bei der Ausbildung des Blutgefäßsystems. Die embryonale Lethalität der EphB4 -/-Mäuse im Embryonalstadium d14, zeigt die besondere Rolle von EphB4 in diesem Prozeß (Gerety S.S: et al 1999, Mol. Cell 4, 403-414). Eine Modulation dieser Rezeptoren z.B. durch die Inhibition ihrer Kinaseaktivität führt beispielsweise dazu, dass das Tumorwachstum und/oder die Tumormetastasierung entweder durch eine direkte antitumorale oder durch eine indirekte antiangiogene Wirkung unterbunden wird.

Nicht-Rezeptortyrosinkinasen kommen in löslicher Form intrazellulär vor und sind an der Verarbeitung von extrazellulären Signalen (z.B. von Wachstumsfaktoren, Cytokinen, Antikörpern, Adhäsionsmolekülen) innerhalb der Zelle beteiligt. Zu ihnen zählen u.a. die Familien der src(sarcoma)-Kinasen, der Tec(tyrosine kinase expressed in hepatocellular carcinoma)-Kinasen, der Abl(Abelson)-kinasen und der Brk(breast tumor kinase)-Kinasen, sowie die fokale Adhesionskinase (FAK).

Eine veränderte Aktivität dieser Proteintyrosinkinasen kann zu verschiedensten physiologischen Störungen im menschlichen Organismus führen und dadurch z.B. entzündliche, neurologische und onkologische Erkankungen verursachen.

Pyrazolopyridine werden als antimikrobielle Substanzen beschrieben (z.B. Attaby et al, Phosphorus, Sulfur and Silicon and the related Elements (1999), 149, 49-64; ibid. (1999), 155, 253-270).

In US 5,478,830 werden Pyrazolopyridine offenbart.

Die DE 30 01 498 A1 (Agfa-Gevaert AG) offenbart Pyrazole die für fotografische Materialien verwendet werden können.

In der DE 26 43 753 A1 (Dr. Karl Thomae GmbH) werden Pyrazolopyridine beschrieben, die über eine antiphlogistische und insbesondere antithrombotische Wirkung verfügen. Die offenbarten Substituenten sind immer über ein Stickstoffatom mit dem Pyridin an der Position 6 verknüpft. F.E. Goda et al. 2004, Bioorganic & Medicinal Chemistry, 12(8), S. 1845-1852 offenbart Pyrazolopyridine, die über eine antimikrobielle Wirkung verfügen.

B. Narsaiah et al. 2001, Journal of Gluorine Chemistry, 109, 183-7 beschreibt verschiedene mit CF₃-substituierte Pyrazolopyridine.

Die WO 01/19828 A2 offenbart ein Pyrazolopyridin welches mit einer Diaminopyrazolyl- und einer Methylgruppe substituiert ist. Die in dieser Patentschrift beschriebenen Substanzen können als Haarfärbemittel verwendet werden.

In WO 01/19828 (BASF AG) werden Grundkörper für verschiedenste Kinaseinhibitoren offenbart. Die Beschreibung beinhaltet Pyrazolopyridine (Struktur 95, Seite 101). In den Beispielen sind jedoch keine Pyrazlopyridine offenbart und die Herstellung der darin beanspruchten Pyrazolopyridine ist nicht beschrieben.

Auf weiteren Stand der Technik wird an geeigneten Stellen im Text verwiesen.

Unter den Rezeptortyrosinkinaseinhibitoren sind jedoch keine Eph-Rezeptorinhibitoren beschrieben.

Es ist Aufgabe der vorliegenden Erfindung Verbindungen bereitzustellen, die Proteintyrosinkinasen, insbesondere Eph-Rezeptoren, inhibieren.

### Erste Ausführungsform der Erfindung:

In einer ersten Ausführungsform der vorliegenden Erfindung wurde nun gefunden, dass Verbindungen der allgemeinen Formel (I) 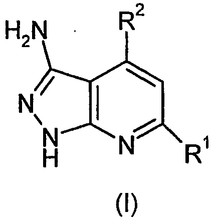 in der,
- R¹: für gegebenenfalls ein- oder mehrfach, gleich oder verschieden mit K substituiertes C₁-C₆-alkyl, C₂-C₆-alkenyl, C₂-C₆-alkinyl, C₃-C₁₀-Cycloalkyl, C₃-C₁₀-Heterocycloalkyl, Aryl oder Heteroaryl steht, wobei das C₃-C₁₀-Heterocycloalkyl selbst durch mindestens eins der folgenden Atome Stickstoff, Sauerstoff und/ oder Schwefel im Ring unterbrochen ist und C₃-C₁₀-Cycloalkyl und/ oder C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthalten sein können,
- K: für Halogen, Hydroxy oder die Gruppe -O-R³ steht oder für gegebenenfalls ein- oder mehrfach, gleich oder verschieden mit L substituiertes C₃-C₁₀-Cycloalkyl, C₃-C₁₀-Heterocycloalkyl, Aryl oder Heteroaryl steht, wobei das C₃-C₁₀-Heterocycloalkyl selbst durch mindestens eins der folgenden Atome Stickstoff, Sauerstoff und/ oder Schwefel im Ring unterbrochen ist und C₃-C₁₀-Cycloalkyl und / oder C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthalten sein können,
- L: für C₁-C₆-alkyl oder für die Gruppe -COR⁴ oder -NR⁵R⁶ steht,
- R²: für gegebenenfalls ein- oder mehrfach, gleich oder verschieden mit M substituiertes C₁-C₆-alkyl, Aryl oder Heteroaryl steht,
- R³: für gegebenenfalls ein- oder mehrfach, gleich oder verschieden mit der Gruppe-NR⁵R⁶ substituiertes C₁-C₆-alkyl, Aryl oder -(CH₂)ₙ-Aryl steht,
- R⁴: für Wasserstoff, Hydroxy, C₁-C₆-alkyl oder C₁-C₆-alkoxy steht,
- M: für Cyano, Halogen, Hydroxy, Nitro oder für gegebenenfalls ein- oder mehrfach, gleich oder verschieden mit Amino, Cyano, Halogen, Hydroxy, C₁-C₆-Alkoxy substituiertes C₁-C₆-alkyl, C₂-C₆-Alkenyl, C₂-C₆-Alkinyl steht oder für die Gruppe -O-R³ oder -COR⁴ steht,
- R⁵ und R⁶: unabhängig voneinander für Wasserstoff, C₁-C₆-alkyl oder für die Gruppe -COR⁴ stehen und
- n: für 1 bis 4 steht,
bedeuten, sowie deren Solvate, Hydrate, Stereoisomere, Diastereomere, Enantiomere und Salze mit der Vorgabe, dass wenn
- R¹: für Methyl steht, dann darf R² nicht gleichzeitig für Methyl, -CH₂-O-CH₃, Phenyl, Chlorphenyl, mit Hydroxy und/oder Methoxy substituiertes Benzofuranyl oder Furanyl stehen oder wenn
- R¹: für -CH₂-O-CH₃ steht, dann darf R² nicht gleichzeitig für Methyl stehen oder wenn
- R¹: für -CH=CH-Phenyl steht, dann darf R² nicht gleichzeitig für Phenyl stehen oder wenn
- R¹: für -CH=CH-Chlorphenyl steht, dann darf R² nicht gleichzeitig für Phenyl oder Chlorphenyl stehen oder wenn
- R¹: für-CH=CH-Methoxyphenyl steht, dann darf R² nicht gleichzeitig für Phenyl oder Methoxyphenyl stehen oder wenn
- R¹: für Phenyl steht, dann darf R² nicht gleichzeitig für -CF₃ oder Phenyl stehen oder wenn
- R¹: für Chlorphenyl steht, dann darf R² nicht gleichzeitig für Chlorphenyl stehen oder wenn
- R¹: für Hydroxyphenyl steht, dann darf R² nicht gleichzeitig für Heterocycloalkyl oder mit-COO-*tert-*Butyl substituiertes Heterocycloalkyl stehen oder wenn
- R¹: für Benzyloxyphenyl steht, dann darf R² nicht gleichzeitig für mit -COO-*tert*-Butyl substituiertes Heterocycloalkyl stehen, Proteintyrosinkinasen, insbesondere Eph-Rezeptoren, inhibieren.

Bevorzugt sind Verbindungen der vorgenannten allgemeinen Formel I, in der
- R¹: für gegebenenfalls ein- oder mehrfach, gleich oder verschieden mit K substituiertes C₁-C₆ alkyl, C₃-C₁₀-Cycloalkyl, Aryl oder Heteroaryl steht,
- K: für Halogen, Hydroxy oder gegebenenfalls ein- oder mehrfach, gleich oder verschieden mit L substituiertes Morpholinyl, Piperazinyl, Piperidinyl oder Phenoxy steht,
- L: für C₁-C₆-alkyl oder -COO-C₁-C₆ alkyl steht,
- R²: für gegebenenfalls ein- oder mehrfach, gleich oder verschieden mit M substituiertes C₁-C₆ alkyl, Aryl oder Heteroaryl steht,
- M: für Cyano, Halogen, Hydroxy, Nitro oder für gegebenenfalls ein- oder mehrfach, gleich oder verschieden mit Amino, Cyano, Halogen, Hydroxy, C₁-C₆-Alkoxy substituiertes C₁-C₆-alkyl, C₂-C₆-Alkenyl, C₂-C₆-Alkinyl steht oder für die Gruppe -O-C₁-C₆-alkyl, -O-(CH₂)ₙ-N(C₁-C₆-alkyl)₂, -CO-C₁-C₆-alkyl, -O-Phenyl oder -O-(CH₂)ₙ-Phenyl steht und
- n: für 1 bis 4 steht
bedeuten, sowie deren Solvate, Hydrate, Stereoisomere, Diastereomere, Enantiomere und Salze.

Besonders bevorzugt sind Verbindungen der vorgenannten allgemeinen Formel I, in der
- R¹: für gegebenenfalls ein- oder mehrfach, gleich oder verschieden mit K substituiertes *tert*-Butyl, Cyclopropyl, Cyclohexyl, Cyclohexanon, 1,4-Dioxa-spiro[4.5]dec-8-yl, Phenyl, 1,3-Benzodioxolyl oder Pyridinyl steht,
- K: für Halogen, Hydroxy oder gegebenenfalls ein- oder mehrfach, gleich oder verschieden mit L substituiertes Morpholinyl, Piperazinyl, Piperidinyl, Methoxy oder Phenoxy steht,
- L: für Methyl oder -COO-*tert*-Butyl steht,
- R²: für gegebenenfalls ein- oder mehrfach, gleich oder verschieden mit M substituiertes Isopropyl, Phenyl, Chinolinyl, Imidazolyl, Indolyl oder Pyridinyl steht und
- M: für Cyano, Halogen, Hydroxy, Nitro, Methyl, -CF₃ oder für Methoxy steht oder für die Gruppe -CO-Methyl, -O-(CH₂)₃-N(Methyl)₂, Phenoxy oder Benzyloxy steht
bedeuten, sowie deren Solvate, Hydrate, Stereoisomere, Diastereomere, Enantiomere und Salze.

Desweiteren sind Verbindungen der erstgenannten allgemeinen Formel I bevorzugt, in der
- R¹: für gegebenenfalls ein- oder mehrfach, gleich oder verschieden mit K substituiertes C₁-C₆-alkyl, C₂-C₆-alkenyl, C₂-C₆-alkinyl, C₃-C₁₀-Cycloalkyl, C₃-C₁₀-Heterocycloalkyl, Aryl oder Heteroaryl steht, wobei das C₃-C₁₀-Heterocycloalkyl selbst durch mindestens eins der folgenden Atome Stickstoff, Sauerstoff und/ oder Schwefel im Ring unterbrochen ist und C₃-C₁₀-Cycloalkyl und / oder C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder
- K: mehrere Doppelbindungen im Ring enthalten sein können, für Halogen, Hydroxy oder die Gruppe -O-R³ oder -NR⁵R⁶ steht oder für gegebenenfalls ein- oder mehrfach, gleich oder verschieden mit L substituiertes C₃-C₁₀-Cycloalkyl, C₃-C₁₀-Heterocycloalkyl, Aryl oder Heteroaryl steht, wobei das C₃-C₁₀-Heterocycloalkyl selbst durch mindestens eins der folgenden Atome Stickstoff, Sauerstoff und/ oder Schwefel im Ring unterbrochen ist und C₃-C₁₀-Cycloalkyl und / oder C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthalten sein können,
- L: für C₁-C₆-alkyl oder die Gruppe -COR⁴ oder -NR⁵R⁶ steht,
- R²: für gegebenenfalls ein- oder mehrfach, gleich oder verschieden mit M substituiertes C₁-C₆-alkyl, Aryl oder Heteroaryl steht,
- R³: für gegebenenfalls ein- oder mehrfach, gleich oder verschieden mit der Gruppe-NR⁵R⁶ substituiertes C₁-C₆-alkyl, Aryl oder -(CH₂)ₙ-Aryl steht
- R⁴: für Wasserstoff, Hydroxy, C₁-C₆-alkyl oder C₁-C₆-alkoxy steht,
- M: für Cyano, Halogen, Hydroxy, Nitro oder für gegebenenfalls ein- oder mehrfach, gleich oder verschieden mit Amino, Cyano, Halogen, Hydroxy, C₁-C₆-Alkoxy substituiertes C₁-C₆-alkyl, C₂-C₆-Alkenyl oder C₂-C₆-Alkinyl steht oder für die Gruppe -O-C₁-C₆-alkyl, -O-(CH₂)ₙ-NR⁵R⁶ oder -COR⁴ steht,
- R⁵ und R⁶: unabhängig voneinander für Wasserstoff, C₁-C₆-alkyl oder für die Gruppe -COR⁴ stehen und
- n: für 1 bis 4 steht
bedeuten, sowie deren Solvate, Hydrate, Stereoisomere, Diastereomere, Enantiomere und Salze.

Weiterhin sind solche Verbindungen der vorgenannten allgemeinen Formel I Bevorzugt, in der
- R¹: für gegebenenfalls ein- oder mehrfach, gleich oder verschieden mit K substituiertes C₁-C₆ alkyl, C₃-C₁₀-Cycloalkyl, Aryl oder Heteroaryl steht,
- K: für Halogen, Hydroxy oder gegebenenfalls ein- oder mehrfach, gleich oder verschieden mit L substituiertes Morpholinyl, Piperazinyl, Piperidinyl oder Phenyoxy steht,
- L: für C₁-C₆-alkyl oder -COO-C₁-C₆ alkyl steht,
- R²: für gegebenenfalls ein- oder mehrfach, gleich oder verschieden mit M substituiertes C₁-C₆ alkyl, Aryl oder Heteroaryl steht,
- M: für Cyano, Halogen, Hydroxy, Nitro oder für gegebenenfalls ein- oder mehrfach, gleich verschieden mit Amino, Cyano, Halogen, Hydroxy, C₁-C₆-Alkoxy substituiertes C₁-C₆-alkyl, C₂-C₆-Alkenyl, C₂-C₆-Alkinyl steht oder für die Gruppe -O-C₁-C₆-alkyl,-O-(CH₂)ₙ-N(C₁-C₆-alkyl)₂ oder -CO-C₁-C₆-alkyl steht und
- n: für 1 bis 4 steht
bedeuten, sowie deren Solvate, Hydrate, Stereoisomere, Diastereomere, Enantiomere und Salze.

Besonders bevorzugt sind solche Verbindungen der vorgenannten allgemeinen Formel, in der
- R¹: für gegebenenfalls ein- oder mehrfach, gleich oder verschieden mit K substituiertes C₁-C₆-alkyl, C₃-C₁₀-Cycloalkyl, Cyclohexanon, 1,4-Dioxa-spiro[4.5]dec-8-yl, Phenyl, 1,3-Benzodioxolyl oder Pyridinyl steht,
- K: für Halogen, Hydroxy oder gegebenenfalls ein- oder mehrfach, gleich oder verschieden mit L substituiertes Morpholinyl, Piperazinyl, Piperidinyl, Methoxy oder Phenoxy steht,
- L: für C₁-C₆-alkyl oder -COO-C₁-C₆ alkyl steht,
- R²: für gegebenenfalls ein- oder mehrfach, gleich oder verschieden mit M substituiertes C₁-C₆-alkyl, Phenyl oder Chinolinyl, Imidazolyl, Indolyl oder Pyridinyl steht,
- M: für Cyano, Halogen, Hydroxy, Nitro oder für gegebenenfalls ein- oder mehrfach, gleich oder verschieden mit Amino, Cyano, Halogen, Hydroxy, C₁-C₆-Alkoxy substituiertes C₁-C₆-alkyl steht oder für die Gruppe -O-C₁-C₆-alkyl, -O-(CH₂)ₙ-N(C₁-C₆-alkyl)₂ oder -CO-C₁-C₆-alkyl steht und
- n: für 1 bis 4 steht
bedeuten, sowie deren Solvate, Hydrate, Stereoisomere, Diastereomere, Enantiomere und Salze.

Überaus bevorzugt sind solche Verbindungen der allgemeinen Formel I, in der
- R¹: für gegebenenfalls ein- oder mehrfach, gleich oder verschieden mit K substituiertes *tert*-Butyl, Cyclopropyl, Cyclohexyl, Cyclohexanon, 1,4-Dioxa-spiro[4.5]dec-8-yl, Phenyl, 1,3-Benzodioxolyl oder Pyridinyl steht,
- K: für Halogen, Hydroxy oder gegebenenfalls ein- oder mehrfach, gleich oder verschieden mit L substituiertes Morpholinyl, Piperazinyl, Piperidinyl, Methoxy oder Phenoxy steht,
- L: für Methyl oder -COO-*tert*-Butyl steht,
- R²: für gegebenenfalls ein- oder mehrfach, gleich oder verschieden mit M substituiertes Isopropyl, Phenyl, Chinolinyl, Imidazolyl, Indolyl oder Pyridinyl steht und
- M: für Cyano, Halogen, Hydroxy, Nitro, Methyl, -CF₃ oder für Methoxy steht oder für die Gruppe -CO-Methyl, -O-(CH₂)₃-N(Methyl)₂, Phenoxy oder Benzyloxy steht
bedeuten, sowie deren Solvate, Hydrate, Stereoisomere, Diastereomere, Enantiomere und Salze.

Die für die Herstellung der erfindungsgemäßen Verbindungen der allgemeinen Formel I nach dieser ersten Ausführungsform der Erfindung vorzugsweise verwendeten Zwischenprodukte sind folgende Verbindungen der allgemeinen Formeln II und III 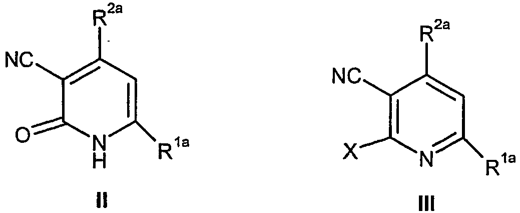 in denen
- X: für Halogen oder -O-SO₂-CₘF₂ₘ₊₁ steht,
- m: für 1 bis 4 steht,
- R^{1a}: für gegebenenfalls ein- oder mehrfach, gleich oder verschieden mit K^{a} substituiertes C₁-C₆-alkyl, C₁-C₆-alkenyl, C₂-C₆-alkinyl, C₃-C₁₀-Cycloalkyl, C₃-C₁₀-Heterocycloalkyl, Aryl oder Heteroaryl steht, wobei das C₃-C₁₀-Heterocycloalkyl selbst durch mindestens eins der folgenden Atome Stickstoff, Sauerstoff und/ oder Schwefel im Ring unterbrochen ist und C₃-C₁₀-Cycloalkyl und / oder C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthalten sein können,
- K^{a}: für Halogen, Hydroxy, die Gruppe -O-R^{3a}, -COR^{4a} oder -NR^{5a}R^{6a} steht oder für gegebenenfalls ein- oder mehrfach, gleich oder verschieden mit L^{a} substituiertes C₃-C₁₀-Cycloalkyl , C₃-C₁₀-Heterocycloalkyl, Aryl oder Heteroaryl steht, wobei das C₃-C₁₀-Heterocycloalkyl selbst durch mindestens eins der folgenden Atome Stickstoff, Sauerstoff und/ oder Schwefel im Ring unterbrochen ist und C₃-C₁₀-Cycloalkyl und / oder C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthalten sein können,
- L^{a}: für C₁-C₆-alkyl oder die Gruppe -COR^{4a} oder-NR^{5a}R^{6a} steht,
- R^{2a}: für gegebenenfalls ein- oder mehrfach, gleich oder verschieden mit M^{a} substituiertes C₁-C₆-alkyl, Aryl oder Heteroaryl steht,
- R^{3a}: für Trimethylsilyl (TMS), *tert*-Butyl-dimethylsilyl (TBDMS), *tert-*Butyl-diphenylsilyl (TBDPS), Triethylsilyl (TES), C₁-C₂-Alkyl, C₃-C₆-Allyl, Benzyl oder für die Gruppe -COR^{4a} oder für gegebenenfalls ein- oder mehrfach, gleich oder verschieden mit der Gruppe -NR^{5a}R^{6a} substituiertes C₁-C₆-alkyl, Aryl oder -(CH₂)ₙ-Aryl steht,
- R^{4a}: für Wasserstoff, C₁-C₆-alkyl oder C₁-C₆-alkoxy steht,
- M^{a}: für Cyano, Halogen, Hydroxy, Nitro oder für gegebenenfalls ein- oder mehrfach, gleich oder verschieden mit Amino, Cyano, Halogen, Hydroxy, C₁-C₆-Alkoxy substituiertes C₁-C₆-alkyl, C₂-C₆-Alkenyl, C₂-C₆-Alkinyl steht oder für die Gruppe -O-R^{3a} oder -COR^{4a} steht,
- R^{5a} und R^{6a}: unabhängig voneinander für Wasserstoff, C₁-C₆-alkyl oder für die Gruppe-COR^{4a} stehen und
- n^{a}: für 1 bis 4 steht
bedeuten, sowie deren Solvate, Hydrate, Stereoisomere, Diastereomere, Enantiomere und Salze.

Unter Alkyl ist jeweils ein geradkettiger oder verzweigter Alkylrest, wie beispielsweise Methyl, Ethyl, Propyl, Isopropyl, Butyl, Isobutyl, sek. Butyl, *tert*-Butyl, Pentyl, Isopentyl, Hexyl, Heptyl, Octyl, Nonyl und Decyl, zu verstehen.

Unter Alkoxy ist jeweils ein geradkettiger oder verzweigter Alkoxyrest, wie beispielsweise Methyloxy, Ethyloxy, Propyloxy, Isopropyloxy, Butyloxy, Isobutyloxy, sek. Butyloxy, Pentyloxy, Isopentyloxy, Hexyloxy, Heptyloxy, Octyloxy, Nonyloxy oder Decyloxy zu verstehen.

Die Alkenyl-Substituenten sind jeweils geradkettig oder verzweigt, wobei beispielsweise folgenden Reste gemeint sind: Vinyl, Propen-1-yl, Propen-2-yl, But-1-en-1-yl, But-1-en-2-yl, But-2-en-1-yl, But-2-en-2-yl, 2-Methyl-prop-2-en-1-yl, 2-Methyl-prop-1-en-1-yl, But-1-en-3-yl, But-3-en-1-yl, Allyl.

Unter Alkinyl ist jeweils ein geradkettiger oder verzweigter Alkinyl-Rest zu verstehen, der 2 - 6, bevorzugt 2 - 4 C-Atome enthält. Beispielsweise seien die folgenden Reste genannt: Acetylen, Propin-1-yl, Propin-3-yl, But-1-in-1-yl, But-1-in-4-yl, But-2-in-1-yl, But-1-in-3-yl, etc.

Heterocyclyl oder C₃-C₁₀-Herterocycloalkyl steht für einen 3 - 10 Kohlenstoffatome umfassenden Alkylring, durch mindestens eins der folgenden Atome Stickstoff, Sauerstoff und/ oder Schwefel im Ring unterbrochen ist und der C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthalten sein können. Es sind allerdings nur diejenigen Kombinationen gemeint, die aus Sicht eines Fachmanns sinnvoll sind, insbesondere in Bezug auf die Ringspannung.
Als Heterocyclyle seien z. B. genannt: Oxiranyl, Oxethanyl, Aziridinyl, Azetidinyl, Tetrahydrofuranyl, Pyrrolidinyl, Dioxolanyl, Imidazolidinyl, Pyrazolidinyl, Dioxanyl, Piperidinyl, Morpholinyl, Dithianyl, Thiomorpholinyl, Piperazinyl, Trithianyl, Chinuclidinyl etc..

Unter Cycloalkyl sind monocyclische C₃ -C₁₀ Alkylringe wie Cyclopropyl, Cyclobutyl, Cyclopentyl, Cyclohexyl oder Cycloheptyl, aber auch bicyclische Ringe oder tricyclische Ringe wie zum Beispiel Adamantanyl oder 1,4-Dioxa-spiro[4.5]dec-8-yl zu verstehen. Die Cycloalkylringe können unsubstituiert oder ein- oder mehrfach substituiert sein.

Ein Arylrest hat jeweils 6 - 12 Kohlenstoffatome wie beispielsweise Naphthyl, Biphenyl und insbesondere Phenyl.

Der Heteroarylrest umfaßt jeweils 3 - 16 Ringatome und enthält anstelle des Kohlenstoffs einoder mehrere, gleiche oder verschiedene Heteroatome aus der Gruppe Sauerstoff, Stickstoff oder Schwefel, kann mono-, bi- oder tricyclisch sein und kann zusätzlich jeweils benzokondensiert sein. Es sind allerdings nur diejenigen Kombinationen gemeint, die aus Sicht eines Fachmanns sinnvoll sind, insbesondere in Bezug auf die Ringspannung.
Die Heteroarylringe können unsubstituiert oder ein- oder mehrfach substituiert sein. Beispielsweise seien genannt:
Thienyl, Furanyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, Pyridyl, Pyridazinyl, Pyrimidinyl, Pyrazinyl, Triazinyl sowie Benzoderivate davon, wie z. B. 1,3-Benzodioxolyl, Benzofuranyl, Benzothienyl, Benzoxazolyl, Benzimidazolyl, Indazolyl, Indolyl, Isoindolyl, Oxepinyl, Azocinyl, Indolizinyl, Indolyl, Isoindolyl, Indazolyl, Benzimidazolyl, Purinyl, oder Chinolinyl, Isochinolinyl, Cinnolinyl, Phthalazinyl, Chinazolinyl, Chinoxalinyl, Naphthyridinyl, Pteridinyl, Carbazolyl, Acridinyl, Phenazinyl, Phenothiazinyl, Phenoxazinyl, Xanthenyl etc.

Unter Halogen ist jeweils Fluor, Chlor, Brom oder Jod zu verstehen.

### Weitere Ausführungsform der Erfindung:

Bei einer weiteren Ausführungsform der vorliegenden Erfindung welche die Aufgabe löst, handelt es sich gemäß Anspruch 1 um Verbindungen der allgemeinen Formel (I), 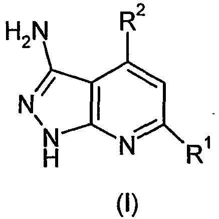 in der,
- R¹: für gegebenenfalls ein- oder mehrfach, gleich oder verschieden mit K substituiertes C₁-C₆-Alkyl, C₂-C₆-Alkenyl, C₂-C₆-Alkinyl, C₃-C₁₀-Cycloalkyl, C₃-C₁₀-Heterocycloalkyl, Aryl oder Heteroaryl steht, wobei das C₃-C₁₀-Heterocycloalkyl und/oder das Heteroaryl selbst durch mindestens eins der folgenden Atome Stickstoff, Sauerstoff und/oder Schwefel im Ring unterbrochen ist und das C₃-C₁₀-Heterocycloalkyl und/oder Heteroaryl nur über ein Kohlenstoffringatom mit dem Pyrazolopyridin verknüpft sein kann, steht und C₃-C₁₀-Cycloalkyl und/oder C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthalten sein können, oder für ein mit der Gruppe -O-(CH₂)ₙ-O-substituiertes C₃-C₁₀-Cycloalkyl, Aryl, C₃-C₁₀-Heterocycloalkyl oder Heteroaryl steht, wobei die endständigen Sauerstoffatome der -O-(CH₂)ₙ-O- Gruppe mit einem gleichen oder direkt benachbarten C₃-C₁₀-Cycloalkylring-, Arylring-, C₃-C₁₀-Heterocycloalkyring- oder Heteroarylring-Kohlenstoffatom verknüpft sind,
- K: für Halogen, Hydroxy oder die Gruppe -OR³, -COR⁴ oder -NR⁵R⁶ steht oder für gegebenenfalls ein- oder mehrfach, gleich oder verschieden mit L substituiertes C₃-C₁₀-Cycloalkyl, C₃-C₁₀-Hoterocycloalkyl, Aryl oder Heteroaryl steht, wobei das C₃-C₁₀-Heterocycloalkyl und/ oder das Heteroaryl selbst durch mindestens eins der folgenden Atome Stickstoff, Sauerstoff und/oder Schwefel im Ring unterbrochen ist und C₃-C₁₀-Cycloalkyl und/oder C₃-C₁₀-Heterocycloalkyl gegebenenfalls durch ein oder mehrere, gleich oder verschiedene -(CO)-, -SO-oder -SO₂- Gruppen im Ring unterbrochen sein kann und gegebenenfalls ein oder mehrere Doppelbindungen im Ring enthalten sein können,
- L: für C₁-C₆-Alkyl oder die Gruppe -COR⁴, -OR³ oder -NR⁵R⁶ steht,
- R²: für gegebenenfalls ein- oder mehrfach, gleich oder verschieden mit M substituiertes C₁-C₆ Alkyl, C₃-C₁₀-Cycloalkyl. Aryl oder Heteroaryl steht,
- R³: für gegebenenfalls ein- oder mehrfach, gleich oder verschieden mit der Gruppe-NR⁵R⁶ substituiertes C₁-C₅-Alkyl, Aryl oder -(CH₂)ₙ-Aryl steht,
- R⁴: für Wasserstoff, Hydroxy, C₁-C₆-Alkyl oder C₁-C₆-Alkoxy steht,
- M: für Amino, Cyano, Halogen, Hydroxy, Nitro oder für gegebenenfalls ein- oder mehrfach, gleich oder verschieden mit Amino, Cyano, Halogen, Hydroxy, Nitro, C₁-C₆-Alkoxy substituiertes C₁-C₆-Alkyl, C₂-C₆-Alkenyl, C₂-C₆-Alkinyl steht oder für die Gruppe -OR³, -COR⁴, oder -CO-N-R⁷ steht,
- R⁵ und R⁶: unabhängig voneinander für Wasserstoff, C₁-C₆-Alkyl oder für die Gruppe-COR⁴ stehen und
- R⁷: Wasserstoff oder NH₂ steht.
- n: für 1 bis 4 steht,
mit der Vorgabe, dass wen
- R¹: for Methyl steht, dann darf R² nicht gleichzeitig für -CH₂O-CH₃, Chlorphenyl, mit Hydroxy und/oder Methoxy substituiertes Benzofuranyl, -CF₃ oder Furanyl stehen oder wenn
- R¹: für -CH₂-O-CH₃ steht, dann darf R² nicht gleichzeitig für Methyl stehen oder wenn
- R¹: für -CH=CH-Phenyl steht, dann darf R² nicht gleichzeitig für Phenyl stehen oder wenn
- R¹: für -CH=CH-Chlorphenyl steht, dann darf R² nicht gleichzeitig für Phenyl oder Chlorphenyl stehen oder wenn
- R¹: für -CH=CH-Methoxyphenyl steht, dann darf R² nicht gleichzeitig für Phenyl oder Methoxyphenyl stehen oder wenn
- R¹: für Phenyl steht, dann darf R² nicht gleichzeitig für -CF₃, p-Methylphenyl oder Methoxyphenyl stehen oder wenn
- R¹: für Methoxyphenyl steht, dann darf R² nicht gleichzeitig für -CF₃ stehen oder wenn
- R¹: für Methylphenyl steht, dann darf R² nicht gleichzeitig -CF₃ stehen oder wenn
- R¹: für Chlorphenyl steht, dann darf R² nicht gleichzeitig für Chlorphenyl oder -CF₃ stehen oder wenn
- R¹: für Dichlorphenyl steht, dann darf R² nicht gleichzeitig für Trimethoxyphenyl stehen oder wenn
- R¹: für Bromphenyl steht, dann darf R² nicht gleichzeitig nicht für Trimethoxyphenyl stehen oder wenn
- R¹: für Alkyl, Alkenyl, Aryl, Aralkyl, Cycloalkyl, für ein mit Phenyl substituiertes Alkyl oder p-Methoxyphenyl steht, dann darf R² nicht gleichzeitig auch für Alkyl, Alkenyl, Aryl, Aralkyl, heterocyclischer Rest oder Cycloalkyl stehen oder wenn
- R¹: für Hydroxyphenyl steht, dann darf R² nicht gleichzeitig für Heterocycyl oder mit-COO-*tert*-Butyl substituiertes Heterocycloalkyl stehen oder wenn
- R¹: für Benzyloxyphenyl steht, dann darf R² nicht gleichzeitig für mit -COO-*tert-*Butyl substituiertes Heterocycloalkyl stehen, oder wenn
- R¹: für p-Methylphenyl steht, dann darf R² nicht gleichzeitig für Phenyl stehen, oder wenn
- R¹: für Cyclopropyl steht, dann darf R² nicht gleichzeitig für p-Methoxyphenyl stehen ;
und mit der Vorgabe, dass wenn :
- R¹: für unsubstituiertes C₁-C₆-Alkyl, Aryl, C₃-C₁₀-Cycloalkyl steht, oder für einen mit K substituiertes C₁-C₆-Alkyl steht und K für Aryl steht, dann steht
- R²: für ein- oder mehrfach, gleich oder verschieden mit M substituiertes C₁-C₆-Alkyl, C₃-C₁₀-Cycloalkyl, Aryl oder Heteroaryl;
oder wenn :
- R²: für unsubstituiertes C₁-C₆-Alkyl, C₃-C₁₀-Cycloalkyl oder Aryl steht, dann steht
- R¹: für ein- oder mehrfach, gleich oder verschieden mit M substituiertes C₁-C₆-Alkyl, C₃-C₁₀-Cycloalkyl, Aryl oder Heteroaryl ;
bedeuten, sowie deren Solvate, Hydrate, Stereoisomere, Diastereomere, Enantiomere und Salze.

Auf Grund des nachfolgenden Standes der Technik wurde der oben beschriebene Disclaimer eingeführt.

Ein Pyrazolopyridin mit R¹ = Methyl und R²= Methyl wird beispielsweise von Kaupp. G. et al. 2003, European Journal of Organic Chemistry, (8) S.1545-1551, Gamal A., 1994, Zagazig Journal of Pharmaceutical Sciences, 3(2) 148-53, Kalme Z. A. et al., 1992, Khimiya Geterotsiklicheskikh Soedinenil, (9), 1218-22, EI-Dean A. M. Kamal et al., 1991, Bulletin of the Faculty of Science, Assiut University, 20(1), 15-21 oder von El-Dean A. M. et al, 1991, Indian Journal of Chemistry, 30B (9), 878-82 beschrieben.

Ein Pyrazolopyridine mit R¹ = Chlorpenyl und R² = Chlorphenyl ist durch Gamal et al., 2001. Journal of Saudi Chemical Society, 5(2), S. 183-187 bekannt.

Mohamed A. et al., 2000, Phosphorus, Sulfur and Silicon, 167, 161-179 beschreibt Pyrazolopyridine bei denen R¹ = Methyl und gleichzeitig R² = Chlorphenyl, sowie R¹ = Methyl und gleichzeitig R² = Furanyl steht. Attaby, Fawzy A. et al., 1999, Phosphorus, Sulfur and Silicon, 149, 49-64 zeigen, dass diese Substanzen antimikrobielle Eigenschaften haben.

Attaby, Fawzy A. et al., 1999, Phosphorus, Sulfur and Silicon, 155, 253-270 offenbaren beispielsweise Pyrazolopyridine mit antimikrobieller Wirkung bei denen R¹ =-CH=CH-Phenyl und gleichzeitig R² = Phenyl, R¹ = -CH=CH-Methoxyphenyl und gleichzeitig R² = Phenyl oder Methoxyphenyl, R¹ = -CH=CH-Chlorphenyl und gleichzeitig R² = Phenyl oder Chlorphenyl sein können. Sanna Eldin M. et al., 1998, Egyptian Journal of Pharmaceutical Sciences, 39, (1-3), 197-209 beschreiben die gleichen Verbindungen und zeigen, dass diese Substanzen antibakterielle Eigenschaften aufweisen.

Arustamova, I. S. et al., 1999, Chemistry of Heterocyclic Compounds, 35(1), 58-63 beschreibt Pyrazolopyridine bei denen R¹ = Methyl und gleichzeitig R²=-CH₂-O-CH₃ bedeuten.

Lacan M. und Tabakovic K. (1975) Chroatica Chemica Acta 47 (2), 127-133 offenbaren Pyrazolopyridine bei denen R¹ für Methyl steht während gleichzeitig R² auch für Methyl steht.

Chandra Sheker Reddy et al., 1997, Journal of Fluorine Chemistry, 86(2) 127-130 beschreibt Pyrazolopyridine bei denen R¹ = Methyl und gleichzeitig R² = Methyl, R¹ = Phenyl und gleichzeitig R²=-CF₃, R¹ = Methylphenyl und gleichzeitig R² = -CF₃, R¹ = Methoxyphenyl und gleichzeitig R²= -CF₃, sowie R¹ = Chlorphenyl und gleichzeitig R²= -CF₃ bedeuten.

Abdel Hafez et al., 1993, Collection of Czechoslovak Chemical Communications, 58(5), 1198-202; Deeb Ali, 1991, Collection of Czechoslovak Chemical Communications, 56(7), 1560-3 offenbaren ein Pyrazolopyridin mit R¹ = Phenyl und gleichzeitig R²= Phenyl.

Gohar, Abdel Kerim et al., 1987, Archiv der Pharmazie (Weinheim, Germany), 320(9), 823-9 offenbaren Pyrazolopyridine bei denen R¹ = Methyl und gleichzeitig R² =mit Hydroxy und/oder Methoxy substituiertes Benzofuranyl bedeuten.

Balicki R. et al., 1979, Polish Journal of Chemistry, 53(7-8), 1515-25 beschreibt ein Pyrazolopyridin beim dem R¹ = Methyl und gleichzeitig R² = -CF₃ bedeuten.

Pejcic Marijan et al., 1977, Acta Pharmaceutica Jugoslavia, 27(3), 143-6 offenbaren Pyrazolopyridine mit antibakteriellen Eigenschaften bei denen R¹ = Methyl und gleichzeitig R²=-CH₂-O-CH₃, sowie R¹ = -CH₂-O-CH₃ und gleichzeitig R²= Methyl bedeuten.

Bomika Z. et al., 1976, Khimiya Geterotsiklicheskikh Soedinenii, (8), 1085-8 beschreibt ein Pyrazolopyridin bei dem R¹ = Phenyl und gleichzeitig R² = Phenyl, R¹ = Methyl und gleichzeitig R² = Phenyl bedeuten.

Yoshida Kei et al., 1976, Yakugaku Zasshi, 96(1), 33-6 beschreibt ein Pyrazolopyridin bei dem R¹ = Methyl und gleichzeitig R²= -CH₂-O-CH₃ bedeuten,

Die DE 30 01 498 A1 (Agfa-Gevaert AG) offenbart Pyrazole die für fotografische Materialien verwendet werden können. Die Patentschrift offenbart Pyrazolopyridine bei denen R¹ für eine unsubstituierte Alkyl, Alkenyl, Aryl, Aralkyl oder Cycloalkylgruppe steht und R² gleichzeitig für eine unsubstituierte Alkyl, Alkenyl, Aryl, Aralkyl oder Cycloalkylgruppe steht.

In der DE 26 43 753 A1 (Dr. Karl Thomae GmbH) werden Pyrazolopyridine beschrieben, die über eine antiphlogistische und insbesondere antithrombotische Wirkung verfügen. Die offenbarten Substituenten sind immer über ein Stickstoffatom mit dem Pyridin (des Pyrazolopyridins) an der Position 6 verknüpft.

F.E. Goda et al. 2004, Bioorganic & Medicinal Chemistry, 12(8), S. 1845-1852 offenbart Pyrazolopyridine, die über eine antimikrobielle Wirkung verfügen. R¹ steht dabei für Dichlorphenyl und R² gleichzeitig für Trimethoxyphenyl (Verbindung 5b) oder R¹ steht für Bromphenyl und R² dann gleichzeitig für Trimethoxyphenyl (Verbindung 5c). Die Verbindungen 3a bis 5a in der Tabelle 1 auf Seite 1846 betreffen Vorstufen von Pyrazolopyridinen, aber nicht Pyrazolopyridine selber.

Registry Nummer 774531-14-5 offenbart ein Pyrazolopyridin bei dem R¹ = Hydroxyphenyl und gleichzeitig R² ein Pyperidin ist. Registry Nummer 692775-16-9 offenbart ein Pyrazolopyridin bei dem R¹ = Phenyl und gleichzeitig R² ein Methoxyphenyl ist. Registry Nummer 201224-90-0 offenbart ein Pyrazolopyridin bei dem R¹ = Methoxyphenyl und gleichzeitig R² ein -CF₃ ist.

B. Narsaiah et al. 2001, Journal of Fluorine Chemistry, 109, 183-7 beschreibt verschiedene mit CF₃-substituierte Pyrazolopyridine. R² steht dabei für-CF₃ während R¹ entweder für Phenyl, Methylphenyl oder Chlorphenyl steht.

Die WO 01/19828 A2 offenbart ein Pyrazolopyridin welches mit einer Diaminopyrazolyl- (an R¹ Position) und einer Methylgruppe (an R² Position) substituiert ist. Das Diaminopyrazolyl ist über das Stickstoffatom mit dem Pyridin (des Pyrazolopyridins) an der Position 6 verknüpft. Die in dieser Patentschrift beschriebenen Substanzen können als Haarfärbemittel verwendet werden

Die deutsche Offenlegungsschrift DE2160780 offenbart Pyridinopyrazole für die Verwendung als Diazokomponenten bei denen R¹ für Phenyl oder Methyl steht und gleichzeitig R² für Phenyl oder Methyl steht.

Die deutsche Offenlegungsschrift DE2238400 Pyrazolopyridine als Farbstoffe bei denen R¹ für Wasserstoff, einen gegebenenfalls substituierten Alkyl-, Aralkyl-, Cycloalkyl-, oder Arylrest steht und R² für Wasserstoff, einen gegenenfalls substituierten Alkyl-, Aralkyl-, Cycloalkyl-, Aryl- oder heterocyclischen Rest oder eine Cyanogruppe steht. In der Anmeldung ist nicht definiert, welche Substituitionen in Frage kommen können. In den Beispielen findet sich für R¹ nur eine mit einem Phenylring substituierte Ethylgruppe, ein p-Nitrophenyl und ein p-Methoxyphenyl. Für R² findet man keine Beispiele mit Substitutionen. Auch für R² definiert als heterocyclischen Rest findet man keine Definition und auch keine Beispiele.

Die deutsche Offenlegungsschrift DE2355967 offenbart polycyclische Farbstoffe zu deren Herstellung Pyrazolopyridine verwendet werden bei denen R¹ für einen niederen Alkylrest (in den Beispielen ist Methyl genannt), eine Alkoxy- oder Aryloxygruppe oder eine primäre, sekundäre oder tertiäre Aminogruppe steht und R² gleichzeitig für einen niederen Alkylrest steht (in den Beispielen ist Methyl und Phenyl für R² genannt).

Keine der oben genannten Publikationen und Patentschriften offenbaren Eph-Rezeptorinhibitoren.

Gegenstand der Erfindung gemäß dieser Ausführungsform sind auch Verbindungen der allgemeinen Formel I in Anspruch 2, gemäß Anspruch 1, in der
- R¹: für gegebenenfalls ein- oder mehrfach, gleich oder verschieden mit K substituiertes C₁-C₆ Alkyl, C₃-C₁₀-Cycloalkyl, wobei das C₃-C₁₀-Cycloalkyl gegebenenfalls durch ein oder mehrere -(CO)-, -SO- oder -SO₂-Gruppen im Ring unterbrochen ist und gegenenfalls ein oder mehrere Doppelbindungen im Ring enthalten sein können, steht, oder für gegebenenfalls ein- oder mehrfach, gleich oder verschieden mit K substituiertes Aryl, C₃-C₁₀-Heterocycloalkyl oder Heteroaryl steht, wobei das C₃-C₁₀-Heterocycloalkyl und/oder das Heteroaryl selbst durch mindestens ein Stickstoff/ Sauerstoff und/oder Schwefel unterbrochen ist und das C₃-C₁₀-Heterocycloalkyl und/oder Heteroaryl nur über ein Kohlenstoffringatom mit dem Pyrazolopyridin verknüpft sein kann, steht, oder für ein mit der Gruppe -O-(CH₂)ₙ-O- substituiertes C₃-C₁₀-Cycloalkyl, Aryl, C₃-C₁₀-Heterocycloalkyl oder Heteroaryl steht, wobei die endständigen Sauerstoffatome der -O-(CH₂)ₙ-O- Gruppe mit einem gleichen oder direkt benachbarten C₃-C₁₀-Cycloalkylring-, Arylring-, C₃-C₁₀-Heterocycloalkylring-, oder Heteroarylring-Kohlenstoffatom verknüpft sind,
- K: für Halogen, Hydroxy oder die Gruppe -OR³, -COR⁴ steht oder für gegebenenfalls ein- oder mehrfach, gleich oder verschieden mit L substituiertes C₃-C₁₀-Cycloalkyl, C₃-C₁₀-Heterocycloalkyl, Aryl oder Heteroaryl steht, wobei das C₃-C₁₀-Heterocycloalkyl und/oder das Heteroaryl selbst durch mindestens eins der folgenden Atome Stickstoff, Sauerstoff und/oder Schwefel im Ring unterbrochen ist und C₃-C₁₀-Cycloalkyl und/oder C₃-C₁₀-Heterocycloalkyl gegebenenfalls durch ein oder mehrere, gleich oder verschiedene -(CO)-, -SO-oder -SO₂- Gruppen im Ring unterbrochen sein kann und gegebenenfalls ein oder mehrere Doppelbindungen im Ring enthalten sein können,
- R²: für gegebenenfalls ein- oder mehrfach, gleich oder verschieden mit M substituiertes C₁-C₆-Alkyl, C₃-C₁₀-Cycloalkyl, Aryl oder Heteroaryl steht, wobei das Heteroaryl durch mindestens einen Stickstoff, Sauerstoff und/oder Schwefel unterbrochen ist, steht,
- R³: für C₁-C₆-Alkyl, Aryl oder -(CH₂)ₙ-Aryl steht,
- M: für Amino, Cyano, Halogen, Hydroxy, Nitro oder für gegebenenfalls ein- oder mehrfach, gleich oder verschieden mit Amino, Cyano, Halogen, Hydroxy, Nitro, C₁-C₆-Alkoxy substituiertes C₁-C₆-Alkyl steht oder für die Gruppe -O-C₁-C₆-Alkyl, -O-(CH₂)ₙ-NR⁵R⁶, -COR⁴,-O-Phenyl, -O-(CH₂)ₙ-Phenyl oder -CO-N-R⁷ steht,
bedeuten, sowie deren Solvate, Hydrate, Stereoisomere, Diastereomere, Enantiomere und Salze.

Ein weiterer Gegenstand der vorliegenden Erfindung gemäß dieser Ausführungsform sind auch Verbindungen der allgemeinen Formel I in Anspruch 3, gemäß Anspruch 2, in der
- R¹: für gegebenenfalls ein- oder mehrfach, gleich oder verschieden mit K substituiertes C₁-C₆ Alkyl, C₃-C₁₀-Cycloalkyl, wobei das C₃-C₁₀-Cycloalkyl gegebenenfalls durch ein oder mehrere -(CO)-, -SO- oder -SO₂-Gruppen im Ring unterbrochen ist und gegenenfalls ein oder mehrere Doppelbindungen im Ring enthalten sein können, steht, oder für gegebenenfalls ein- oder mehrfach, gleich oder verschieden mit K substituiertes Aryl oder Heteroaryl steht, wobei das Heteroaryl selbst durch mindestens ein Stickstoff, Sauerstoff und/oder Schwefel unterbrochen ist und das Heteroaryl nur über ein Kohlenstoffringatom mit dem Pyrazolopyridin verknüpft sein kann, steht, oder für ein mit der Gruppe -O-(CH₂)ₙ-O- substituiertes C₃-C₁₀-Cycloalkyl, Aryl oder Heteroaryl steht, wobei die endständigen Sauerstoffatome der -O-(CH₂)ₙ-O- Gruppe mit einem gleichen oder direkt benachbarten C₃-C₁₀-Cycloalkylring-, Arylring- oder Heteroarylring-Kohlenstoffatom verknüpft sind,
- K: für Halogen, Hydroxy oder die Gruppe -OR³, -COR⁴ steht oder für gegebenenfalls ein- oder mehrfach, gleich oder verschieden mit L substituiertes C₃-C₁₀-Cycloalkyl,oder C₃-C₁₀-Heterocycloalkyl, wobei das C₃-C₁₀-Heterocycloalkyl selbst durch mindestens eins der folgenden Atome Stickstoff, Sauerstoff und/oder Schwefel im Ring unterbrochen ist und C₃-C₁₀-Cycloalkyl und/oder C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthalten sein können,
- L: für C₁-C₆-Alkyl oder die Gruppe -COR⁴, -OR³ steht,
- R³: für C₁-C₆-Alkyl oder Aryl steht,
- M: für Amino, Cyano, Halogen, Hydroxy, Nitro oder für gegebenenfalls ein- oder mehrfach, gleich oder verschieden mit Amino, Cyano, Halogen, Hydroxy, Nitro, C₁-C₆-Alkoxy substituiertes C₁-C₆-Alkyl steht oder für die Gruppe -O-C₁-C₆-Alkyl, -O-(CH₂)ₙ-NR⁵R⁶, -CO-C₁-C₆-Alkyl, -O-Phenyl, -O-(CH₂)ₙ-Phenyl oder -CO-N-R⁷ steht,
bedeuten, sowie deren Solvate, Hydrate, Stereoisomere, Diastereomere, Enantiomere und Salze.

Ein weiterer Gegenstand der vorliegenden Erfindung gemäß dieser Ausführungsform sind auch Verbindungen der allgemeinen Formel I in Anspruch 4, gemäß Anspruch 3, in der
- R¹: für gegebenenfalls ein- oder mehrfach, gleich oder verschieden mit K substituiertes C₁-C₆ Alkyl, C₃-C₁₀-Cycloalkyl, wobei das C₃-C₁₀-Cycloalkyl gegebenenfalls durch ein oder mehrere -(CO)-Gruppen im Ring unterbrochen ist, steht, oder für gegebenenfalls ein- oder mehrfach, gleich oder verschieden mit K substituiertes Aryl oder Heteroaryl steht, wobei das Heteroaryl selbst durch mindestens ein Stickstoff unterbrochen ist und das Heteroaryl nur über ein Kohlenstoffringatom mit dem Pyrazolopyridin verknüpft sein kann, steht, oder für ein mit der Gruppe -O-(CH₂)ₙ-O- substituiertes C₃-C₁₀-Cycloalkyl, Aryl oder Heteroaryl steht, wobei die endständigen Sauerstoffatome der -O-(CH₂)ₙ-O-Gruppe mit einem gleichen oder direkt benachbarten C₃-C₁₀-Cycloalkylring-, Arylring- oder Heteroarylring-Kohlenstoffatom verknüpft sind,
- K: für Halogen, Hydroxy oder für die Gruppe -OR³ steht, oder gegebenenfalls einoder mehrfach, gleich oder verschieden mit L substituiertes C₃-C₁₀-Heterocycloalkyl, wobei das C₃-C₁₀-Heterocycloalkyl selbst durch mindestens eins der folgenden Atome Stickstoff und/oder Sauerstoff im Ring unterbrochen ist, steht,
- L: für C₁-C₆-Alkyl oder -COO-C₁-C₆-Alkyl steht,
- R²: für gegebenenfalls ein- oder mehrfach, gleich oder verschieden mit M substituiertes C₁-C₆ Alkyl, C₃-C₆-Cycloalkyl, Aryl oder Heteroaryl, wobei das Heteroaryl durch mindestens einen Stickstoff unterbrochen ist, steht,
- M: für Amino, Cyano, Halogen, Hydroxy, Nitro oder für gegebenenfalls ein- oder mehrfach, gleich verschieden mit Amino, Cyano, Halogen, Hydroxy, Nitro oder C₁-C₆-Alkoxy substituiertes C₁-C₆-Alkyl steht oder für die Gruppe -O-C₁-C₆-Alkyl, -O-(CH₂)ₙ-N(C₁-C₆-Alkyl)₂, -CO-C₁-C₆-Alkyl, Phenoxy, Benzyloxy oder -CO-N-NH₂ steht,
bedeuten, sowie deren Solvate, Hydrate; Stereoisomere, Diastereomere, Enantiomere und Salze.

Ein weiterer Gegenstand der vorliegenden Erfindung gemäß dieser Ausführungsform sind Verbindungen der allgemeinen Formel I in Anspruch 5, gemäß einem der Ansprüche 1 bis 4, in der
- M: für Amino, Cyano, Halogen, Hydroxy, Nitro oder für gegebenenfalls ein- oder mehrfach, gleich verschieden mit Amino, Cyano, Halogen, Hydroxy, Nitro oder C₁-C₆-Alkoxy substituiertes C₁-C₆-Alkyl steht oder für die Gruppe -O-C₁-C₆-Alkyl, -O-(CH₂)ₙ-N(C₁-C₆-Alkyl)₂ oder -CO-C₁-C₆-Alkyl steht und
bedeuten, sowie deren Solvate, Hydrate, Stereoisomere, Diastereomere, Enantiomere und Salze.

Ein weiterer Gegenstand der vorliegenden Erfindung gemäß dieser Ausführungsform sind Verbindungen der allgemeinen Formel I in Anspruch 6, gemäß Anspruch 4, in der
- R¹: für gegebenenfalls ein- oder mehrfach, gleich oder verschieden mit K substituiertes C₁-C₆-Alkyl, C₃-C₆-Cycloalkyl, Cyclohexanon, 1,4-Dioxa-spiro[4.5]dec-8-yl, Phenyl, 1,3-Benzodioxolyl oder Pyridinyl steht,
- K: für Halogen, Hydroxy, C₁-C₆-Alkoxy oder gegebenenfalls ein- oder mehrfach, gleich oder verschieden mit L substituiertes Morpholinyl, Piperazinyl, Piperidinyl, oder Phenoxy steht,
- R²: für gegebenenfalls ein- oder mehrfach, gleich oder verschieden mit M substituiertes C₁-C₆-Alkyl, Phenyl oder Chinolinyl, Imidazolyl, Indolyl oder Pyridinyl steht,
- M: für Amino, Cyano, Halogen, Hydroxy, Nitro, C₁-C₆-Alkyl, -CF₃, oder für C₁-C₃-Alkoxy steht oder für die Gruppe -CO-C₁-C₆-Alkyl, -O-(CH₂)ₙ-N(C₁-C₆-Alkyl)₂, Phenoxy oder Benzyloxy,
bedeuten, sowie deren Solvate, Hydrate, Stereoisomere, Diastereomere, Enantiomere und Salze.

Ein weiterer Gegenstand der vorliegenden Erfindung gemäß dieser Ausführungsform sind Verbindungen der allgemeinen Formel I in Anspruch 7, gemäß Anspruch 6, in der
- R¹: für gegebenenfalls ein- oder mehrfach, gleich oder verschieden mit K substituiertes C₃-C₆ Alkyl, Cyclopropyl, Cyclohexyl, Cyclohexanon, 1,4-Dioxa-spiro[4.5]dec-8-yl, Phenyl, 1,3-Benzodioxolyl oder Pyridinyl steht,
- K: für Halogen, Hydroxy, Methoxy oder gegebenenfalls ein- oder mehrfach, gleich oder verschieden mit L substituiertes Morpholinyl, Piperazinyl, Piperidinyl oder Phenoxy steht,
- L: für C₁-C₃-Alkyl oder -COO-C₃-C₅-Alkyl steht,
- R²: für gegebenenfalls ein- oder mehrfach, gleich oder verschieden mit M substituiertes Isopropyl, Phenyl, Chinolinyl, Imidazolyl, Indolyl oder Pyridinyl steht und
- M: für Cyano, Halogen, Hydroxy, Nitro, Methyl, -CF₃ oder für Methoxy steht oder für die Gruppe -CO-C₁-C₃-Alkyl oder -O-(CH₂)₃-N(Methyl)₂ steht,
bedeuten, sowie deren Solvate, Hydrate, Stereoisomere, Diastereomere, Enantiomere und Salze.

Ein weiterer Gegenstand der vorliegenden Erfindung gemäß dieser Ausführungsform sind Verbindungen der allgemeinen Formel (I,) in Anspruch 8, gemäß Anspruch 7, in der
- R¹: für gegebenenfalls ein- oder mehrfach, gleich oder verschieden mit K substituiertes *tert*-Butyl, Cyclopropyl, Cyclohexyl, Cyclohexanon, 1,4-Dioxa-spiro[4.5]dec-8-yl, Phenyl, 1,3-Benzodioxolyl oder Pyridinyl steht,
- L: für Methyl oder -COO-*tert*-Butyl steht,
- R²: für gegebenenfalls ein- oder mehrfach, gleich oder verschieden mit M substituiertes Isopropyl, Phenyl, Chinolinyl, Imidazolyl, Indolyl oder Pyridinyl steht und
- M: für Cyano, Halogen, Hydroxy, Nitro, Methyl, -CF₃ oder für Methoxy steht oder für die Gruppe-CO-Methyl oder -O-(CH₂)₃-N(Methyl)₂ steht,
bedeuten, sowie deren Solvate, Hydrate, Stereoisomere, Diastereomere, Enantiomere und Salze.

Ein Gegenstand der Erfindung gemäß dieser Ausführungsform sind Verbindungen der allgemeinen Formel (I), gemäß einem der Ansprüche 1 bis 8, bei der R¹ für gegebenenfalls ein- oder mehrfach, gleich oder verschieden mit K substituiertes C₁-C₆-Alkyl, C₂-C₆-Alkenyl, C₂-C₆-Alkinyl, C₃-C₁₀-Cycloalkyl, C₃-C₁₀-Heterocycloalkyl, Aryl oder Heteroaryl steht, wobei das C₃-C₁₀-Heterocycloalkyl und/oder das Heteroaryl selbst durch mindestens eins der folgenden Atome Stickstoff, Sauerstoff und/oder Schwefel im Ring unterbrochen ist und das C₃-C₁₀-Heterocycloalkyl und/oder Heteroaryl nur über ein Kohlenstoffringatom mit dem Pyrazolopyridin verknüpft sein kann, steht und C₃-C₁₀-Cycloalkyl und/oder C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthalten sein können, oder für ein mit der Gruppe -O-(CH₂)ₙ-O- substituiertes C₃-C₁₀-Cycloalkyl, Aryl, C₃-C₁₀-Heterocycloalkyl oder Heteroaryl steht, wobei die endständigen Sauerstoffatome der -O-(CH₂)ₙ-O- Gruppe mit einem gleichen oder direkt benachbarten C₃-C₁₀-Cycloalkylring-, Arylring-, C₃-C₁₀-Heterocycloalkyring- oder Heteroarylring-Kohlenstoffatom verknüpft sind,

Ein weiterer Gegenstand der Erfindung gemäß dieser Ausführungsform sind Verbindungen der allgemeinen Formel (I), gemäß einem der Ansprüche 1 bis 8, in der R¹ für gegebenenfalls ein- oder mehrfach, gleich oder verschieden mit K substituiertes C₁-C₆ Alkyl, C₃-C₁₀-Cycloalkyl steht, wobei das C₃-C₁₀-Cycloalkyl gegebenenfalls durch ein oder mehrere -(CO)-, -SO- oder -SO₂-Gruppen im Ring unterbrochen ist und gegenenfalls ein oder mehrere Doppelbindungen im Ring enthalten sein können, oder für gegebenenfalls ein- oder mehrfach, gleich oder verschieden mit K substituiertes Aryl, C₃-C₁₀-Heterocycloalkyl oder Heteroaryl, wobei das C₃-C₁₀-Heterocycloalkyl und/oder das Heteroaryl selbst durch mindestens ein Stickstoff, Sauerstoff und/oder Schwefel unterbrochen ist und das C₃-C₁₀-Heterocycloalkyl und/oder Heteroaryl nur über ein Kohlenstoffringatom mit dem Pyrazolopyridin verknüpft sein kann, oder für ein mit der Gruppe -O-(CH₂)ₙ-O- substituiertes C₃-C₁₀-Cycloalkyl, Aryl, C₃-C₁₀-Heterocycloalkyl oder Heteroaryl steht, wobei die endständigen Sauerstoffatome der-O-(CH₂)ₙ-O- Gruppe mit einem gleichen oder direkt benachbarten C₃-C₁₀-Cycloalkylring-, Arylring-, C₃-C₁₀-Heterocycloalkylring-, oder Heteroarylring-Kohlenstoffatom verknüpft sind.

Ein weiterer Gegenstand der Erfindung gemäß dieser Ausführungsform sind Verbindungen der allgemeinen Formel (I), gemäß einem der Ansprüche 1 bis 8, bei der R¹ für gegebenenfalls ein- oder mehrfach, gleich oder verschieden mit K substituiertes C₁-C₆ Alkyl, C₃-C₁₀-Cycloalkyl steht, wobei das C₃-C₁₀-Cycloalkyl gegebenenfalls durch ein oder mehrere -(CO)-, -SO- oder -SO₂-Gruppen im Ring unterbrochen ist und gegenenfalls ein oder mehrere Doppelbindungen im Ring enthalten sein können, oder für gegebenenfalls ein- oder mehrfach, gleich oder verschieden mit K substituiertes Aryl oder Heteroaryl steht, wobei das Heteroaryl selbst durch mindestens ein Stickstoff, Sauerstoff und/oder Schwefel unterbrochen ist und das Heteroaryl nur über ein Kohlenstoffringatom mit dem Pyrazolopyridin verknüpft sein kann, oder für ein mit der Gruppe -O-(CH₂)ₙ-O- substituiertes C₃-C₁₀-Cycloalkyl, Aryl oder Heteroaryl steht, wobei die endständigen Sauerstoffatome der -O-(CH₂)ₙ-O- Gruppe mit einem gleichen oder direkt benachbarten C₃-C₁₀-Cycloalkylring-, Arylring- oder Heteroarylring-Kohlenstoffatom verknüpft sind.

In einem weiteren Gegenstand der Erfindung gemäß dieser Ausführungsform sind Verbindungen der allgemeinen Formel (I), gemäß einem der Ansprüche 1 bis 8, bei der R¹ für gegebenenfalls ein- oder mehrfach, gleich oder verschieden mit K substituiertes C₁-C₆ alkyl, C₃-C₁₀-Cycloalkyl steht, wobei das C₃-C₁₀-Cycloalkyl gegebenenfalls durch ein oder mehrere -(CO)-Gruppen im Ring unterbrochen ist, oder für gegebenenfalls einoder mehrfach, gleich oder verschieden mit K substituiertes Aryl oder Heteroaryl steht, wobei das Heteroaryl selbst durch mindestens ein Stickstoff unterbrochen ist und das Heteroaryl nur über ein Kohlenstoffringatom mit dem Pyrazolopyridin verknüpft sein kann, oder für ein mit der Gruppe -O-(CH₂)ₙ-O- substituiertes C₃-C₁₀-Cycloalkyl, Aryl oder Heteroaryl steht, wobei die endständigen Sauerstoffatome der -O-(CH₂)ₙ-O-Gruppe mit einem gleichen oder direkt benachbarten C₃-C₁₀-Cycloalkylring-, Arylringoder Heteroarylring-Kohlenstoffatom verknüpft sind.

Ein bevorzugter Gegenstand der Erfindung gemäß dieser Ausführungsform sind Verbindungen der allgemeinen Formel (I), gemäß einem der Ansprüche 1 bis 8, bei der R¹ für gegebenenfalls ein- oder mehrfach, gleich oder verschieden mit K substituiertes C₁-C₆-Alkyl, C₃-C₆-Cycloalkyl, Cyclohexanon, 1,4-Dioxa-spiro[4.5]dec-8-yl, Phenyl, 1,3-Benzodioxolyl oder Pyridinyl steht.

Ein bevorzugter Gegenstand der Erfindung gemäß dieser Ausführungsform sind Verbindungen der allgemeinen Formel (I), gemäß einem der Ansprüche 1 bis 8, bei der R¹ für gegebenenfalls ein- oder mehrfach, gleich oder verschieden mit K substituiertes C₃-C₆ Alkyl, Cyclopropyl, Cyclohexyl, Cyclohexanon, 1,4-Dioxa-spiro[4.5]dec-8-yl, Phenyl, 1,3-Benzodioxolyl oder Pyridinyl steht.

Ein bevorzugter Gegenstand der Erfindung gemäß dieser Ausführungsform sind Verbindungen der allgemeinen Formel (I), gemäß einem der Ansprüche 1 bis 8, bei der R¹ für gegebenenfalls ein- oder mehrfach, gleich oder verschieden mit K substituiertes *tert-*Butyl, Cyclopropyl, Cyclohexyl, Cyclohexanon, 1,4-Dioxa-spiro[4.5]dec-8-yl, Phenyl, 1,3-Benzodioxolyl oder Pyridinyl steht.

Ein besonders bevorzugter Gegenstand der Erfindung gemäß dieser Ausführungsform sind Verbindungen der allgemeinen Formel (I), gemäß einem der Ansprüche 1 bis 8, bei der R¹ für gegebenenfalls ein- oder mehrfach, gleich oder verschieden mit K substituiertes C₁-C₆-Alkyl und besonders bevorzugt *tert*-Butyl steht.

Ein besonders bevorzugter Gegenstand der Erfindung gemäß dieser Ausführungsform sind Verbindungen der allgemeinen Formel (I), gemäß einem der Ansprüche 1 bis 8, bei der R¹ für gegebenenfalls ein- oder mehrfach, gleich oder verschieden mit K substituiertes C₃-C₆-Cycloalkyl und besonders bevorzugt Cyclopropyl und/oder Cyclohexyl steht.

Ein besonders bevorzugter Gegenstand der Erfindung gemäß dieser Ausführungsform sind Verbindungen der allgemeinen Formel (I), gemäß einem der Ansprüche 1 bis 8, bei der R¹ für gegebenenfalls ein- oder mehrfach, gleich oder verschieden mit K substituiertes C₃-C₁₀-Cycloalkyl welches durch ein oder mehrere -(CO)-Gruppen im Ring unterbrochen ist, steht. Besonders bevorzugt steht R¹ dann für Cyclohexanon.

Ein besonders bevorzugter Gegenstand der Erfindung gemäß dieser Ausführungsform sind Verbindungen der allgemeinen Formel (I), gemäß einem der Ansprüche 1 bis 8, bei der R¹ für ein mit der Gruppe -O-(CH₂)ₙ-O- substituiertes C₃-C₁₀-Cycloalkyl, Aryl oder Heteroaryl steht, wobei die endständigen Sauerstoffatome der -O-(CH₂)ₙ-O- Gruppe mit einem gleichen oder direkt benachbarten C₃-C₁₀-Cycloalkylring-, Arylring- oder Heteroarylring-Kohlenstoffatom verknüpft sind. Besonders bevorzugt steht dann R¹ für 1,4-Dioxa-spiro[4.5]dec-8-yl und/oder 1,3-Benzodioxolyl steht.

Ein besonders bevorzugter Gegenstand der Erfindung gemäß dieser Ausführungsform sind Verbindungen der allgemeinen Formel (I), gemäß einem der Ansprüche 1 bis 8, bei der R¹ für gegebenenfalls ein- oder mehrfach, gleich oder verschieden mit K substituiertes Aryl oder Heteroaryl, wobei das Heteroaryl selbst durch mindestens ein Stickstoff unterbrochen ist und das Heteroaryl nur über ein Kohlenstoffringatom mit dem Pyrazolopyridin verknüpft sein kann, steht. Besonders bevorzugt steht dann R¹ für Phenyl und/oder Pyridinyl.

Ein weiterer Gegenstand der Erfindung gemäß dieser Ausführungsform sind Verbindungen der allgemeinen Formel (I), gemäß einem der Ansprüche 1 bis 8, in der R¹ für ein C₃-C₁₀-Heterocycloalkyl und/oder Heteroaryl steht, wobei das C₃-C₁₀-Heterocycloalkyl und/oder Heteroaryl nur über ein Kohlenstoffringatom mit dem Pyrazolopyridin verknüpft sein.

Ein besonders bevorzugter Gegenstand der Erfindung gemäß dieser Ausführungsform sind Verbindungen der allgemeinen Formel (I), gemäß einem der Ansprüche 1 bis 8, bei der R¹ ein- oder mehrfach, gleich oder verschieden mit K substituiert sind. Ein besonders bevorzugter Gegenstand der Erfindung gemäß dieser Ausführungsform sind Verbindungen der allgemeinen Formel (I), gemäß einem der Ansprüche 1 bis 8, bei der R¹ einfach mit K substituiert ist.

Ein Gegenstand der Erfindung gemäß dieser Ausführungsform sind Verbindungen der allgemeinen Formel (I), gemäß einem der Ansprüche 1 bis 8, bei der K steht für Halogen, Hydroxy oder die Gruppe -OR³, -COR⁴ oder -NR⁵R⁶ oder für gegebenenfalls ein- oder mehrfach, gleich oder verschieden mit L substituiertes C₃-C₁₀-Cycloalkyl, C₃-C₁₀-Heterocycloalkyl, Aryl oder Heteroaryl steht, wobei das C₃-C₁₀-Heterocycloalkyl und/ oder das Heteroaryl selbst durch mindestens eins der folgenden Atome Stickstoff, Sauerstoff und/oder Schwefel im Ring unterbrochen ist und C₃-C₁₀-Cycloalkyl und/oder C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthalten sein können.

Ein weiterer Gegenstand der vorliegenden Erfindung gemäß dieser Ausführungsform sind Verbindungen der allgemeinen Formel (I), gemäß einem der Ansprüche 1 bis 8, bei der K für Halogen, Hydroxy oder die Gruppe -OR³, -COR⁴ oder für ein gegebenenfalls ein- oder mehrfach, gleich oder verschieden mit L substituiertes C₃-C₁₀-Cycloalkyl, C₃-C₁₀-Heterocycloalkyl, Aryl oder Heteroaryl steht, wobei das C₃-C₁₀-Heterocycloalkyl und/ oder das Heteroaryl selbst durch mindestens eins der folgenden Atome Stickstoff, Sauerstoff und/oder Schwefel im Ring unterbrochen ist und C₃-C₁₀-Cycloalkyl und/oder C₃-C₁₀-Heterocycloalkyl gegebenenfalls durch ein oder mehrere, gleich oder verschiedene -(CO)-, -SO- oder-SO₂- Gruppen im Ring unterbrochen sein kann und gegebenenfalls ein oder mehrere Doppelbindungen im Ring enthalten sein können.

Ein weiterer Gegenstand der vorliegenden Erfindung gemäß dieser Ausführungsform sind Verbindungen der allgemeinen Formel (I), gemäß einem der Ansprüche 1 bis 8, bei der K für Halogen, Hydroxy oder die Gruppe -OR³, -COR⁴ oder für ein gegebenenfalls ein- oder mehrfach, gleich oder verschieden mit L substituiertes C₃-C₁₀-Cycloalkyl,oder C₃- C₁₀-Heterocycloalkyl steht, wobei das C₃-C₁₀-Heterocycloalkyl selbst durch mindestens eins der folgenden Atome Stickstoff, Sauerstoff und/oder Schwefel im Ring unterbrochen ist und C₃-C₁₀-Cycloalkyl und/oder C₃-C₁₀-Heterocycloalkyl gegebenenfalls durch ein oder mehrere, gleich oder verschiedene -(CO)-, -SO- oder-SO₂- Gruppen im Ring unterbrochen sein kann und gegebenenfalls ein oder mehrere Doppelbindungen im Ring enthalten sein können.

Ein bevorzugter Gegenstand der Erfindung gemäß dieser Ausführungsform sind Verbindungen der allgemeinen Formel (I), gemäß einem der Ansprüche 1 bis 8, bei der K für Halogen, Hydroxy oder für die Gruppe -OR³, oder für gegebenenfalls ein- oder mehrfach, gleich oder verschieden mit L substituiertes C₃-C₁₀-Heterocycloalkyl steht, wobei das C₃-C₁₀-Heterocycloalkyl selbst durch mindestens eins der folgenden Atome Stickstoff und/oder Sauerstoff im Ring unterbrochen ist.

Ein bevorzugter Gegenstand der Erfindung gemäß dieser Ausführungsform sind Verbindungen der allgemeinen Formel (I), gemäß einem der Ansprüche 1 bis 8, bei der K für Halogen, Hydroxy, C₁-C₆-Alkoxy oder gegebenenfalls ein- oder mehrfach, gleich oder verschieden mit L substituiertes Morpholinyl, Piperazinyl, Piperidinyl, oder Phenoxy steht.

Ein bevorzugter Gegenstand der Erfindung gemäß dieser Ausführungsform sind Verbindungen der allgemeinen Formel (I), gemäß einem der Ansprüche 1 bis 8, bei der K für für Halogen, Hydroxy, Methoxy oder gegebenenfalls ein- oder mehrfach, gleich oder verschieden mit L substituiertes Morpholinyl, Piperazinyl, Piperidinyl oder Phenoxy steht.

Ein Gegenstand der Erfindung gemäß dieser Ausführungsform sind Verbindungen der allgemeinen Formel (I), gemäß einem der Ansprüche 1 bis 8, bei der L für C₁-C₆-Alkyl oder die Gruppe -COR⁴, -OR³ oder -NR⁵R⁶ steht. Ein weiterer Gegenstand der Erfindung sind gemäß dieser Ausführungsform Verbindungen der allgemeinen Formel (I), gemäß einem der Ansprüche 1 bis 8, bei der L für C₁-C₆-Alkyl oder die Gruppe - 1 COR⁴ oder -OR³ steht. Ein weiterer Gegenstand der vorliegenden Erfindung gemäß dieser Ausführungsform sind Verbindungen der allgemeinen Formel (I), gemäß einem der Ansprüche 1 bis 8, bei der L für C₁-C₆-Alkyl oder -COO-C₁-C₆ Alkyl steht. Ein bevorzugter Gegenstand der Erfindung gemäß dieser Ausführungsform sind Verbindungen der allgemeinen Formel (I), gemäß einem der Ansprüche 1 bis 8, bei der L für C₁-C₃-Alkyl oder -COO-C₃-C₅-Alkyl steht. Ein weiterer bevorzugter Gegenstand der Erfindung gemäß dieser Ausführungsform sind Verbindungen der allgemeinen Formel (I), gemäß einem der Ansprüche 1 bis 8, bei der L für Methyl oder -COO-*tert-*Butyl steht.

Ein Gegenstand der Erfindung gemäß dieser Ausführungsform sind Verbindungen der allgemeinen Formel (I), gemäß einem der Ansprüche 1 bis 8, bei der R² für gegebenenfalls ein- oder mehrfach, gleich oder verschieden mit M substituiertes C₁-C₆-Alkyl, C₃-C₁₀-Cycloalkyl, Aryl oder Heteroaryl steht.

Ein weiterer Gegenstand der vorliegenden Erfindung gemäß dieser Ausführungsform sind Verbindungen der allgemeinen Formel (I), gemäß einem der Ansprüche 1 bis 8, bei der R² für gegebenenfalls ein- oder mehrfach, gleich oder verschieden mit M substituiertes C₁-C₆-Alkyl, C₃-C₁₀-Cycloalkyl, Aryl oder Heteroaryl, wobei das Heteroaryl durch mindestens einen Stickstoff, Sauerstoff und/oder Schwefel unterbrochen ist, steht.

Ein weiterer Gegenstand der vorliegenden Erfindung gemäß dieser Ausführungsform sind Verbindungen der allgemeinen Formel (I), gemäß einem der Ansprüche 1 bis 8, bei der R² für gegebenenfalls ein- oder mehrfach, gleich oder verschieden mit M substituiertes C₁-C₆ Alkyl, C₃-C₆-Cycloalkyl, Aryl oder Heteroaryl, wobei das Heteroaryl durch mindestens einen Stickstoff unterbrochen ist, steht.

Ein bevorzugter Gegenstand der vorliegenden Erfindung gemäß dieser Ausführungsform sind Verbindungen der allgemeinen Formel (I), gemäß einem der Ansprüche 1 bis 8, bei der R² für gegebenenfalls ein- oder mehrfach, gleich oder verschieden mit M substituiertes C₁-C₆-Alkyl, Phenyl oder Chinolinyl, Imidazolyl, Indolyl oder Pyridinyl steht.

Ein weiterer bevorzugter Gegenstand der vorliegenden Erfindung gemäß dieser Ausführungsform sind Verbindungen der allgemeinen Formel (I), gemäß einem der Ansprüche 1 bis 8, bei der R² für gegebenenfalls ein- oder mehrfach, gleich oder verschieden mit M substituiertes Isopropyl, Phenyl, Chinolinyl, Imidazolyl, Indolyl oder Pyridinyl, steht.

Ein weiterer bevorzugter Gegenstand der vorliegenden Erfindung gemäß dieser Ausführungsform sind Verbindungen der allgemeinen Formel (I), gemäß einem der Ansprüche 1 bis 8, bei der R² für gegebenenfalls ein- oder mehrfach, gleich oder verschieden mit M substituiertes Isopropyl, Phenyl, Chinolinyl, Imidazolyl, Indolyl oder Pyridinyl steht.

Ein Gegenstand der Erfindung gemäß dieser Ausführungsform sind Verbindungen der allgemeinen Formel (I), gemäß einem der Ansprüche 1 bis 8, bei der R³ für gegebenenfalls ein- oder mehrfach, gleich oder verschieden mit der Gruppe -NR⁵R⁶ substituiertes C₁-C₆-Alkyl, Aryl oder -(CH₂)ₙ-Aryl steht. Ein weiterer Gegenstand der vorliegenden Erfindung gemäß dieser Ausführungsform sind Verbindungen der allgemeinen Formel (I), gemäß einem der Ansprüche 1 bis 8, bei der R³ für C₁-C₆-Alkyl, Aryl oder -(CH₂)ₙ-Aryl steht. Ein weiterer Gegenstand der vorliegenden Erfindung gemäß dieser Ausführungsform sind Verbindungen der allgemeinen Formel (I), gemäß einem der Ansprüche 1 bis 8, bei der R³ für C₁-C₆-Alkyl oder Aryl steht. Ein bevorzugter Gegenstand der vorliegenden Erfindung gemäß dieser Ausführungsform sind Verbindungen der allgemeinen Formel (I), gemäß einem der Ansprüche 1 bis 8, bei der R³ für C₁-C₆-Alkyl oder Phenyl steht. Ein weiterer bevorzugter Gegenstand der vorliegenden Erfindung gemäß dieser Ausführungsform sind Verbindungen der allgemeinen Formel (I), gemäß einem der Ansprüche 1 bis 8, bei der R³ für C₁-C₃-Alkyl oder Phenyl steht. Ein weiterer bevorzugter Gegenstand der vorliegenden Erfindung gemäß dieser Ausführungsform sind Verbindungen der allgemeinen Formel (I), gemäß einem der Ansprüche 1 bis 8, bei der R³ für C₁-C₃-Alkyl steht.

Ein Gegenstand der Erfindung gemäß dieser Ausführungsform sind Verbindungen der allgemeinen Formel (I), gemäß einem der Ansprüche 1 bis 8, bei der R⁴ für Wasserstoff, Hydroxy, C₁-C₆-Alkyl oder C₁-C₆-Alkoxy. Ein weiterer Gegenstand der vorliegenden Erfindung gemäß dieser Ausführungsform sind Verbindungen der allgemeinen Formel (I), gemäß einem der Ansprüche 1 bis 8, bei der R⁴ für Wasserstoff, Hydroxy, C₁-C₃-Alkyl oder C₁-C₃-Alkoxy steht.

Ein Gegenstand der Erfindung gemäß dieser Ausführungsform sind Verbindungen der allgemeinen Formel (I), gemäß einem der Ansprüche 1 bis 8, bei der M für Amino, Cyano, Halogen, Hydroxy, Nitro oder für gegebenenfalls ein- oder mehrfach, gleich oder verschieden mit Amino, Cyano, Halogen, Hydroxy, Nitro, C₁-C₆-Alkoxy substituiertes C₁-C₆-Alkyl, C₂-C₆-Alkenyl, C₂-C₆-Alkinyl oder für die Gruppe -OR³,-COR⁴ oder -CO-N-R⁷ steht.

Ein weiterer Gegenstand der vorliegenden Erfindung gemäß dieser Ausführungsform sind Verbindungen der allgemeinen Formel (I), gemäß einem der Ansprüche 1 bis 8, bei der M für Amino, Cyano, Halogen, Hydroxy, Nitro oder für gegebenenfalls ein- oder mehrfach, gleich oder verschieden mit Amino, Cyano, Halogen, Hydroxy, Nitro, C₁-C₆-Alkoxy substituiertes C₁-C₆-Alkyl oder für die Gruppe -O-C₁-C₆-Alkyl, -O-(CH₂)ₙ-NR⁵R⁶, -COR⁴,-O-Phenyl, -O-(CH₂)ₙ-Phenyl oder -CO-N-R⁷ steht. Ein weiterer bevorzugter Gegenstand der vorliegenden Erfindung sind Verbindungen der allgemeinen Formel (I), gemäß einem der Ansprüche 1 bis 8, bei der M für Amino, Cyano, Halogen, Hydroxy, Nitro oder für gegebenenfalls ein- oder mehrfach, gleich oder verschieden mit Amino, Cyano, Halogen, Hydroxy, Nitro, C₁-C₆-Alkoxy substituiertes C₁-C₆-Alkyl oder für die Gruppe -O-C₁-C₆-Alkyl, -O-(CH₂)ₙ-NR⁵R⁶, -COR⁴ oder -CO-N-R⁷ steht.

Ein weiterer Gegenstand der vorliegenden Erfindung gemäß dieser Ausführungsform sind Verbindungen der allgemeinen Formel (I), gemäß einem der Ansprüche 1 bis 8 , bei der M für Amino, Cyano, Halogen, Hydroxy, Nitro oder für gegebenenfalls ein- oder mehrfach, gleich oder verschieden mit Amino, Cyano, Halogen, Hydroxy, Nitro, C₁-C₆-Alkoxy substituiertes C₁-C₆-Alkyl oder für die Gruppe -O-C₁-C₆-Alkyl, -O-(CH₂)ₙ-NR⁵R⁶, -CO-C₁-C₆-Alkyl, -O-Phenyl, -O-(CH₂)ₙ-Phenyl oder -CO-N-R⁷ steht. Ein weiterer bevorzugter Gegenstand der vorliegenden Erfindung gemäß dieser Ausführungsform sind Verbindungen der allgemeinen Formel (I), gemäß einem der Ansprüche 1 bis 8, bei der M für Amino, Cyano, Halogen, Hydroxy, Nitro oder für gegebenenfalls ein- oder mehrfach, gleich oder verschieden mit Amino, Cyano, Halogen, Hydroxy, Nitro, C₁-C₆-Alkoxy substituiertes C₁-C₆-Alkyl oder für die Gruppe -O-C₁-C₆-Alkyl, -O-(CH₂)ₙ-NR⁵R⁶ -CO-C₁-C₆-Alkyl oder-CO-N-R⁷ steht.

Ein weiterer Gegenstand der vorliegenden Erfindung gemäß dieser Ausführungsform sind Verbindungen der allgemeinen Formel (I), gemäß einem der Ansprüche 1 bis 8, bei der M für Amino, Cyano, Halogen, Hydroxy, Nitro oder für gegebenenfalls ein- oder mehrfach, gleich verschieden mit Amino, Cyano, Halogen, Hydroxy, Nitro oder C₁-C₆-Alkoxy substituiertes C₁-C₆-Alkyl oder für die Gruppe -O-C₁-C₆-Alkyl, -O-(CH₂)ₙ-N(C₁-C₆-Alkyl)₂, -CO-C₁-C₆-Alkyl, Phenoxy, Benzyloxy oder-CO-N-NH₂ steht Ein weiterer bevorzugter Gegenstand der vorliegenden Erfindung gemäß dieser Ausführungsform sind Verbindungen der allgemeinen Formel (I), gemäß einem der Ansprüche 1 bis 8, bei der M für Amino, Cyano, Halogen, Hydroxy, Nitro oder für gegebenenfalls ein- oder mehrfach, gleich verschieden mit Amino, Cyano, Halogen, Hydroxy, Nitro oder C₁-C₆-Alkoxy substituiertes C₁-C₆-Alkyl oder für die Gruppe -O-C₁-C₆-Alkyl, -O-(CH₂)ₙ-N(C₁-C₆-Alkyl)₂, -CO-C₁-C₆-Alkyl oder-CO-N-NH₂ bevorzugt ohne -CO-N-NH₂ steht.

Ein weiterer bevorzugter Gegenstand der vorliegenden Erfindung gemäß dieser Ausführungsform sind Verbindungen der allgemeinen Formel (I), gemäß einem der Ansprüche 1 bis 8, bei der M auch für Amino, Cyano, Halogen, Hydroxy, Nitro, C₁-C₆-Alkyl, -CF₃, oder für C₁-C₃-Alkoxy oder für die Gruppe -CO-C₁-C₆-Alkyl, -O-(CH₂)ₙ-N(C₁-C₆-Alkyl)₂, Phenoxy oder Benzyloxy steht. Ein weiterer bevorzugter Gegenstand der vorliegenden Erfindung gemäß dieser Ausführungsform sind Verbindungen der allgemeinen Formel (I), gemäß einem der Ansprüche 1 bis 8, bei der M für Amino, Cyano, Halogen, Hydroxy, Nitro, C₁-C₆-Alkyl, -CF₃, oder für C₁-C₃-Alkoxy steht oder für die Gruppe -CO-C₁-C₆-Alkyl oder -O-(CH₂)ₙ-N(C₁-C₆-Alkyl)₂ steht.

Ein weiterer bevorzugter Gegenstand der vorliegenden Erfindung gemäß dieser Ausführungsform sind Verbindungen der allgemeinen Formel (I), gemäß einem der Ansprüche 1 bis 8, bei der M für Cyano, Halogen, Hydroxy, Nitro, Methyl, -CF₃ oder für Methoxy oder für die Gruppe -CO-C₁-C₃-Alkyl oder -O-(CH₂)₃-N(Methyl)₂ steht.

Ein weiterer bevorzugter Gegenstand der vorliegenden Erfindung gemäß dieser Ausführungsform sind Verbindungen der allgemeinen Formel (I), gemäß einem der Ansprüche 1 bis 8, bei der M auch für Cyano, Halogen, Hydroxy, Nitro, Methyl, -CF₃ oder für Methoxy oder für die Gruppe -CO-Methyl oder-O-(CH₂)₃-N(Methyl) steht.

Ein weiterer Gegenstand der vorliegenden Erfindung gemäß dieser Ausführungsform sind Verbindungen der allgemeinen Formel (I), gemäß einem der Ansprüche 1 bis 8, bei der R² gemäß der allgemeinen Formel (I) ein- oder mehrfach, gleich oder verschieden mit M substituiert ist. Ein weiterer Gegenstand der vorliegenden Erfindung sind Verbindungen der allgemeinen Formel (I), gemäß einem der Ansprüche 1 bis 8, bei der R² ein Aryl oder Heteroaryl ist und das Aryl oder Heteroaryl ein- oder mehrfach, gleich oder verschieden mit M substituiert ist. Ein weiterer Gegenstand der vorliegenden Erfindung sind Verbindungen der allgemeinen Formel (I), gemäß einem der Ansprüche 1 bis 8, bei der R² ein Aryl oder Heteroaryl ist und das Aryl oder Heteroaryl ein- oder mehrfach, gleich oder verschieden und zumindest einmal in Meta-Position mit M substituiert ist. Ein weiterer bevorzugter Gegenstand der vorliegenden Erfindung sind Verbindungen der allgemeinen Formel (I), gemäß einem der Ansprüche 1 bis 8, bei der R² ein Aryl ist und das Aryl ein oder mehrfach, gleich oder verschieden und zumindest einmal in Meta-Position mit M substituiert ist. Ein weiterer bevorzugter Gegenstand der vorliegenden Erfindung sind Verbindungen der allgemeinen Formel (I), gemäß einem der Ansprüche 1 bis 8, bei der R² ein Phenyl ist und ein oder mehrfach, gleich oder verschieden und zumindest einmal in Meta-Position mit M substituiert ist. Ein weiterer Gegenstand der vorliegenden Erfindung sind Verbindungen der allgemeinen Formel (I), gemäß einem der Ansprüche 1 bis 8, bei der R² mehrfach, gleich oder verschieden, und zumindest einmal in Meta-Position mit M substituiert ist. Ein weiterer bevorzugter Gegenstand der vorliegenden Erfindung sind Verbindungen der allgemeinen Formel (I), gemäß einem der Ansprüche 1 bis 8, bei der R² mehrfach, gleich oder verschieden und zweimal in Meta-Position mit M substituiert ist. Ein weiterer bevorzugter Gegenstand der vorliegenden Erfindung sind Verbindungen der allgemeinen Formel (I), gemäß einem der Ansprüche 1 bis 8, bei der R² gleich und zweimal in Meta-Position mit M substituiert ist. Ein weiterer bevorzugter Gegenstand der vorliegenden Erfindung sind Verbindungen der allgemeinen Formel (I), gemäß einem der Ansprüche 1 bis 8, bei der R² einfach an der Meta-Position mit M substituiert ist.

Ein Gegenstand der Erfindung gemäß dieser Ausführungsform sind Verbindungen der allgemeinen Formel (I), gemäß einem der Ansprüche 1 bis 8, bei der R⁵ und R⁶ unabhängig voneinander für Wasserstoff, C₁-C₆-Alkyl oder für die Gruppe -COR⁴ stehen. Ein weiterer Gegenstand der vorliegenden Erfindung sind Verbindungen der allgemeinen Formel (I), gemäß einem der Ansprüche 1 bis 8, bei der R⁵ und R⁶ unabhängig voneinander für Wasserstoff oder C₁-C₆-Alkyl stehen.

R⁷ gemäß der allgemeinen Formel (I), gemäß einem der Ansprüche 1 bis 8,steht für Wasserstoff oder NH₂.

Ein Gegenstand der Erfindung gemäß dieser Ausführungsform sind Verbindungen der allgemeinen Formel (I), gemäß einem der Ansprüche 1 bis 8, bei der n für 1 bis 4 steht. Ein weiterer Gegenstand der vorliegenden Erfindung gemäß dieser Ausführungsform sind Verbindungen der allgemeinen Formel (I), gemäß einem der Ansprüche 1 bis 8, bei der n 1 bis 3 bedeutet. Ein weiterer Gegenstand der vorliegenden Erfindung gemäß dieser Ausführungsform sind Verbindungen der allgemeinen Formel (I), gemäß einem der Ansprüche 1 bis 8, bei der n 1 bis 2 bedeutet. Ein weiterer Gegenstand der vorliegenden Erfindung gemäß dieser Ausführungsform sind Verbindungen der allgemeinen Formel (I), gemäß einem der Ansprüche 1 bis 8, bei der n 1 bedeutet..

Ein weiterer Gegenstand der vorliegenden Erfindung gemäß dieser Ausführungsform ist die Verwendung der Verbindungen der allgemeinen Formel I in Anspruch 9, gemäß einem der Ansprüche 1 bis 8, wobei wenn
- R¹: für Alkyl, Alkenyl, Aryl, Aralkyl, Cycloalkyl, für ein mit Phenyl substituiertes Alkyl oder p-Methoxyphenyl steht, dann darf R² gleichzeitig auch für Alkyl, Alkenyl, Aryl, Aralkyl, Cyano, heterocyclischer Rest oder Cycloalkyl stehen,oder wenn
- R¹: für einen C₁-C₆-Alkylrest, Alkoxy oder Aryloxy steht, dann darf R² auch gleichzeitig für ein C₁-C₆-Alkylrest stehen,
zur Herstellung eines Arzneimittels.

Ein weiterer bevorzugter Gegenstand der vorliegenden Erfindung gemäß dieser Ausführungsform ist die Verwendung der Verbindungen der allgemeinen Formel I in Anspruch 10, gemäß einem der Ansprüche 1 bis 8 zur Herstellung eines Arzneimittels.

Ein weiterer Gegenstand der vorliegenden Erfindung gemäß dieser Ausführungsform ist die Verwendung der Verbindungen der allgemeinen Formel I in Anspruch 11, gemäß einem der Ansprüche 1 bis 8, in der
- R¹: für gegebenenfalls ein- oder mehrfach, gleich oder verschieden mit K substituiertes C₁-C₆-Alkyl, C₂-C₆-Alkenyl, C₂-C₆-Alkinyl, C₃-C₁₀-Cycloalkyl, C₃-C₁₀-Heterocycloalkyl, Aryl oder Heteroaryl steht, wobei das C₃-C₁₀-Heterocycloalkyl und/oder das Heteroaryl selbst durch mindestens eins der folgenden Atome Stickstoff, Sauerstoff und/oder Schwefel im Ring unterbrochen ist und das C₃-C₁₀-Heterocycloalkyl und/oder Heteroaryl nur über ein Kohlenstoffringatom mit dem Pyrazolopyridin verknüpft sein kann, steht und C₃-C₁₀-Cycloalkyl und/oder C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthalten sein können, oder für ein mit der Gruppe -O-(CH₂)n-O-substituiertes C₃-C₁₀-Cycloalkyl, Aryl, C₃-C₁₀-Heterocycloalkyl oder Heteroaryl steht, wobei die endständigen Sauerstoffatome der -O-(CH₂)ₙ-O- Gruppe mit einem gleichen oder direkt benachbarten C₃-C₁₀-Cycloalkylring-. Arylring-, C₃-C₁₀-Heterocycloalkyring- oder Heteroarylring-Kohlenstoffatom verknüpft sind.
- K: für Halogen, Hydroxy oder die Gruppe -OR³, COR⁴ oder -NR⁵R⁶ steht oder für gegebenenfalls ein- oder mehrfach, gleich oder verschieden mit L substituiertes C₃-C₁₀-Cycloalkyl, C₃-C₁₀-Heterocycloalkyl, Aryl oder Heteroaryl steht, wobei das C₃-C₁₀-Heterocycloalkyl und/oder das Heteroaryl selbst durch mindestens eins der folgenden Atome Stickstoff, Sauerstoff und/oder Schwefel im Ring unterbrochen ist und C₃-C₁₀-Cycloalkyl und/oder C₃-C₁₀-Heterocycloalkyl gegebenenfalls durch ein oder mehrere, gleich oder verschiedene -(CO)-, -SO-oder -SO₂- Gruppen im Ring unterbrochen sein kann und gegebenenfalls ein oder mehrere Doppelbindungen im Ring enthalten sein können,
- L: für C₁-C₆-Alkyl oder die Gruppe -COR⁴, -OR³ oder -NR⁵R⁶ steht,
- R²: für gegebenenfalls ein- oder mehrfach, gleich oder verschieden mit M substituiertes C₁-C₆-Alkyl, C₃-C₁₀-Cycloalkyl, Aryl oder Heteroaryl steht,
- R³: für gegebenenfalls ein- oder mehrfach, gleich oder verschieden mit der Gruppe-NR⁵R⁶ substituiertes C₁-C₆-Alkyl, Aryl oder -(CH₂)ₙ-Aryl steht,
- R⁴: für Wasserstoff, Hydroxy, C₁-C₆-Alkyl oder C₁-C₆-Alkoxy steht,
- M: für Amino, Cyano, Halogen, Hydroxy, Nitro oder für gegebenenfalls ein- oder mehrfach, gleich oder verschieden mit Amino, Cyano, Halogen, Hydroxy, Nitro, C₁-C₆-Alkoxy substituiertes C₁-C₆-Alkyl, C₂-C₆-Alkenyl, C₂-C₆-Alkinyl steht oder für die Gruppe -O-R³, -COR⁴ oder -CO-N-R⁷ steht,
- R⁵ und R⁶: unabhängig voneinander für Wasserstoff, C₁-C₆-Alkyl oder für die Gruppe-COR⁴ stehen und
- R⁷: Wasserstoff oder NH₂ steht,
- n: für 1 bis 4 steht,
wobei wenn:
- R¹: für Methyl steht, dann darf R² gleichzeitig auch für Methyl, -CH₂-O-CH₃, Phenyl, Chlorphenyl, mit Hydroxy und/oder Methoxy substituiertes Benzofuranyl, -CF₃ oder Furanyl stehen oder wenn
- R¹: für -CH₂-O-CH₃ steht, dann darf R² gleichzeitig auch für Methyl stehen oder wenn
- R¹: für -CH=CH-Phenyl steht, dann darf R² gleichzeitig auch für Phenyl stehen oder wenn
- R¹: für -CH=CH-Chlorphenyl steht, dann darf R² gleichzeitig auch für Phenyl oder Chlorphenyl stehen oder wenn
- R¹: für -CH=CH-Methoxyphenyl steht, dann darf R² gleichzeitig auch für Phenyl oder Methoxyphenyl stehen oder wenn
- R¹: für Phenyl steht, dann darf R² gleichzeitig auch für-CF₃, p-Methylphenyl, Methyl, Methoxyphenyl oder Phenyl stehen oder wenn
- R¹: für Methoxyphenyl steht, dann darf R² gleichzeitig auch für-CF₃ stehen oder wenn
- R¹: für Methylphenyl steht, dann darf R² gleichzeitig auch -CF₃ stehen oder wenn
- R¹: für Chlorphenyl steht, dann darf R² gleichzeitig auch für Chlorphenyl oder -CF₃ stehen oder wenn
- R¹: für Dichlorphenyl steht, dann darf R² gleichzeitig auch für Trimethoxyphenyl stehen oder wenn
- R¹: für Bromphenyl steht, dann darf R² gleichzeitig auch für Trimethoxyphenyl stehen oder wenn
- R¹: für Alkyl, Alkenyl, Aryl, Aralkyl, Cycloalkyl, für ein mit Phenyl substituiertes Alkyl oder p-Methoxyphenyl steht, dann darf R² gleichzeitig auch für Alkyl, Alkenyl, Aryl, Aralkyl, heterocyclischer Rest oder Cycloalkyl stehen oder wenn
- R¹: für einen C₁-C₆-Alkylrest steht, dann darf R² gleichzeitig auch für ein C₁-C₆-Alkylrest stehen oder wenn
- R¹: für Hydroxyphenyl steht, dann darf R² gleichzeitig auch für Heterocycyl oder mit-COO-*tert*-Eutyl substituiertes Heterocycloalkyl stehen oder wenn
- R¹: für Benzyloxyphenyl steht, dann darf R² gleichzeitig auch für mit -COO-*tert*-Butyl substituiertes Heterocycloalkyl stehen oder wenn
- R¹: für p-Methylphenylsteht, dann darf R² gleichzeitig für Phenyl stehen, oder wenn
- R¹: für Cyclopropyl steht, dann darf R² gleichzeitig für p-Methoxyphenyl stehen, oder wenn
- R¹: für ein C₃-C₁₀-Heterocycloalkyl oder ein Heteroaryl steht, dann darf das C₃-C₁₀-Heterocycloalkyl und/oder das Heteroaryl über ein Kohlenstoffringatom mit dem Pyrazolopyridin verknüpft sein,

zur Herstellung eines Arzneimittels zur Behandlung von Erkankungen, in denen die Angiogenese, Lymphangiogenese oder Vaskulogenese eine Rolle spielen, Erkrankungen der Blutgefäße, Erkankungen, die durch eine Hyperproliferation von Körperzellen hervorgerufen werden, sowie chronische oder akute neurodegenerative Erkrankungen.

Ein weiterer bevorzugter Gegenstand der vorliegenden Erfindung gemäß dieser Ausführungsform ist die Verwendung der Verbindungen der allgemeinen Formel I in Anspruch 12, gemäß Anspruch 11, zur Herstellung eines Arzneimittels zur Behandlung von Erkankungen, in denen die Angiogenese, Lymphangiogenese oder Vaskulogenese eine Rolle spielen, Erkankungen, die durch eine Hyperproliferation von Körperzellen hervorgerufen werden, sowie chronische oder akute neurodegenerative Erkrankungen.

Ein weiterer bevorzugter Gegenstand der vorliegenden Erfindung gemäß dieser Ausführungsform ist die Verwendung der Verbindungen der allgemeinen Formel I in Anspruch 13, gemäß einem der Ansprüche 1 bis 8 zur Herstellung eines Arzneimittels zur Behandlung von Erkankungen, in denen die Angiogenese, Lymphangiogenese oder Vaskulogenese eine Rolle spielen, Erkrankungen der Blutgefäße, Erkankungen, die durch eine Hyperproliferation von Körperzellen hervorgerufen werden, sowie chronische oder akute neurodegenerative Erkrankungen.

Ein weiterer Gegenstand der vorliegenden Erfindung gemäß dieser Ausführungsform ist die Verwendung der Verbindungen der allgemeinen Formel I in Anspruch 14, gemäß einem der Ansprüche 1 bis 8, für diagnostische Zwecke in vitro oder in vivo zur Identifizierung von Rezeptoren in Geweben mittels Autoradiographie oder PET.

Ein weiterer Gegenstand der Erfindung gemäß dieser Ausführungsform ist die Verwendung der Verbindungen der allgemeinen Formeln (II) und/oder (III) : 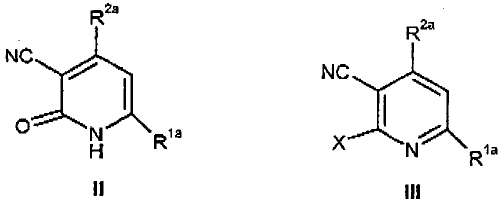 in denen
- X: für Halogen oder -O-SO₂-CₘF₂ₘ₊₁ vorzugsweise für Perfluoralkylsulfonyl steht,
- m: für 1 bis 4 steht, und
- R^{1a} und R^{2a}: die gleiche Bedeutung wie R¹ und R² gemäß einem der Ansprüche 1 bis 8 haben, wobei K jedoch zusätzlich auch für die Gruppe -COR⁴ und R³ zusätzlich auch für die Gruppe Trimethylsilyl (TMS), *tert*-Butyl-dimethylsilyl (TBDMS), *tert-*Butyl-diphenylsilyl (TBDPS), Triethylsilyl (TES), C₁-C₂-Alkyl, C₃-C₆-Allyl, Benzyl oder für die Gruppe -COR^{4a} stehen kann,
bedeuten, sowie deren Solvate, Hydrate, Stereoisomere, Diastereomere, Enantiomere und Salze als Zwischenprodukte zur Herstellung von Verbindungen der allgemeinen Formel (I),

in Anspruch 15 als Zwischenprodukte zur Herstellung von Verbindungen der allgemeinen Formel (I), gemäß Anspruch 1.

Ein weiterer Gegenstand der Erfindung gemäß dieser Ausführungsform sind Arzneimittel in Anspruch 16, die mindestens eine Verbindungen der allgemeinen Formel I gemäß einem der Ansprüche 1 bis 8 enthalten, wobei wenn
- R¹: für Alkyl, Alkenyl, Aryl, Aralkyl, Cycloalkyl, für ein mit Phenyl substituiertes Alkyl oder p-Methoxyphenyl steht, dann darf R² auch gleichzeitig auch für Alkyl, Alkenyl, Aryl, Aralkyl, Cyano, heterocyclischer Rest oder Cycloalkyl stehen,
- R¹: für einen C₁-C₆-Alkylrest, Alkoxy, Aryloxy oder eine primäre, sekundäre oder tertiäre Aminogruppe steht, dann darf R² auch gleichzeitig für ein C₁-C₆-Alkylrest stehen.

Ein weiterer bevorzugter Gegenstand der Erfindung gemäß dieser Ausführungsform sind Arzneimittel in Anspruch 17, die mindestens eine Verbindungen der allgemeinen Formel I gemäß einem der Ansprüche 1 bis 8 enthalten.

Ein weiterer bevorzugter Gegenstand der Erfindung gemäß dieser Ausführungsform sind Verbindungen gemäß den Ansprüchen 1 bis 8 oder Arzneimittel gemäß den Ansprüchen 17 oder 18 mit geeigneten Formulierungs- und Trägerstoffen in Anspruch 19.

Unter Alkyl ist jeweils ein geradkettiger oder verzweigter Alkylrest, wie beispielsweise Methyl, Ethyl, Propyl, Isopropyl, Butyl, Isobutyl, sek. Butyl, *tert*-Butyl, Pentyl, Isopentyl, Hexyl, Heptyl, Octyl, Nonyl und Decyl, zu verstehen.

Die Alkylgruppe von R¹ hat die im vorstehenden Absatz genannte Bedeutung, bevorzugt sind jedoch C₁-C₆-Alkylgruppen. Bevorzugt für die Alkylgruppe R¹ sind C₁-C₅-Alkylgruppen, noch bevorzugter sind C₃-C₅-Alkylgruppen und besonders bevorzugt ist ein C₄-Alkylgruppe, insbesondere eine *tert*-Butylgruppe. Die Alkylgruppe R² hat die im vorstehenden Absatz genannte Bedeutung, bevorzugt sind jedoch C₁-C₆-Alkylgruppen, besonders bevorzugt sind C₂-C₄-Alkylgruppen und am ganz besonders bevorzugt ist eine C₃-Alkylgruppe, insbesondere eine Isopropylgruppe. Die Alkylgruppe R³, R⁴, R⁵, R⁶, L und M haben die im vorstehenden Absatz genannte Bedeutung, bevorzugt sind jedoch C₁-C₆-Alkylgruppen, besonders bevorzugt sind C₁-C₃-Alkylgruppen und ganz besonders bevorzugt ist eine Methylgruppe.

Unter Alkoxy ist jeweils ein geradkettiger oder verzweigter Alkoxyrest, wie beispielsweise Methyloxy, Ethyloxy, Propyloxy, Isopropyloxy, Butyloxy, Isobutyloxy, sek. Butyloxy, Pentyloxy, Isopentyloxy, Hexyloxy, Heptyloxy, Octyloxy, Nonyloxy oder Decyloxy zu verstehen.

Die Alkoxygruppe K, R⁴ und M haben die im vorstehenden Absatz genannte Bedeutung, bevorzugt sind jedoch C₁-C₆- Alkoxygruppen, besonders bevorzugt sind C₁-C₃-Alkoxygruppen und besonders bevorzugt ist eine Methoxygruppe.

Die Alkenyl-Substituenten sind jeweils geradkettig oder verzweigt, wobei beispielsweise folgenden Reste gemeint sind: Vinyl, Propen-1-yl, Propen-2-yl, But-1-en-1-yl, But-1-en-2-yl, But-2-en-1-yl, But-2-en-2-yl, 2-Methyl-prop-2-en-1-yl, 2-Methyl-prop-1-en-1-yl, But-1-en-3-yl, But-3-en-1-yl, Allyl.

Unter Alkinyl ist jeweils ein geradkettiger oder verzweigter Alkinyl-Rest zu verstehen, der 2 - 6, bevorzugt 2 - 4 C-Atome enthält. Beispielsweise seien die folgenden Reste genannt: Acetylen, Propin-1-yl, Propin-3-yl, But-1-in-1-yl, But-1-in-4-yl, But-2-in-1-yl, But-1-in-3-yl, etc.

Heterocyclyl oder C₃-C₁₀-Heterocycloalkyl steht für einen 3 -10 Kohlenstoffatome, bevorzugt für einen 3 bis 10 Kohlenstoffatome und besonders bevorzugt für einen 5 bis 6 Kohlenstoffatome, umfassenden Alkylring, der durch mindestens eins der folgenden Atome Stickstoff, Sauerstoff und/oder Schwefel im Ring unterbrochen ist und der gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthalten sein können. Es sind allerdings nur diejenigen Kombinationen gemeint, die aus Sicht eines Fachmanns sinnvoll sind, insbesondere in Bezug auf die Ringspannung.
Als Heterocyclyle seien z. B. genannt: Oxiranyl, Oxethanyl, Aziridinyl, Azetidinyl, Tetrahydrofuranyl, Pyrrolidinyl, Dioxolanyl, Imidazolidinyl, Pyrazolidinyl, Dioxanyl, Piperidinyl, Morpholinyl, Dithianyl, Thiomorpholinyl, Piperazinyl, Trithianyl, Chinuclidinyl etc..

Unter Cycloalkyl sind monocyclische C₃ -C₁₀ Alkylringe wie Cyclopropyl, Cyclobutyl, Cyclopentyl, Cyclohexyl oder Cycloheptyl, aber auch bicyclische Ringe oder tricyclische Ringe wie zum Beispiel Adamantanyl und bei der ersten Ausführungsform der vorliegenden Erfindung zu verstehen. Die Cycloalkylringe können unsubstituiert oder ein- oder mehrfach substituiert sein. Cycloalkyle gemäß dieser Ausführungsform der Erfindung enthalten C₃-C₁₀ Kohlenwasserstoffatomen, bevorzugt sind Cycloalkyle mit C₃-C₆-Kohlenwasserstoffatomen.

Ein Arylrest hat jeweils 6 - 12 Kohlenstoffatome wie beispielsweise Naphthyl, Biphenyl und insbesondere Phenyl. Der Rest kann mono- oder bicyclisch, beispielsweise Naphthyl, Biphenyl und insbesondere Phenyl, sein.

Der Heteroarylrest umfaßt jeweils 3 - 16 Ringatome, bevorzugt 5 bis 10 Ringatome und besonders bevorzugt 5 bis 7 Ringatome, und enthält anstelle des Kohlenstoffs ein- oder mehrere, gleiche oder verschiedene Heteroatome aus der Gruppe Sauerstoff, Stickstoff oder Schwefel, kann mono-, bi- oder tricyclisch sein und kann zusätzlich jeweils benzokondensiert sein. Es sind allerdings nur diejenigen Kombinationen gemeint, die aus Sicht eines Fachmanns sinnvoll sind, insbesondere in Bezug auf die Ringspannung.

Die Heteroarylringe können unsubstituiert oder ein- oder mehrfach substituiert sein.

Beispielsweise seien genannt:
Thienyl, Furanyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, Pyridyl, Pyridazinyl, Pyrimidinyl, Pyrazinyl, Triazinyl sowie Benzoderivate davon, wie z. B. Benzofuranyl, Benzothienyl, Benzoxazolyl, Benzimidazolyl, Indazolyl, Indolyl, Isoindolyl, Oxepinyl, Azocinyl, Indolizinyl, Indolyl, Isoindolyl, Indazolyl, Benzimidazolyl, Purinyl, oder Chinolinyl, Isochinolinyl, Cinnolinyl, Phthalazinyl, Chinazolinyl, Chinoxalinyl, Naphthyridinyl, Pteridinyl, Carbazolyl, Acridinyl, Phenazinyl, Phenothiazinyl, Phenoxazinyl, Xanthenyl etc.

Unter Halogen ist jeweils Fluor, Chlor, Brom oder Jod zu verstehen. Bevorzugt ist ein Fluor-, Chlor- oder Bromatom. Besonders bevorzugt sind das Fluor- und das Chloratom.

So wie in dieser Anmeldung verwendet bezeichnet "C₁-C₆", zum Beispiel im Zusammenhang mit der Definition von "C₁-C₆-Alkyl" eine Alkylgruppe mit einer endlichen Anzahl von 1 bis 6 Kohlenstoffatomen, d.h. 1,2,3,4,5, oder 6 Kohlenstoffatome. Weiter wird die Definition "C₁-C₆" so interpretiert, dass jeder mögliche Teilbereich, wie beispielsweise, C₁-C₆, C₂-C₆, C₃-C₆, C₄-C₆, C₅-C₆, C₁-C₂, C₁-C₃, C₁-C₄, C₁-C₅, C₁-C₆ mitbeinhaltet ist.

Analog dazu bezeichnet "C₂-C₆", zum Beispiel in Zusammenhang mit der Definition von "C₂-C₆ Alkenyl" und "C₂-C₆-Alkinyl" eine Alkenylgruppe oder Alkinylgruppe mit einer endlichen Anzahl von 2 bis 10 Kohlenstoffatomen, d.h. 2,3,4,5 oder 6 Kohlenstoffatome. Die Definition "C₂-C₆" wird so interpretiert, dass jeder mögliche Teilbereich, wie beispielsweise C₂-C₆, C₃-C₆, C₄-C₆ , C₅-C₆, C₂-C₃, C₂-C₄, C₂-C₅, C₂-C₆, bevorzugt C₂-C₄, mitbeinhaltet ist.

Weiter bezeichnet "C₁-C₆", zum Beispiel in Zusammenhang mit der Definition von "C₁-C₆-Alkoxy" eine Alkoxygruppe mit einer endlichen Anzahl von 1 bis 6 Kohlenstoffatomen, d.h. 1,2,3,4,5 oder 6 Kohlenstoffatome. Die Definition "C₁-C₆" wird so interpretiert, dass jeder mögliche Teilbereich, wie beispielsweise C₁-C₆, C₂-C₅, C₃-C₄, C₁-C₂, C₁-C₃, C₁-C₄, C₁-C₅, C₁-C₆ in der Definition mitbeinhaltet ist.

Alle hier nicht explizit aufgeführten Bereichsangaben der Anmeldung sind analog wie die oben exemplarisch genannten Bereiche "C₁-C₆", "C₂-C₆" und "C₁-C₆" definiert.

Die oben aufgeführte Definitionen beziehen sich auf die Verbindungen der allgemeinen Formel (I). Die Definitionen sind nicht so zu verstehen, dass sie auch zur Interpretation des ausgeschlossenen (disclaimten) Standes der Technik verwendet werden können. Falls beispielsweise im hier aufgeführten Stand der Technik ein Cycloalkyl genannt wird, ist Cycloalkyl nicht so zu verstehen, dass auch substituiertes Cycloalkyl mitbeinhaltet sein könnte (So wie in dieser Anmeldung bei den Definitionen auf Seite 32 Zeilen 13 bis 18, insbesondere Zeile 16 erwähnt). Die Definiton dieses Cycloalkyls richtet sich einzig und alleine nach dem Originaldokument des der Öffentlichkeit zugänglichen gemachten Standes der Technik.

### Folgende Angaben betreffen beide Ausführungsformen der Erfindung gleichermaßen:

Unter Isomeren sind chemische Verbindungen der gleichen Summenformel aber unterschiedlicher chemischer Struktur zu verstehen. Man unterscheidet im allgemeinen Konstitutionsisomere und Stereoisomere.

Konstitutionsisomere besitzen die gleiche Summenformel, unterscheiden sich jedoch durch die Verknüpfungsweise ihrer Atome oder Atomgruppen. Hierzu zählen funktionelle Isomere, Stellungsisomere, Tautomere oder Valenzisomere.

Stereoisomere haben grundsätzlich die gleiche Struktur (Konstitution) - und damit auch die gleiche Summenformel - unterscheiden sich aber durch die räumliche Anordnung der Atome. Man unterscheidet im allgemeinen Konfigurationsisomere und Konformationsisomere. Konfigurationsisomere sind Stereoisomere, die sich nur durch Bindungsbruch ineinander überführen lassen. Hierzu zählen Enantiomere, Diastereomere und E / Z (cis / trans) Isomere. Enantiomere sind Stereoisomere, die sich wie Bild und Spiegelbild zueinander verhalten und keine Symmetrieebene aufweisen. Alle Stereoisomere, die keine Enantiomere sind, bezeichnet man als Diastereomere. Ein Spezialfall sind E / Z (cis / trans) Isomere an Doppelbindungen. Konformationsisomere sind Stereoisomere, die sich durch die Drehung von Einfachbindungen ineinander überführen lassen.

Zur Abgrenzung der Isomerie-Arten voneinander siehe auch die IUPAC Regeln Sektion E (Pure Appl. Chem. 45, 11-30, 1976).

Die erfindungsgemäßen Verbindungen der allgemeinen Formel I beinhalten auch die möglichen tautomeren Formen und umfassen die E- oder Z-Isomeren oder, falls ein chirales Zentrum vorhanden ist, auch die Racemate und Enantiomeren. Hierunter sind auch Doppelbindungsisomeren zu Verstehen.

Die erfindungsgemäßen Verbindungen können auch in Form von Solvaten, insbesondere von Hydraten vorliegen, wobei die erfindungsgemäßen Verbindungen demgemäß polare Lösungsmittel, insbesondere von Wasser, als Strukturelement des Kristallgitters der erfindungsgemäßen Verbindungen enthalten. Der Anteil an polarem Lösungsmittel, insbesondere Wasser kann in einem stöchiometrischen oder auch unstöchiometrischen Verhältnis vorliegen. Bei stöchiometrischen Solvaten, Hydraten spricht man auch von Hemi-, (Semi-), Mono-, Sesqui-, Di-, Tri-, Tetra-, Penta-, usw. Solvaten oder Hydraten.

Ist eine saure Funktion enthalten, sind als Salze die physiologisch verträglichen Salze organischer und anorganischer Basen geeignet, wie beispielsweise die gut löslichen Alkali- und Erdalkalisalze sowie N-Methyl-glukamin, Dimethyl-glukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Amino-2,3,4-butantriol.

Ist eine basische Funktion enthalten, sind die physiologisch verträglichen Salze organischer und anorganischer Säuren geeignet wie Salzsäure, Schwefelsäure, Phosphorsäure, Zitronensäure, Weinsäure u.a.

Funktionelle Gruppen können gegebenenfalls durch Schutzgruppen während der Reaktionsequenz geschützt werden. Solche Schutzgruppen können unter anderem Ester, Amide, - Ketale/Acetale, Nitrogruppen, Carbamate, Alkylether, Allylether, Benzylether oder Silylether sein.. Als Bestandteil von Silylethern können unter anderem folgende Verbindungen wie z.B.Trimethylsilyl (TMS), *tert*-Butyl-dimethylsilyl (TBDMS), *tert-*Butyl-diphenylsilyl (TBDPS), Triethylsilyl (TES), etc. vorkommen. Deren Herstellung wird im experimentellen Teil beschrieben.

Die erfindungsgemäßen Verbindungen der allgemeinen Formel I inhibieren Proteintyrosinkinasen, insbesondere Eph-Rezeptoren, worauf auch deren Wirkung zum Beispiel bei der Behandlung von Erkrankungen, in denen die Angiogenese, Lymphangiogenese oder Vaskulogenese eine Rolle spielen, Erkrankungen der Blutgefäße, Erkrankungen, die durch eine Hyperproliferation von Körperzellen hervorgerufen werden oder chronische oder akute neurodegenerative Erkrankungen. Die vorliegenden Verbindungen der allgemeinen Formel (I) der beiden Ausführungsformen der Erfindung können demnach zur Herstellung eines Arzneimittels verwendet werden. Die vorliegenden Verbindungen der allgemeinen Formel (I) der beiden Ausführungsformen der Erfindung können insbesondere auch zur Herstellung eines Arzneimittels zur Behandlung der oben aufgeführten Kranheiten verwendet werden.

Behandlungen werden bevorzugt am Menschen, aber auch an verwandten Säugetierspezies, wie z.B. Hund und Katze durchgeführt.

Angiogene und/oder vaskulogene Erkrankungen können behandelt werden, indem das Wachstum der Blutgefäße inhibiert (antiangiogen) oder gefördert (proangiogen) wird. Antiangiogene Verwendungen erfolgen z.B. bei der Tumorangiogenese, der Endometriose, bei diabetisch-bedingten oder anderen Retinopathien oder bei altersbedingter Degeneration der Makula.
Proangiogene Verwendungen erfolgen z.B. bei Herzinfarkt oder akut neurodegenerativen Erkrankungen durch Ischämien des Gehirns oder Neurotraumata.

Unter Blutgefäßerkrankungen sind Stenosen, Arteriosklerosen, Restenosen oder Entzündungskrankheiten, wie rheumatische Arthritis, zu verstehen.

Unter hyperproliferativen Erkrankungen, sind solide Tumore, nicht-solide Tumore oder nichtcancerogene Zellhyperproliferationen zu verstehen.
Wobei unter solide Tumore unter anderem Mamma-, Colon-, Nieren-, Lungen- und/oder Gehirntumore zu verstehen ist,
unter nicht-solide Tumore unter anderem Leukämien zu verstehen sind und
unter nicht-cancerogenen Zellhyperproliferationen Psoriasis, Ekzeme und Skleroderma in der Haut oder benigne Hypertrophie der Prostata zu verstehen ist.

Unter chronisch neurodegenerativen Erkrankungen sind u.a. Huntington's Erkrankung, amyotrophe Lateralsklerose, Parkinson'sche Erkrankung, AIDS induzierte Dementia oder Alzheimer'sche Erkrankung zu verstehen.

Verwendung der Verbindungen der allgemeinen Formel I gemäß den beiden Aufsführungsformen der Erfindung können ebenfalls für diagnostische Zwecke in vitro oder in vivo zur Identifizierung von entsprechenden Rezeptoren in Geweben mittels Autoradiographie und/oder PET verwendet werden.

Insbesondere können für diagnostische Zwecke die Substanzen auch radiomarkiert sein.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe, wie Konservierungs-, Stabilisierungs-, Netzmittel oder Emulgatoren; Salze zur Veränderung des osmotischen Drucks oder Puffer.
Diese pharmazeutischen Präparate sind ebenfalls Gegenstand der vorliegenden Erfindung.
Für die parenterale Anwendung sind insbesondere Injektionslösungen oder Suspensionen, insbesondere wässrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposomen oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die enteralen, parenteralen und oralen Applikationen sind ebenfalls Gegenstand der vorliegenden Erfindung.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-1000 mg, wobei die Dosis als einmal zu verabreichende Einzeldosis oder unterteilt in 2 oder mehreren Tagesdosen gegeben werden kann.

Ebenfalls Gegenstand der beiden Ausführungsformen vorliegenden Erfindung sind Arzneimittel zur Behandlung der oben aufgeführten Erkrankungen, die vorzugsweise mindestens eine Verbindung gemäß der allgemeinen Formel (I) enthalten, sowie Arzneimittel mit geeigneten Formulierungs- und Trägerstoffen.

Soweit die Herstellung der Ausgangsverbindungen nicht beschrieben wird, sind diese dem Fachmann bekannt oder analog zu bekannten Verbindungen oder hier beschriebenen Verfahren herstellbar. Es ist ebenfalls möglich, alle hier beschriebenen Umsetzungen in Parallel-Reaktoren oder mittels kombinatorischer Arbeitstechniken durchzuführen.
Die Isomerengemische können nach üblichen Methoden wie beispielsweise Kristallisation, Chromatographie oder Salzbildung in die Enantiomeren bzw. E/Z-Isomeren aufgetrennt werden.

Die Herstellung der Salze erfolgt in üblicher Weise, indem man eine Lösung der Verbindung der Formel I mit der äquivalenten Menge oder einem Überschuß einer Base oder Säure, die gegebenenfalls in Lösung ist, versetzt und den Niederschlag abtrennt oder in üblicher Weise die Lösung aufarbeitet.

### Herstellung der erfindungsgemäßen Verbindungen gemäß beiden Ausführungsformen der Erfindung

### Verfahrensvariante 1

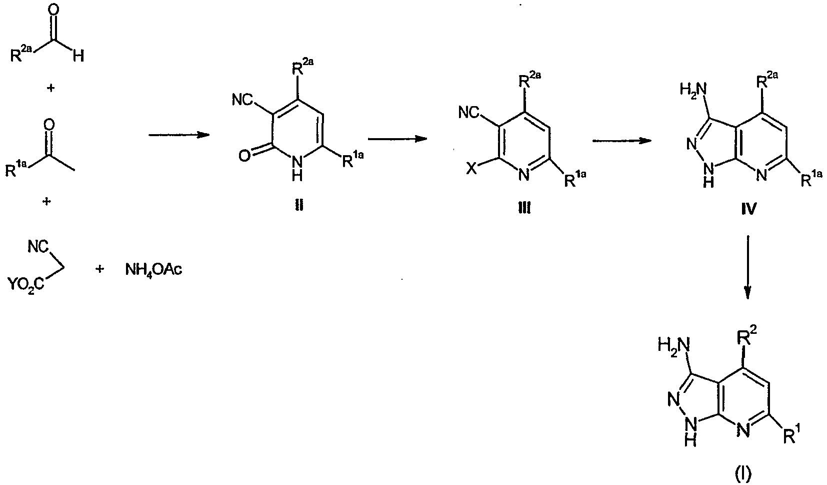

Y steht für einen C₁-C₆-Alkylrest und X steht für Halogen oder Perfluoralkylsulfonyl.

Bei beiden Ausführungsformen haben die Substituenten R^{1a} und R^{2a} die in den allgemeinen Formeln II und III beschriebene Bedeutung.

Die Substituenten R¹ und R² haben die in der allgemeinen Formeln I beschriebene Bedeutung.

Die Herstellung der Verbindungen der allgemeinen Formel I erfolgt auf dem in Verfahrensvariante 1 gezeigten Weg. Durch Reaktion des Aldehyds R^{2a}CHO mit dem Methylketon R^{1a}C(O)CH₃, Cyanessigsäureester sowie Ammoniumacetat werden zunächst die Pyridone der allgemeinen Formel II hergestellt. Verbindungen der allgemeinen Formel II werden dann in Verbindungen der allgemeinen Formel III überführt, worin X die Bedeutung von Halogen oder Perfluoralkylsulfonyl hat. Addition von Hydrazin an Verbindungen der allgemeinen Formel III führt schließlich zu Verbindungen der allgemeinen Formel IV, aus denen dann gegebenenfalls durch weitere Modifikationen der Reste R^{1a} und R^{2a} die Verbindungen der allgemeinen Formel I hergestellt werden können. Diese Modifikationen beinhalten z.B. das Abspalten von Schutzgruppen, aber auch Substitutionen, Additionen, z.B. an Carbonylgruppen oder Nitrile, Alkylierungen, reduktive Aminierungen, Acetylierungen von OH- oder NH-Gruppen und andere Reaktionen. Die Reste R^{1a} und R^{2a} können aber auch schon den späteren Resten R¹ und R² entsprechen, so dass dann durch Hydrazinaddition an Verbindungen der allgemeinen Formel III direkt die Verbindungen der allgemeinen Formel I entstehen.

### Bsp. 1: Herstellung von 6-tert-Butyl-4-p-tolyl-1H-pyrazolo[3,4b]pyridin-3-ylamin

### Bsp.1a) Herstellung von 6-tert-Butyl-2-oxo-4-p-tolyl-1,2-dihydro-pyridin-3-carbonitril

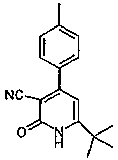

Eine Lösung von 5,13 g Ammoniumacetat, 886 µl Cyanessigsäureethylester, 1,03 ml 3,3-Dimethyl-2-butanon und 1 g 4-Methylbenzaldehyd in 40 ml Ethanol wird 6 Stunden bei 80°C gerührt. Anschließend wird 5 Stunden bei 20°C nachgerührt. Das ausgefallene Produkt wird abfiltriert. Man wäscht das Filtrat mit Ethanol und Hexan nach. Es werden 630 mg Produkt erhalten.
¹H-NMR (CDCl₃): δ 1,44 (9H); 2,42 (3H); 6,30 (1H); 7,31 (2H); 7,52 (2H); 12,41 (1H) ppm.

### Bsp. 1 b) Herstellung von 2-Bromo-6-tert-butyl-4-p-tolyl-nicotinonitril

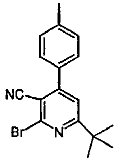

Zu einer Lösung von 315 mg der unter Beispiel 1a) beschriebenen Substanz in 3,5 ml Toluol werden 403 mg Phosphorpentoxid und 460 mg Tetrabutylammoniumbromid addiert. Man kocht eine Stunde unter Rückfluss. Nach dem Abkühlen wird das Reaktionsgemisch mit Ethylacetat verdünnt. Dann gießt man auf Wasser, trennt die Phasen und extrahiert die wäßrige Phase erneut mit Ethylacetat. Die vereinigten organischen Phasen werden mit gesättigter Natriumchloridlösung gewaschen. Man trocknet über Natriumsulfat und engt im Vakuum ein. Das erhaltene Rohprodukt wird an Kieselgel chromatographiert. Man erhält 120 mg Produkt.
¹H-NMR (CDCl₃): δ = 1,39 (9H); 2,43 (3H); 7,32 (3H); 7,47 (2H) ppm.

### Bsp. 1c) Herstellung von 6-tert-Butyl-4-p-tolyl-1H-pyrazolo[3,4b]pyridin-3-ylamin

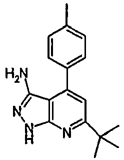

Zu einer Lösung von 117 mg der unter Beispiel 1b) beschriebenen Verbindung in 2 ml Propanol werden 43 µl Hydrazinhydrat-Lösung (80%ig) addiert. Man rührt bei 100°C 3 Stunden nach.

Danach werden erneut 22 µl Hydrazinhydrat-Lösung (80%ig) addiert. Man rührt weitere 1,5 Stunden bei 100°C nach und kühlt anschließend das Reaktionsgemisch auf 0°C ab. Anschließend lässt man 3 Stunden bei 0°C stehen. Das ausgefallene Reaktionsprodukt wird abgesaugt. Es wird mit eiskaltem Propanol nachgewaschen.
¹H-NMR (CDCl₃): δ= 1,46 (9H); 2,44 (3H): 3,93 (2H); 7,01 (1H); 7.33 (2H); 7,48 (2H) ppm.

### Bsp. 2: Herstellung von 4-(3-Amino-6-tert-butyl-1H-pyrazolo[3,4b]pyridin-4-yl)-benzonitril

### Bsp. 2a) Herstellung von 6-tert-Butyl-4-(4-cyanophenyl)-2-oxo-1,2-dihydro-pyridin-3-carbonitril

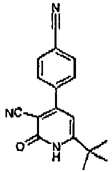

Analog zu Beispiel 1a werden aus 4,7 g Ammoniumacetat, 812 µl Cyanessigsäureethylester, 945 µl 3,3-Dimethyl-2-butanon und 1 g 4-Cyanobenzaldehyd 880 mg Produkt erhalten.
¹H-NMR (d6-DMSO): δ = 1,31 (9H); 6,30 (1 H); 7,84 (2H); 8,03 (2H); 11,30 (1 H) ppm.

### Bsp. 2b) Herstellung von 2-Bromo-6-tert-butyl-4-(4-cyanophenyl)-nicotinonitril

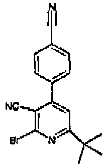

Analog zu Beispiel 1b) werden aus 300 mg der unter Beispiel 2a) beschriebenen Substanz in 5 ml Toluol mit 370 mg Phosphorpentoxid und 418 mg Tetrabutylammoniumbromid 250 mg Produkt erhalten.
¹H-NMR (CDCl₃): δ = 1,40 (9H); 7,33 (1H): 7,68 (2H); 7,83 (2H) ppm.

### Bsp. 2c) Herstellung von 4-(3-Amino-6-tert-butyl-1H-pyrazolo[3,4b]pyridin-4-yl)-benzonitril

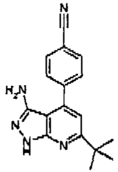

Analog zu Beispiel 1c) werden aus 200 mg der unter Beispiel 2b) beschriebenen Verbindung mit 71 µl Hydrazinhydrat-Lösung (80%ig) in Propanol 98 mg Produkt hergestellt.
¹H-NMR (d6-DMSO): δ =1,39 (9H); 4,56 (2H); 7,00 (1 H); 7,80 (2H); 8,00 (2H); 12,29 (1 H) ppm.

### Bsp. 3: Herstllung von 6-tert-Butyl-4-(1H-indol-3-yl)-1H-pyrazolo[3,4b]pyridin-3-ylamin

### Bsp. 3a) Herstellung von 6-tert-Butyl-4-(4-cyanophenyl)-2-oxo-1,2-dihydro-pyridin-3-carbonitril

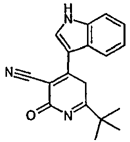

Analog zu Beispiel 1a) werden aus 4,25 g Ammoniumacetat, 733 µl Cyanessigsäureethylester, 852 µl 3,3-Dimethyl-2-butanon und 1 g 3-Indolcarbaldehyd 240 mg Produkt erhalten.
¹H-NMR (d6-DMSO): δ = 1,32 (9H); 6,50 (1 H); 7,20 (2H); 7,53 (1H); 7,72 (2H); 8,09 (1 H); 11,98 (2H) ppm.

### Bsp. 3b) Herstellung von 6-tert-Butyl-3-cyano-4-(1H-indol-3-yl)-2-trifluormethanesulfonylpyridin

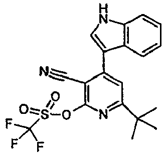

Zu einer Lösung von 200 mg der unter Beispiel 3a) beschriebenen Verbindung in 4 ml Pyridin werden bei 0°C 206 µl Trifluormethansulfonsäureanhydrid getropft. Man lässt 3 Stunden bei 0°C nachrühren und addiert dann weitere 100 µl Trifluoressigsäureanhydrid. Es wird weitere 1,5 Stunden bei 0°C nachgerührt. Danach wird das Reaktionsgemisch auf gesättigte Natriumhydrogencarbonatlösung gegossen. Man extrahiert mit Ethylacetat, wäscht die organische Phase mit gesättigter Natriumchloridlösung, trocknet über Natriumsulfat und engt im Vakuum ein. Nach Säulenchromatographie an Kieselgel werden 116 mg Produkt erhalten.
¹H-NMR (d6-DMSO): δ = 1,34 (9H); 6,50 (1H); 7,20 (2H); 7,54 (1HH); 7,75 (1H); 8,08 (1H); 12,00 (2H) ppm.

### Bsp. 3c) Herstellung von 6-tert-Butyl-4-(1H-indol-3-yl)-1H-pyrazolo[3,4b]pyridin-3-ylamin

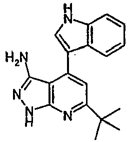

Analog zu Beispiel 1c) werden aus 190 mg der unter Beispiel 3b) beschriebenen Verbindung mit 55 µl Hydrazinhydrat-Lösung (80%ig) in Propanol 75 mg Produkt hergestellt.
¹H-NMR (d6-DMSO): δ= 1,40 (9H); 4,55 (2H); 7,04 (1H); 7,80 (2H); 8,01 (2H); 12,39 (1H) ppm.

### Bsp. 4: Herstellung von 6-tert-Butyl-4-(4-phenoxyphenyl)-1H-pyrazolo[3,4b]pyridin-3-ylamin

### Bsp. 4a) Herstellung von 6-tert-Butyl-2-oxo-4-(4-phenoxyphenyl)-1,2-dihydro-pyridin-3-carbonitril

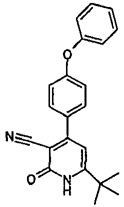

Analog zu Beispiel 1a) werden aus 3,11 g Ammoniumacetat, 540 µl Cyanessigsäureethylester, 624 µl 3,3-Dimethyl-2-butanon und 883 µl 4-Phenoxybenzaldehyd 510 mg Produkt erhalten. ¹H-NMR: (d6-DMSO): δ = 1,30 (9H); 6,27 (1H); 7,13 (4H); 7,20 (1H); 7,45 (2H); 7,70 (2H); 12,20 (1H) ppm.

### Bsp. 4b) Herstellung von 6-tert-Butyl-3-cyano-4-(4-phenoxyphenyl)-2-trifluormethanesulfonylpyridin

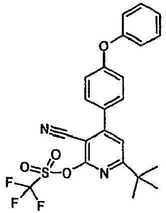

Analog zu Beispiel 3b) werden aus 150 mg der unter Beispiel 4a) beschriebenen Substanz und 162 µl Trifluormethansulfonsäureanhydrid in Pyridin 138 mg Produkt erhalten.
¹H-NMR (CDCl₃): δ = 1,39 (9H); 7,11 (4H); 7,24 (1 H); 7,41 (3H); 7,59 (2H) ppm.

### Bsp. 4c) 6-tert-Butyl-4-(4-phenoxyphenyl)-1H-pyrazolo[3,4b]pyridin-3-ylamin

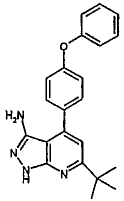

Analog zu Beispiel 3 werden aus 67 mg der unter Beispiel 11 beschriebenen Verbindung mit 35 µl Hydrazinhydrat-Lösung (80%ig) in Propanol 32 mg Produkt hergestellt.
¹H-NMR (d6-DMSO): δ = 1,38 (9H); 4,54 (2H); 6,98 (1H); 7,15 (5H); 7,44 (2H); 7,62 (2H) 12,16 (1H) ppm.

### Bsp. 5: Herstellung von 6-tert-Butyl-4-(4-benzyloxyphenyl)-1H-pyrazolo[3,4b]pyridin-3-ylamin

### Bsp. 5a) Herstellung von 4-(4-Benzyloxyphenyl)-6-tert-butyl-2-oxo-1,2-dihydro-pyridin-3-carbonitril

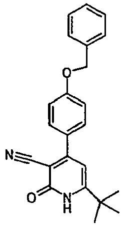

Analog zu Beispiel 1a) werden aus 2,91 g Ammoniumacetat, 500 µl Cyanessigsäureethylester, 585 µl 3,3-Dimethyl-2-butanon und 1 g 4-Benzyloxybenzaldehyd 613 mg Produkt erhalten.
¹H-NMR (d6-DMSO): δ = 1,30 (9H); 5,20 (2H); 6,22 (1H); 7,18 (2H); 7,30-7,50 (5H); 7,65 (1H); 12,18 (1H) ppm.

### Bsp. 5b) Herstellung von 6-tert-Butyl-3-cyano-4-(4-benzyloxyphenyl)-2-trifluormethanesulfonylpyridin

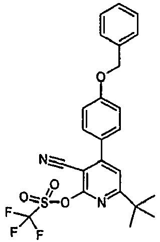

Analog zu Beispiel 3b) werden aus 250 mg der unter Beispiel 5a) beschriebenen Substanz und 258 µl Trifluormethansulfonsäureanhydrid in Pyridin 288 mg Produkt erhalten.
¹H-NMR (CDCl₃): δ= 1,38 (9H); 5,16 (2H); 7,13 (2H); 7,32-7,50 (6H); 7,59 (2H) ppm.

### Bsp. 5c) Herstellung von 6-tert-Butyl-4-(4-benzyloxyphenyl)-1H-pyrazolo[3,4b]pyridin-3-ylamin

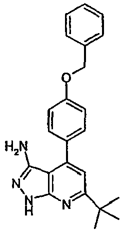

Analog zu Beispiel 1c) werden aus 284 mg der unter Beispiel 5b) beschriebenen Verbindung mit 70 µl Hydrazinhydrat-Lösung (80%ig) in Propanol 159 mg Produkt hergestellt.
¹H-NMR (d6-DMSO): δ = 1,38 (9H); 4,50 (2H); 5,19 (2H); 6,93 (1H); 7,19 (2H); 7,30-7,58 (7H); 12,12 (1H) ppm.

### Bsp. 6: Herstellung von 6-tert-Butyl-4-(3-methoxyphenyl)-1H-pyrazolo[3,4b]pyridin-3-ylamin

### Bsp. 6a) Herstellung von 6-tert-Butyl-4-(3-methoxyphenyl)-2-oxo-1,2-dihydro-pyridin-3-carbonitril

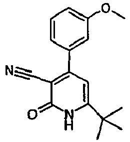

Analog zu Beispiel 1a) werden aus 4,53 g Ammoniumacetat, 782 µl Cyanessigsäureethylester, 910 µl 3,3-Dimethyl-2-butanon und 895 µl 3-Methoxybenzaldehyd 618 mg Produkt erhalten.
¹H-NMR (CDCl₃): δ= 1,45 (9H); 3,86 (3H); 6,31 (1H); 7,04 (1H); 7,16 (2H); 7,42 (1H); 12,40 (1H) ppm.

### Bsp. 6b) Herstellung von 6-tert-Butyl-3-cyano-4-(3-methoxyphenyl)-2-trifluormethanesulfonylpyridin

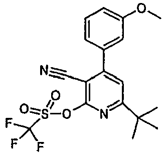

Analog zu Beispiel 3b) werden aus 250 mg der unter Beispiel 6a) beschriebenen Substanz und 328 µl Trifluormethansulfonsäureanhydrid in Pyridin 305 mg Produkt erhalten.
¹H-NMR (CDCl₃): δ = 1,40 (9H); 3,89 (3H); 7,08-7,20 (3H); 7,48 (2H) ppm.

### Bsp. 6c) Herstellung von 6-tert-Butyl-4-(3-methoxyphenyl)-1H-pyrazolo[3,4b]pyridin-3-ylamin

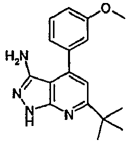

Analog zu Beispiel 1c) werden aus 299 mg der unter Beispiel 6b) beschriebenen Verbindung mit 88 µl Hydrazinhydrat-Lösung (80%ig) in Propanol 137 mg Produkt hergestellt.
¹H-NMR (CDCl₃): δ = 1,45 (9H); 3,89 (3H); 3,97 (2H); 7,03 (2H); 7,14 (2H); 7,45 (1H) 10,40 (1 H) ppm.

### Bsp. 7: Herstellung von 6-tert-Butyl-4-(3-cyanophenyl)-1H-pyrazolo[3,4b]pyridin-3-ylamin

### Bsp. 7a) Herstellung von 6-tert-Butyl-4-(3-cyanophenyl)-2-oxo-1,2-dihydro-pyridin-3-carbonitril

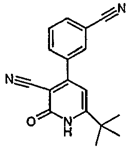

Analog zu Beispiel 1a) werden aus 14 g Ammoniumacetat, 2,43 ml Cyanessigsäureethylester, 2,83 ml 3,3-Dimethyl-2-butanon und 3 g 3-Cyanobenzaldehyd 2,14 g Produkt erhalten.
¹H-NMR (CDCl₃): δ = 1,46 (9H); 6,27 (1H); 7,67 (1H); 7,80-7,92 (3H); 12,42 (1H) ppm.

### Bsp. 7b) Herstellung von 6-tert-Butyl-3-cyano-4-(3-cyanophenyl)-2-trifluormethanesulfonylpyridin

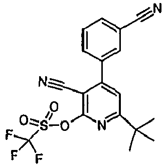

Analog zu Beispiel 3b) werden aus 420 mg der unter Beispiel 7a) beschriebenen Substanz und 560 µl Trifluormethansulfonsäureanhydrid in Pyridin 379 mg Produkt erhalten.
¹H-NMR (CDCl₃): δ = 1,40 (9H); 7,45 (1H); 7,71 (1H); 7,88 (3H) ppm.

### Bsp. 7c) Herstellung von 6-tert-Butyl-4-(3-cyanophenyl)-1H-pyrazolo[3,4b]pyridin-3-ylamin

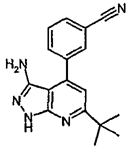

Analog zu Beispiel 1c) werden aus 369 mg der unter Beispiel 7b) beschriebenen Verbindung mit 165 µl Hydrazinhydrat-Lösung (80%ig) in Propanol 147 mg Produkt hergestellt.
¹H-NMR (d6-DMSO): δ = 1,39 (9H); 4,57 (2H); 7,08 (1H); 7,72 (1H); 7,95 (2H); 8,11 (1H); 12,28 (1H) ppm.

### Bsp. 8: Herstellung von 1-[4-(3-Amino-6-tert-butyl-1H-pyrazolo[3,4b]pyridin-4-yl)-phenyl]-ethanon

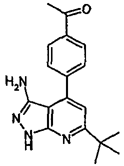

Zu einer Lösung von 150 mg der unter Beispiel 2c) beschriebenen Verbindung in 5 ml Tetrahydrofuran werden bei 0°C 2,2 ml einer 1,6 molaren Lösung von Methyllithium in Diethylether addiert. Man lässt 2 Stunden bei 0°C nachrühren. Anschließend wird das Reaktionsgemisch auf 2 normale Salzsäure gegossen. Man lässt 1 Stunde bei 25°C nachrühren und neutralisiert dann mit 5%iger Natronlauge. Anschließend extrahiert man mit Ethylacetat und wäscht die wäßrige Phase mit gesättigter Natriumchloridlösung. Es wird über Natriumsulfat getrocknet. Das Rohprodukt wird durch Säulenchromatographie an Kieselgel gereinigt. Man erhält 67 mg Produkt
¹H-NMR (d6-DMSO): δ = 1,38 (9H); 2,65 (3H); 4,47 (2H); 7,02 (1H); 7,74 (2H); 8,12 (2H); 12,21 (1H) ppm.

### Bsp. 9: Herstellung von 1-[3-(3-Amino-6-tert-butyl-1H-pyrazolo[3,4b]pyridin-4-yl)-phenyl]-ethanon

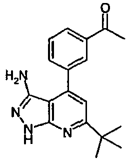

Analog zu Beispiel 8 werden aus 169 mg der unter Beispiel 7c) beschriebenen Verbindung und 1,2 ml einer 1,6 molaren Lösung von Methyllithium in Diethylether 82 mg Produkt erhalten.
¹H-NMR (CDCl₃): δ = 1,46 (9H); 2,69 (3H); 3,80 (2H); 7,06 (1H); 7,65 (1H); 7,80 (1H); 8,09 (1H); 8,19 (1H); 9,57 (1H) ppm.

### Bsp. 10: Herstellung von 6-Cyclohexyl-4-p-tolyl-1H-pyrazolo[3,4b]pyridin-3-ylamin

### Bsp. 10a) Herstellung von 6-Cyclohexyl-2-oxo-4-p-tolyl-1,2-dihydropyridin-3-carbonitril

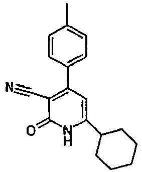

Analog zu Beispiel 1a) werden aus 5,13 g Ammoniumacetat, 886 µl Cyanessigsäureethylester, 1,14 ml Cyclohexylmethylketon und 1 g 4-Methylbenzaldehyd 1,4 g Produkt erhalten.
¹H-NMR (CDCl₃): δ = 1,30-1,70 (6H); 1,82-2,05 (4H); 2,42 (3H); 2,64 (1H); 6,25 (1H); 7,30 (2H); 7,54 (2H); 13,08 (1H) ppm.

### Bsp. 10b) Herstellung von 3-Cyano-6-cyclohexyl-4-p-tolyl)-2-trifluormethansulfonylpyridin

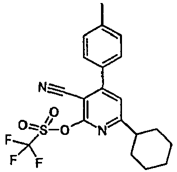

Analog zu Beispiel 3b) werden aus 300 mg der unter Beispiel 10a) beschriebenen Substanz und 380 µl Trifluormethansulfonsäureanhydrid in Pyridin 342 mg Produkt erhalten.
¹H-NMR (CDCl₃): δ = 1,20-1,60 (5H); 1,75 (1H); 1,82-2,00 (4H); 2,44 (3H); 2,78 (1H); 7,30 (1H); 7,35 (2H); 7,51 (1H) ppm.

### Bsp.10c) Herstellung von 6-Cyclohexyl-4-p-tolyl-1H-pyrazolo[3,4b]pyridin-3-ylamin

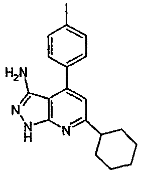

Analog zu Beispiel 1c) werden aus 342 mg der unter Beispiel 10b) beschriebenen Verbindung mit 147 µl Hydrazinhydrat-Lösung (80%ig) in Propanol 195 mg Produkt hergestellt.
¹H-NMR (CDCl₃): δ = 1,22-1,60 (5H); 1,77 (1 H); 1,88 (2H); 2,02 (2H); 2,47 (3H); 2,80 (1 H); 3,92 (2H); 6,82 (1H); 7,32 (2H); 7,48 (2H); 9,80 (1H) ppm.

### Bsp. 11: Herstellung von 6-(1,4-Dioxa-spiro[4.5]dec-8-yl)-4-p-tolyl-1H-pyrazolo[3,4b]pyridin-3-ylamin

### Bsp. 11a) Herstellung von1,4-Dioxa-spiro[4.5]decane-8-carbonsäureethylester

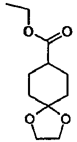

Eine Mischung aus 7,35 g 4-Oxocyclohexylcarbonsäureethylester, 28 ml Trimethylorthoformiat, 64 ml Ethylenglycol und 100 mg p-Toluolsulfonsäure in 120 ml Dichlormethan wird 12 Stunden bei 25°C geführt. Anschließend werden 2 ml Triethylamin addiert. Man wäscht mit gesättigter Natriumhydrogencarbonatlösung und danach mit gesättigter Natriumchloridlösung, trocknet über Natriumsulfat und engt im Vakuum ein. Das erhaltene Rohprodukt wird durch Säulenchromatographie an Kieselgel gereinigt. Man erhält 9,22 g Produkt.
¹H-NMR (CDCl₃): δ = 1,23 (3H); 1,55 (2H); 1,72-1,86 (4H); 1,94 (2H); 2,31 (1H); 3,93 (4H); 4,12 (2H) ppm.

### Bsp. 11b) Herstellung von (1,4-Dioxa-spiro[4.5]dec-8-yl)-methanol

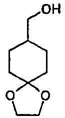

Zu einer Lösung von 1,18 g der unter Beispiel 11a) beschriebenen Verbindung in 20 ml Toluol werden bei 0°C 13,8 ml einer 1,2 molaren Lösung von Düsobutylamin in Toluol addiert. Man lässt eine Stunde bei 0°C nachrühren und addiert dann 6 ml 2-Propanol sowie 6 ml Wasser. Man lässt eine weitere Stunde nachrühren und filtriert anschließend die ausgefallenen Salze über Celite ab. Nach Trocken und Einengen wird das erhaltene Rohprodukt durch Säulenchromatographie an Kieselgel gereinigt. Man erhält 880 mg Produkt.
¹H-NMR (CDCl₃): δ = 1,15-1,40 (3H); 1,48-1,62 (3H); 1,78 (4H); 3,49 (2H); 3,95 (4H) ppm.

### Bsp. 11c) Herstellung von 1,4-Dioxa-spiro[4.5]decan-8-carbaldehyd

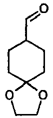

Zu einer Lösung von 4,9 ml Oxalylchlorid in 30 ml Dichlormethan werden bei -78°C 7,96 ml Dimethylsulfoxid addiert. Man lässt 3 Minuten bei -78°C nachrühren und addiert dann eine Lösung von 6,96 g der unter Beispiel 11c) beschriebenen Verbindung in 70 ml Dichlormethan. Es wird 20 Minuten bei -78°C nachgerührt. Dann werden 24 ml Triethylamin addiert. Man lässt das Reaktionsgemisch über 20 Minuten auf 0°C erwärmen. Danach wird auf gesättigte Natriumhydrogencarbonatlösung gegossen und mit Dichlormethan extrahiert. Die organische Phase wird mit gesättigter Natriumchloridlösung gewaschen, über Natriumsulfat getrocknet und im Vakuum eingeeengt. Man erhält 6,9 g Produkt, welches ohne Reinigung in die Folgestufe eingesetzt wird.
¹H-NMR (CDCl₃): δ= 1,50-1,80 (6H); 1,95 (2H); 2,25 (1H); 3,98 (4H); 9,64 (1H) ppm.

### Bsp. 11d) Herstellung von 1-(1,4-Dioxa-spiro[4.5]dec-8-yl)-ethanol

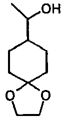

Zu einer Lösung von 6,88 g der unter Beispiel 11c) beschriebenen Verbindung in 100 ml Tetrahydrofuran werden bei 0°C 38 ml einer 1,6 molaren Lösung von Methyllithium Diethylether addiert. Man lässt eine Stunde bei 0°C nachrühren und gießt danach das Reaktionsgemisch auf gesättigte Ammoniumchloridlösung. Danach wird mit Ethylacetat extrahiert. Man wäscht die organische Phase mit gesättigter Natriumchloridlösung, trocknet über Natriumsulfat und engt im Vakuum ein. Nach Chromatographie an Kieslegel werden 6,02 g Produkt erhalten.
¹H-NMR (CDCl₃): δ = 1,18 (3H); 1,25-1,45 (4H); 1,53 (2H); 1,70-1,92 (3H); 3,60 (1H); 3,93 (4H) ppm.

### Bsp. 11e) Herstellung von1-(1,4-Dioxa-spiro[4.5]dec-8-yl)-ethanon

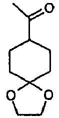

Zu einer Lösung von 6 g der unter Beispiel 11d) beschriebenen Verbindung in 100 ml Dichlormethan werden 7,2 g N-Methylmorpholin-N-oxid, 565 mg Tetrapropylammoniumperuthenat sowie etwas Molsieb addiert. Man lässt 20 Stunden bei 25°C rühren und filtriert danach über Celite. Nach Chromatographie an Kieselgel werden 5,1 g Produkt erhalten.
¹H-NMR (CDCl₃): δ = 1,50-1,98 (8H); 2,17 (3H); 2,34 (1H); 3,94 (4H) ppm.

### Bsp. 11f) Herstellung von 6-(1,4-Dioxa-spiro[4.5]dec-8-yl)-2-oxo-4-p-tolyl-1,2-dihydropyridin-3-carbonitril

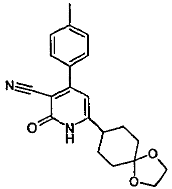

Analog zu Beispiel 1a) werden aus 2,17 g Ammoniumacetat, 375 µl Cyanessigsäureethylester, 650 mg der unter Beispiel 11e) beschriebenen Verbindung und 415 µl 4-Methylbenzaldehyd 580 mg Produkt erhalten.
¹H-NMR (CDCl₃): δ = 1,70-1,95 (6H); 2,03 (2H); 2,42 (3H); 2,72 (1H); 3,96 (4H); 6,30 (1H); 7,30 (2H); 7,52 (2H) ppm.

### Bsp. 11g) Herstellung von 3-Cyano-6-(1,4-dioxa-spiro[4.5]dec-8-yl)-4-p-tolyl-2-trifluormethansulfonylpyridin

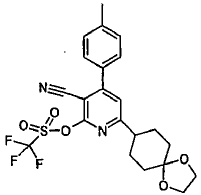

Analog zu Beispiel 3b werden aus 900 mg der unter Beispiel 11f) beschriebenen Substanz und 950 µl Trifluormethansulfonsäureanhydrid in Pyridin 843 mg Produkt erhalten.
¹H-NMR (CDCl₃): δ = 1,60-1,78 (2H); 1,80-2,08 (6H); 2,45 (3H); 2,83 (1H); 3,98 (3H); 7,35 (3H); 7,51 (2H) ppm.

### Bsp. 11h) Herstellung von 6-(1,4-Dioxa-spiro[4.5]dec-8-yl)-4-p-tolyl-1H-pyrazolo[3,4b]pyridin-3-ylamin

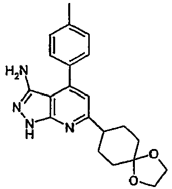

Analog zu Beispiel 1c) werden aus 838 mg der unter Beispiel 11g) beschriebenen Verbindung mit 317 µl Hydrazinhydrat-Lösung (80%ig) in Propanol 496 mg Produkt hergestellt.
¹H-NMR (CDCl₃): δ = 1,60-2,10 (8H); 2,44 (3H); 3,36 (1H); 3,98 (6H); 6,86 (1H); 7,32 (2H); 7,46 (2H) ppm.

### Bsp. 12: Herstellung von 4-(3-Amino-4-p-tolyl-1H-pyrazolo[3,4b]pyridin-6-yl)-cyclohexanon

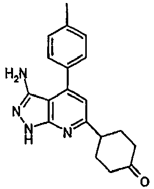

Zu einer Lösung von 342 mg der unter Beispiel 11h) beschriebenen Substanz in 20 ml Aceton werden 1,2 ml 4 normale Salzsäure addiert. Man lässt 3,5 Stunden bei 25°C nachrühren und addiert dann 1 ml Triethylamin. Anschließend wird filtriert und das Filtrat im Vakuum eingeengt. Der Rückstand wird in Ethylacetat gelöst. Man wäscht mit gesättigter Natriumchloridlösung, trocknet über Natriumsulfat und engt erneut im Vakuum ein. Nach Umkristallisation aus einem Gemisch aus Dichlormethan/ Diisopropylether werden 240 mg Produkt erhalten.
¹H-NMR (CDCl₃): δ= 2,10-2,45 (4H); 2,46 (3H); 2,52-2,65(4H); 3,32 (1H); 4,00 (2H); 6,88 (1H); 7,35 (2H); 7,49 (2H); 10,75 (1 H) ppm.

### Bsp. 13: Herstellung von 4-[4-(3-Amino-4-p-tolyl-1H-pyrazolo[3,4b]pyridin-6-yl)-cyclohexyl]-piperazin-1-carbonsäure tert-butyl ester

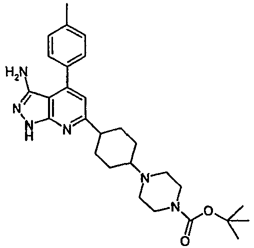

Zu einer Lösung von 100 mg der unter Beispiel 12 beschriebenen Verbindung in 4 ml Dichlormethan werden 291 mg 1-*tert*-Butoxycarbonylpiperazin, 90 µl Eisessig sowie Molsieb addiert. Man rührt 30 Minuten bei 25°C und addiert dann 40 mg Natriumacetoxyborhydrid. Es wird 30 Minuten bei 25°C nachgerührt. Dann werden weitere 40 mg Natriumacetoxyborhydrid addiert. Man lässt 3 Stunden bei 25°C nachrühren und vedünnt danach mit Ethylacetat. Es wird mit gesättigter Natriumhydrogencarbonat und mit gesättigter Natriumchloridlösung gewaschen, über Natriumsulfat getrocknet und im Vakuum eingeengt. Das Rohprodukt über Säulenchromatographie gereinigt. Man erhält 108 mg Produkt.
¹H-NMR (CDCl₃): δ = 1,45 (9H); 1,60-2,35 (8H); 2,46 (3H); 2,81 (4H); 3,00 (1H); 3,40 (5H); 3,92 (2H); 6,88 (1 H); 7,33 (2H); 7,47 (2H); 9,70 (1 H) ppm.

### Bsp. 14: Herstellung von 6-(4-Piperazin-1-yl-cyclohexyl)-4-p-tolyl-1H-pyrazolo[3,4b]pyridin-3-ylamin

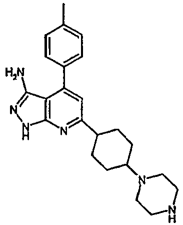

Eine Lösung von 50 mg der unter Beispiel 13 beschriebenen Verbindung in 1 ml Dichlormethan wird mit 300 µl einer 2 molaren HCl Lösung in Diethylether versetzt. Man lässt eine Stunde bei 25°C nachrühren und addiert dann erneut 300 µl einer 2 molaren HCl Lösung in Diethylether. Es wird eine weitere Stunde bei 25°C nachgerührt. Danach wird im Vakuum eingeengt und durch Säulenchromatographie aufgereinigt. Es werden 29 mg Produkt erhalten.
¹H-NMR (d6-DMSO): δ = 1,75-2,02 (8H); 2,41 (3H); 3,00-4,00 (12H); 7,20 (1H); 7,38 (2H); 7,60 (2H); 9,80 (2H) ppm.

### Bsp. 15: Herstellung von 6-[4-(4-Methyl-piperazin-1-yl)-cyclohexyl]-4-p-tolyl-1H-pyrazolo[3,4b]pyridin-3-ylamin

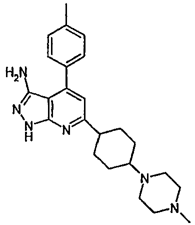

Analog zu Beispiel 13 werden aus 48 mg der unter Beispiel 12 beschriebenen Verbindung, 85 µl N-Methylpiperazin, 43 µl Eisessig und 40 mg Natriumtriacetoxyborhydrid 41 mg Produkt erhalten.
¹H-NMR (d6-DMSO): δ = 1,50-2,40 (8H); 2,22 (3H); 2,39 (3H); 2,91 (1H); 3,20-3,55 (9H); 4,48 (2H); 6,79 (1 H); 7,37 (2H); 7,48 (2H) ppm.

### Bsp. 16: Herstellung von 6-(4-Piperidin-1-yl-cyclohexyl)-4-p-tolyl-1H-pyrazolo[3,4b]pyridin-3-ylamin

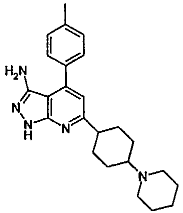

Analog zu Beispiel 13 werden aus 70 mg der unter Beispiel 12 beschriebenen Verbindung, 110 µl Piperidin, 63 µl Eisessig und 50 mg Natriumtriacetoxyborhydrid 52 mg Produkt erhalten.
¹H-NMR (CDCl₃): δ = 1,40 (2H); 1,50-1,80 (7H); 1,90 (2H); 2,20-2,38 (5H); 2,40-2,60 (6H); 3,05 (1 H); 3,92 (2H); 6,90 (1H); 7,32 (2H); 7,48 (2H) ppm.

### Bsp. 17: Herstellung von 6-(4-Morpholin-4-yl-cyclohexyl)-4-p-totyl-1H-pyrazolo[3,4b]pyridin-3-ylamin

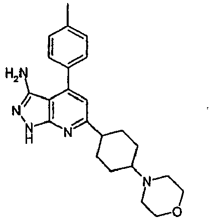

Analog zu Beispiel 13 werden aus 70 mg der unter Beispiel 12 beschriebenen Verbindung, 100 µl Morpholin, 63 µl Eisessig und 50 mg Natriumtriacetoxyborhydrid 48 mg Produkt erhalten.
¹H-NMR (d6-DMSO): δ = 1,50-1,70 (4H); 1,90-2,20 (6H); 2,30-2,50 (6H); 2,95 (1H); 3,58 (4H); 4,46 (2H); 6,79 (1 H); 7,37 (2H); 7,49 (2H); 12,11 (1 H) ppm.

### Bsp. 18: Herstellung von 6-(1,4-Dioxa-spiro[4.5]dec-8-yl)-4-(4-phenoxyphenyl)-1H-pyrazolo[3,4b]pyridin-3-ylamine

### Bsp. 18a) Herstellung von 6-(1,4-Dioxa-spiro[4.5]dec-8-yl)-2-oxo-4-(4-phenoxy-phenyl)-1,2-dihydro-pyridin-3-carbonitril

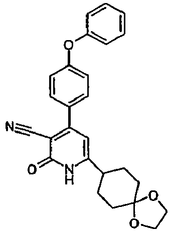

Analog zu Beispiel 1a) werden aus 5,28 g Ammoniumacetat, 910 µl Cyanessigsäureethylester, 1,58 g der unter Beispiel 11e) beschriebenen Verbindung und 1,5 ml 4-Phenoxybenzaldehyd 1,86 g Produkt erhalten.
¹H-NMR (CDCl₃): δ = 1,70-1,95 (6H); 2,05 (2H); 2,70 (1H); 3,98 (4H); 6,30 (1H); 7,08 (4H); 7,19 (1 H); 7,40 (2H); 7,60 (2H) 12,89 (1 H) ppm.

### Bsp. 18b): Herstellung von 3-Cyano-6-(1,4-dioxa-spiro[4.5]dec-8-yl)-4-(4-phenoxy-phenyl)-2-trifluormethansulfonylpyridin

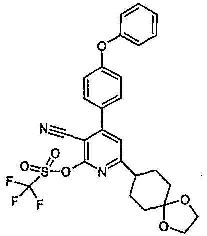

Analog zu Beispiel 3b) werden aus 1,86 g der unter Beispiel 18 beschriebenen Substanz und 1,61 ml Trifluormethansulfonsäureanhydrid in Pyridin 2,01g Produkt erhalten.
¹H-NMR (CDCl₃): δ = 1,60-2,10 (8H); 2,85 (1H); 3,98 (4H); 7,10 (4H); 7,21 (1H); 7,40 (2H); 7,58 (2H) ppm.

### Bsp. 18c): Herstellung von 6-(1,4-Dioxa-spiro[4.5]dec-8-yl)-4-(4-phenoxyphenyl)-1H-pyrazolo[3,4b]pyridin-3-ylamine

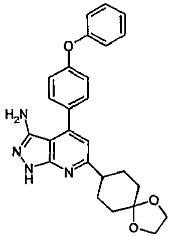

Analog zu Beispiel 1c) werden aus 1,36 mg der unter Beispiel 19 beschriebenen Verbindung mit 445 µl Hydrazinhydrat-Lösung (80%ig) in Propanol 970 mg Produkt hergestellt.
¹H-NMR (CDCl₃): δ = 1,60-2,10 (8H); 2,88 (1H); 3,99 (4H); 6,88 (1H); 7,05-7,14 (5H); 7,40 (2H); 7,53 (2H); 10,00 (1H) ppm.

### Bsp. 19: Herstellung von 4-[3-Amino-4-(4-phenoxy-phenyl)-1H-pyrazolo[3,4b]pyridin-6-yl]-cyclohexanon

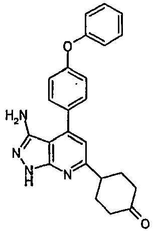

Analog zu Beispiel 12 werden aus 1,19 g der unter Beispiel 18c) beschriebenen Verbindung durch Umsetzung mit 4 normaler Salzsäure in Aceton 890 mg Produkt erhalten.
¹H-NMR (CDCl₃): δ = 2,10-2,60 (8H); 3,30 (1H); 3,99 (2H); 6,88 (1H); 7,03-7,22 (5H); 7,40 (2H); 7,55 (2H); 10,08 (1H) ppm.

### Bsp. 20: Herstellung von 4-{4-[3-Amino-4-(4-phenoxy-phenyl)-1H-pyrazolo[3,4b]pyridin-6-yl]-cyclohexyl}-piperazine-1-carbonsäure tert-butyl ester

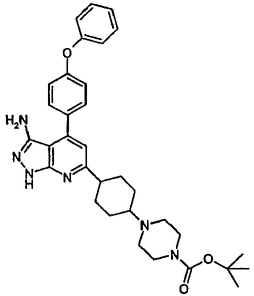

Analog zu Beispiel 13 werden durch Umsetzung von 170 mg der unter Beispiel 18c) beschriebenen Substanz mit 400 mg 1-*tert*-Butoxycarbonylpiperazin, 125 µl Eisessig und 100 mg Natriumacetoxyborhydrid in Dichlormethan 142 mg Produkt erhalten.
¹H-NMR (d6-DMSO): δ = 1,40 (9H); 1,50-1,70 (5H); 1,90 (2H); 2,10 (2H); 2,22 (2H); 2,40 (4H); 2,95 (1H); 4,52 (2H); 6,81 (1H); 7,10-7,25 (5H); 7,45 (2H); 7,60 (2H) ppm.

### Bsp. 21: Herstellung von 4-(4-Phenoxyphenyl)-6-(4-piperazin-1-yl-cyclohexyl)-1H-pyrazolo[3,4b]pyridin-3-ylamin

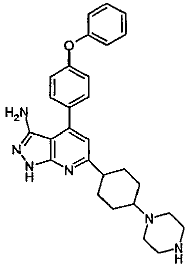

Analog zu Beispiel 14 werden durch Umsetzung von 20 mg der unter Beispiel 20 beschriebenen Substanz mit 300 µl HCl Lösung (2 molar in Diethylether) in Dichlormethan 9 mg Produkt erhalten.
¹H-NMR (d6-DMSO): δ = 1,75-2,00 (8H); 3,10-4,00 (12H); 7,05-7,20 (5H); 7,45 (2H); 7,68 (2H); 9,70 (1 H); 11,20 (1 H) ppm.7,20 (1 H); 7,38 (2H); 7,60 (2H); 9,80 (2H) ppm.

### Bsp. 22: Herstellung von 6-[4-(4-Methyl-piperazin-1-yl)-cyclohexyl]-4-(4-phenoxyphenyl)-1 H-pyrazolo[3,4b]pyridin-3-ylamin

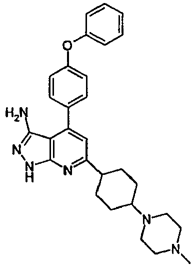

Analog zu Beispiel 13 werden aus 37 mg der unter Beispiel 19) beschriebenen Verbindung, 52 µl N-Methylpiperazin, 27 µl Eisessig und 25 mg Natriumtriacetoxyborhydrid 32 mg Produkt erhalten.
¹H-NMR (CDCl₃): δ = 1,60 (2H); 1,95 (2H); 2,20-2,35 (4H); 2,29 (3H); 2,40-2,65 (8H); 3,00 (1H); 3,92 (2H); 6,89 (1 H); 7,05-7,22 (5H); 7,40 (2H); 7,55 (2H); 9,74 (1 H) ppm.

### Bsp. 23: Herstellung von 4-(4-Phenoxy-phenyl)-6-(4-piperidin-1-yl-cyclohexyl)-1H-pyrazolo[3,4b]pyridin-3-ylamin

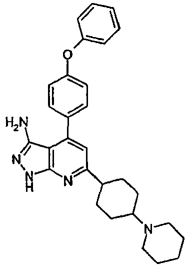

Analog zu Beispiel 13 werden aus 100 mg der unter Beispiel 19) beschriebenen Verbindung, 125 µl Piperidin, 72 µl Eisessig und 60 mg Natriumtriacetoxyborhydrid 58 mg Produkt erhalten.
¹H-NMR (d6-DMSO): δ = 1,10-2,00 (10H); 2,05-2,20 (4H); 2,40-2,60 (4H); 2,97 (1H); 4,52 (2H); 6,82 (1 H); 7,05-7,25 (5H); 7,43 (2H); 7,60 (2H); 12,12 (1H) ppm.

### Bsp. 24: Herstellung von 6-(4-Morpholin-4-yl-cyclohexyl)-4-(4-phenoxyphenyl)-1H-pyrazolo[3,4b]pyridin-3-ylamin

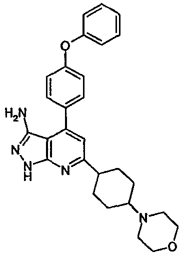

Analog zu Beispiel 13 werden aus 90 mg der unter Beispiel 19 beschriebenen Verbindung, 100 µl Morpholin, 63 µl Eisessig und 50 mg Natriumtriacetoxyborhydrid 52 mg Produkt erhalten.
¹H-NMR (d6-DMSO): δ = 1,50-1,70 (4H); 1,80-2,20 (6H); 2,40 (4H); 2,98 (1H); 3,60 (4H); 4,52 (2H); 6,81 (1H); 7,10-7,25 (5H); 7,45 (2H); 7,60 (2H) 12,12 (1H) ppm.

### Bsp. 25: Herstellung von 6-(1,1-Dimethyl-2-piperidin-1-yl-ethyl)-4-p-tolyl-1H-pyrazolo[3,4b]pyridin-3-ylamin

### Bsp. 25a) Herstellung von 3-(tert-Butyldimethylsilyloxy)-2,2-dimethyl-propan-1-ol

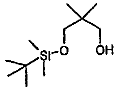

Zu einer Suspension von 3,85 g Natriumhydrid (60%ig) in 30 ml Tetrahydrofuran wird eine Lösung von 10 g 2,2-Dimethyl-1,3-propandiol in 100 ml Tetrahydrofuran getropft. Man lässt 45 Minuten nachrühren und addiert dann 14,5 g *tert*-Butyldimethylsilylchlorid. Anschließend wird eine Stunde bei 25°C nachgerührt. Danach wird das Reaktionsgemisch auf gesättigte Natriumhydrogencarbonatlösung gegossen. Man extrahiert mit Ethylacetat, wäscht die organische Phase mit gesättigter Natriumchloridlösung, trocknet über Natriumsulfat und engt im Vakuum ein. Das Rohprodukt wird durch Säulenchromatographie an Kieslegel gereinigt. Man erhält 18,4 g Produkt.
¹H-NMR (CDCl₃): δ = 0,05 (6H); 0,90 (15H); 2,86 (1 H); 3,49 (4H) ppm.

### Bsp. 25b) Herstellung von 3-(tert-Butyldimethylsilyloxy)-2,2-dimethyl-propan-1-al

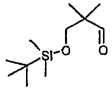

Analog zu Beispiel 11c) werden aus 1 g der unter Beispiel 25a) beschriebenen Verbindung, 910 µl Dimethylsulfoxid, 555 µl Oxalylchlorid und 2,8 ml Triethylamin in Dichlormethan 890 mg Rohprodukt erhalten, welches ohne Aufreinigung in die Folgestufe eingesetzt wird.
¹H-NMR (CDCl₃): δ = 0,03 (6H); 0,89 (9H); 1.02 (6H); 3,61 (2H); 9,57 (1H) ppm.

### Bsp. 25c) Herstellung von 4-(tert-Butyldimethylsilyloxy)-3,3-dimethyl-butan-2-ol

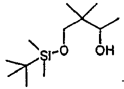

Analog zu Beispiel 11d) werden aus 2,6 g der unter Beispiel 25b) beschriebenen Verbindung und 10,8 ml einer 1,6 molaren Lösung von Methyllithium in Diethylether nach Säulenchromatographie 2,08 g Produkt erhalten.
¹H-NMR (CDCl₃): δ = 0,09 (6H); 0,80 (3H); 0,90 (12H); 1,10 (3H); 3,48 (2H); 3,70 (1H); 3,82 (1H) ppm.

### Bsp: 25d) Herstellung von 4-(tert-Butyldimethylsilyloxy)-3,3-dimethyl-butan-2-on

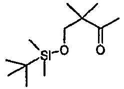

Analog zu Beispiel 11e) werden aus 2,1 g der unter Beispiel 25c) beschriebenen Verbindung, 2 g N-Methylmorpholin-N-oxid und 158mg Tetrapropylammoniumperuthenat in Dichlormethan 1,61 g Produkt erhalten.
¹H-NMR (CDCl₃): δ = 0,02 (6H); 0,87 (9H); 1,10 (6H); 2,17 (3H); 3,58 (2H) ppm.

### Bsp. 25e) Herstellung von 6-(2-Hydroxy-1,1-dimethylethyl)-2-oxo-4-p-tolyl-1,2-dihydro-pyridin-3-carbonitril

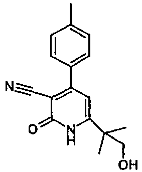

Analog zu Beispiel 1a) werden aus 16,6 g Ammoniumacetat, 2,9 ml Cyanessigsäureethylester, 6,2 g der unter Beispiel 25d) beschriebenen Verbindung und 3,2 ml 4-Methylbenzaldehyd 4,8 g Produkt erhalten.
¹H-NMR (d6-DMSO): δ = 1,23 (6H); 2,39 (3H); 3,57 (2H); 6,27 (1 H); 7,37 (2H); 7,53 (2H) ppm.

### Bsp. 25f) Herstellung von 6-(1,1-Dimethyl-2-oxo-ethyl)-2-oxo-4-p-tolyl-1,2-dihydro-pyridin-3-carbonitril

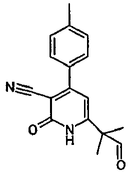

Zu einer Lösung aus 400 mg der unter Beispiel 25e) beschrieben Verbindung, 1,5 ml Dimethylsulfoxid und 980 µl Triethylamin in 8 ml Dichlormethan wird bei 0°C eine Lösung von 680 mg Schwefeltrioxid-Pyridin Komplex in 7 ml Dimethylsulfoxid addiert. Man rührt 3 Stunden bei 0°C nach. Dann wird Wasser zum Reaktionsgemisch addiert. Man extrahiert mit Dichlormethan, wäscht mit gesättigter Natriumchloridlösung, trocknet über Natriumsulfat und engt im Vakuum ein. Nach Säulenchromatographie an Kieslegel werden 232 mg Produkt erhalten.
¹H-NMR (CDCl₃): δ = 1,49 (6H); 2,40 (3H); 6,39 (1 H); 7,28 (2H); 7,50 (2H); 9,65 (1 H) ppm.

### Bsp. 25g) Herstellung von 6-(1,1-Dimethyl-2-piperidin-1-yl-ethyl)-2-oxo-4-p-tolyl-1,2-dihydro-pyridin-3-carbonitril

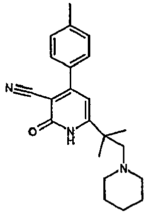

Analog zu Beispiel 13 werden aus 184 mg der unter Beispiel 25f) beschriebenen Verbindung, 325 µl Piperidin und 192 mg Natriumtriacetoxyborhydrid 118 mg Produkt erhalten.
¹H-NMR (CDCl₃): δ = 1,38 (6H); 1,40-1,80 (6H); 2,41 (3H); 2,51 (2H); 2,60 (4H); 6,09 (1H); 7,30 (2H); 7,50 (2H) ppm.

### Bsp. 25h) Herstellung von 3-Cyano-6-(1,1-dimethyl-2-piperidin-1-yl-ethyl)-4-p-tolyl-2-trifluormethansulfonylpyridin

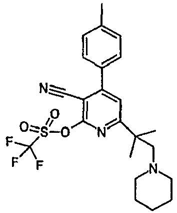

Analog zu Beispiel 3b) werden aus 87 mg der unter Beispiel 25g) beschriebenen Substanz und 92 µl Trifluormethansulfonsäureanhydrid in Pyridin 110 mg Produkt erhalten.
¹H-NMR (CDCl₃): δ = 1,40 (6H); 1,60 (6H); 2,10-2,30 (4H); 2,47 (3H); 2,53 (2H); 7,37 (2H); 7,52 (3H) ppm.

### Bsp. 25i) Herstellung von 6-(1,1-Dimethyl-2-piperidin-1-yl-ethyl)-4-p-tolyl-1H-pyrazolo[3,4b]pyridin-3-ylamin

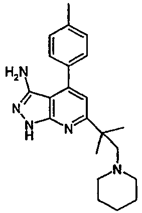

Analog zu Beispiel 1c) werden aus 106 mg der unter Beispiel 25h) beschriebenen Verbindung mit 41 µl Hydrazinhydrat-Lösung (80%ig) in Propanol 45 mg Produkt hergestellt.
¹H-NMR (CDCl₃): δ = 1,40 (6H); 1,50-1,70 (6H); 2,20-2,35 (4H); 2,49 (3H); 2,60 (2H); 3,93 (2H); 7,07 (1 H); 7,35 (2H); 7,48 (2H); 9,70 (1 H) ppm.

### Bsp. 26: Herstellung von 6-(1,1-Dimethyl-2-morpholin-1-yl-ethyl)-4-p-tolyl-1H-pyrazolo[3,4b]pyridin-3-ylamin

### Bsp. 26a) Herstellung von 6-(1,1-Dimethyl-2-morpholin-4-yl-ethyl)-2-oxo-4-p-tolyl-1,2-dihydro-pyridin-3-carbonitril

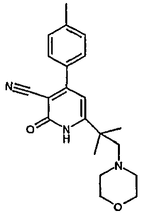

Analog zu Beispiel 13 werden aus 181 mg der unter Beispiel 25f) beschriebenen Verbindung, 282 µl Morpholin und 180 mg Natriumtriacetoxyborhydrid 139 mg Produkt erhalten.
¹H-NMR (CDCl₃): δ = 1,30 (6H); 2,42 (3H); 2,70 (4H); 3,83 (4H); 6,13 (1H); 7,30 (2H); 7,51 (2H) ppm.

### Bsp. 26b) Herstellung von 3-Cyano-6-(1,1-dimethyl-2-morpholin-1-yl-ethyl)-4-p-tolyl-2-trifluormethansulfonylpyridin

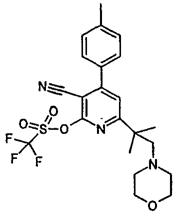

Analog zu Beispiel 3b) werden aus 135 mg der unter Beispiel 26a) beschriebenen Substanz und 142 µl Trifluormethansulfonsäureanhydrid in Pyridin 165 mg Produkt erhalten.
¹H-NMR (CDCl₃): δ = 1,37 (6H); 2,30 (4H); 2,62 (3H); 3,50 (4H); 7,40-7,55 (5H) ppm.

### Bsp. 26c) Herstellung von 6-(1,1-Dimethyl-2-morpholin-1-yl-ethyl)-4-p-tolyl-1H-pyrazolo[3,4b]pyridin-3-ylamin

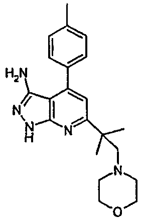

Analog zu Beispiel 1c) werden aus 165 mg der unter Beispiel 26b) beschriebenen Verbindung mit 70 µl Hydrazinhydrat-Lösung (80%ig) in Propanol 65 mg Produkt hergestellt.
¹H-NMR (d6-DMSO): δ = 1,35 (6H); 2,20 (4H); 2,39 (3H); 2,62 (2H); 3,40 (4H); 4,47 (2H); 6,97 (1 H); 7,38 (2H); 7,46 (2H) ppm.

### Bsp. 27: Herstellung von 6-[1,1-Dimethyl-2-(4-methyl-piperazin-1-yl)-ethyl]-4-p-tolyl-1H-pyrazolo[3,4b]pyridin-3-ylamin

### Bsp. 27a) Herstellung von 6-(1,1-Dimethyl-2-(4-methyl-piperazin-1-yl)-ethyl]-2-oxo-4-p-tolyl-1,2-dihydro-pyridin-3-carbonitril

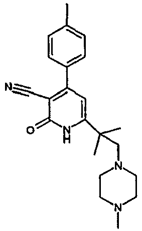

Analog zu Beispiel 13 werden aus 106 mg der unter Beispiel 25f) beschriebenen Verbindung, 210 µl N-Methylpiperazin und 100 mg Natriumtriacetoxyborhydrid 68 mg Produkt erhalten.
¹H-NMR (d6-DMSO): δ = 1,24 (6H); 2,13 (3H); 2,30 (4H); 2,38 (3H); 2,45 (4H); 2,58 (2H); 6,23 (1H); 7,38 (2H); 7,55 (2H); 12,52 (1H) ppm.

### Bsp. 27b) Herstellung von 3-Cyano-6-[1,1-dimethyl-2-(4-methyl-piperazin-1-yl)-ethyl)]-4-p-tolyl-2-trifluormethansulfonylpyridin

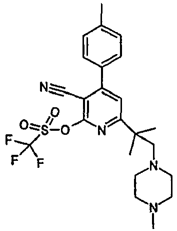

Analog zu Beispiel 3b) werden aus 44 mg der unter Beispiel 27a) beschriebenen Substanz und 45 µl Trifluormethansulfonsäureanhydrid in Pyridin 50 mg Produkt erhalten.
¹H-NMR (CDCl₃): δ = 1,37 (6H); 1,98 (4H); 2,30 (3H); 2,35 (3H); 2,43 (4H); 2,68 (2H); 7,40 (2H); 7,50 (3H) ppm.

### Bsp. 27c) Herstellung von 6-[1,1-Dimethyl-2-(4-methyl-piperazin-1-yl)-ethyl]-4-p-tolyl-1H-pyrazolo[3,4b]pyridin-3-ylamin

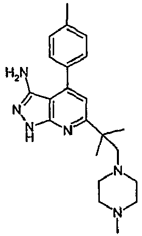

Analog zu Beispiel 1c) werden aus 50 mg der unter Beispiel 27b) beschriebenen Verbindung mit 22 µl Hydrazinhydrat-Lösung (80%ig) in Propanol 24 mg Produkt hergestellt.
¹H-NMR (d6-DMSO): δ = 1,33 (6H); 2,10 (3H); 2,24 (4H); 2,40 (3H); 2,55 (4H); 2,62 (2H); 4,46 (2H); 6,95 (1H); 7,37 (2H); 7,48 (2H); 12,12 (1H) ppm.

### Bsp 28: Herstellung von 4-[2-(3-Amino-4-p-tolyl-1H-pyrazolo[3,4b]pyridin-6-yl)-2-methylpropyl]-piperazin-1-carbonsäure tert-butyl ester

### Bsp. 28a) Herstellung von 4-[2-(5-Cyano-6-oxo-4-p-tolyl-1,6-dihydro-pyridin-2-yl)-2-methylpropyl]-piperazin-1-carbonsäure tert-butyl ester

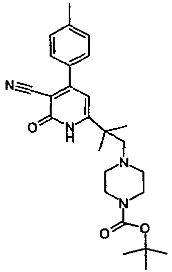

Analog zu Beispiel 13 werden aus 182 mg der unter Beispiel 25f) beschriebenen Verbindung, 605 mg 1-*tert*-Butoxycarbonylpiperazin und 150 mg Natriumtriacetoxyborhydrid 245 mg Produkt erhalten.
¹H-NMR (CDCl₃): δ = 1,30 (6H); 1,41 (9H); 2,41 (3H); 2,60 (6H); 3,59 (4H); 6,12 (1H); 7,30 (2H); 7,50 (2H); 12,40 (1 H) ppm.

### Bsp. 28b) Herstellung von 4-[2-(5-Cyano-4-p-tolyl-6-trifluoromethansulfonyloxy-pyridin-2-yl)-2-methyl-propyl]-piperazin-1-carbonsäure tert-butyl ester

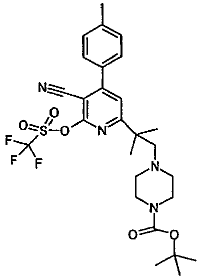

Analog zu Beispiel 3b) werden aus 240 mg der unter Beispiel 28a) beschriebenen Substanz und 200 µl Trifluormethansulfonsäureanhydrid in Pyridin 301 mg Produkt erhalten.
¹H-NMR (CDCl₃): δ = 1,35 (6H); 1,41 (9H); 2,26 (4H); 2,45 (3H); 2,63 (2H); 3,23 (4H); 7,38 (2H); 7,50 (3H) ppm.

### Bsp. 28c) Herstellung von 4-[2-(3-Amino-4-p-tolyl-1H-pyrazolo[3,4b]pyridin-6-yl)-2-methyl-propyl]-piperazin-1-carbonsäure tert-butyl ester

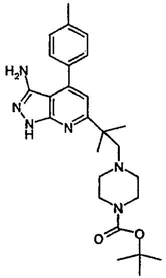

Analog zu Beispiel 1c) werden aus 300 mg der unter Beispiel 28b) beschriebenen Verbindung mit 97 µl Hydrazinhydrat-Lösung (80%ig) in Propanol 168 mg Produkt hergestellt.
¹H-NMR (CDCl₃): δ = 1,41 (9H); 1,43 (6H); 2,25 (4H); 2,46 (3H); 2,69 (2H); 3,23 (4H); 3,93 (2H); 7,03 (1 H); 7,35 (2H); 7,48 (2H); 9,98 (1 H) ppm.

### Bsp. 29: Herstellung von 6-(1,1-Dimethyl-2-piperazin-1-yl-ethyl)-4-p-tolyl-1H-pyrazolo[3,4b]pyridin-3-ylamin

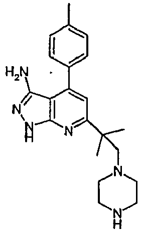

Analog zu Beispiel 14 werden durch Umsetzung von 80 mg der unter Beispiel 28c) beschriebenen Substanz mit 430 µl HCl Lösung (2 molar in Diethylether) in Dichlormethan 43 mg Produkt erhalten.
¹H-NMR (d6-DMSO): δ = 1,50 (6H); 2,41 (3H); 2,50 (6H); 3,45 (4H); 3,70 (2H); 7,20 (1H); 7,40 (2H); 7,60 (2H); 9,95 (1 H) ppm.

### Bsp. 30: Herstellung von 6-(1,1-Dimethyl-2-(4-methyl-piperazin-1-yl)-ethyl]-4-(4-phenoxyphenyl)-1H-pyrazolo[3,4b]pyridin-3-ylamin

### Bsp. 30a) Herstellung von 6-(2-Hydroxy-1,1-dimethylethyl)-2-oxo-4-(4-phenoxyphenyl)-1,2-dihydro-pyridin-3-carbonitril

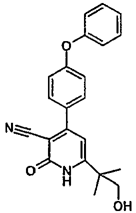

Analog zu Beispiel 1a) werden aus 6,7 g Ammoniumacetat, 1,15 ml Cyanessigsäureethylester, 2,5 g der unter Beispiel 25d) beschriebenen Verbindung und 2,15 g 4-Phenoxybenzaldehyd 1,85 g Produkt erhalten.
¹H-NMR (d6-DMSO): δ = 1,24 (6H); 3,55 (2H); 6,28 (1H); 7,10 (4H); 7,23 (1H); 7,46 (2H); 7,68 (2H) ppm.

### Bsp. 30b) Herstellung von 6-(1,1-Dimethyl-2-oxo-ethyl)-2-oxo-4-(4-phenoxyphenyl)-1,2-dihydro-pyridin-3-carbonitril

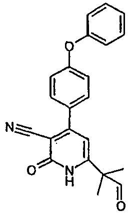

Analog zu Beispiel 25f) werden aus 442 mg der unter Beispiel 30a) beschriebenen Verbindung, 585 mg Schwefeltrioxid-Pyridin Komplex und 850 µl Triethylamin in Dimethylsulfoxid 184 mg Produkt erhalten.
¹H-NMR (CDCl₃): δ = 1,60 (6H); 6,27 (1H); 7,10 (4H); 7,20 (1H); 7,40 (2H); 7,59 (2H); 9,70 (1H); 12,55 (1 H) ppm.

### Bsp. 30c) Herstellung von 6-[1,1-Dimethyl-2-(4-methylpiperazin-1-yl)-ethyl]-2-oxo-4-(4-phenoxyphenyl)-1,2-dihydro-pyridin-3-carbonitril

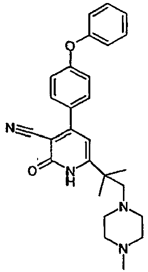

Analog zu Beispiel 13 werden aus 130 mg der unter Beispiel 30b) beschriebenen Verbindung, 200 µl N-Methylpiperazin und 100 mg Natriumtriacetoxyborhydrid 101 mg Produkt erhalten.
¹H-NMR (CDCl₃): δ = 1,31 (6H); 1,63 (4H); 2,32 (3H); 2,58 (2H); 2,62 (2H); 2,72 (2H); 6,10 (1H); 7,08 (4H); 7,19 (1 H); 7,40 (2H); 7,58 (2H); 12,70 (1 H) ppm.

### Bsp. 30d) Herstellung von 3-Cyano-6-[1,1-dimethyl-2-(4-methylpiperazin-1-yl)-ethyl)-4-(4-phenoxyphenyl)-2-trifluormethansulfonylpyridin

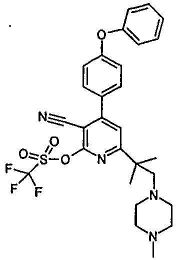

Analog zu Beispiel 3b) werden aus 96 mg der unter Beispiel 30c) beschriebenen Substanz und 80 µl Trifluormethansulfonsäureanhydrid in Pyridin 105 mg Produkt erhalten.
¹H-NMR (CDCl₃): δ = 1,37 (6H); 1,76 (4H); 2,27 (3H); 2,40 (4H); 2,67 (2H); 7,05-7,28 (6H); 7,40 (2H); 7,60 (2H) ppm.

### Bsp. 30e) Herstellung von 6-(1,1-Dimethyl-2-(4-methyl-piperazin-1-yl)-ethyl]-4-(4-phenoxyphenyl)-1H-pyrazolo[3,4b]pyridin-3-ylamin

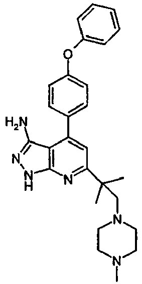

Analog zu Beispiel 1c) werden aus 134 mg der unter Beispiel 30d) beschriebenen Verbindung mit 40 µl Hydrazinhydrat-Lösung (80%ig) in Propanol 49 mg Produkt hergestellt.
¹H-NMR (d6-DMSO): δ = 1,33 (6H); 2,05 (3H); 2,13 (4H); 2,27 (4H); 2,60 (2H); 4,52 (2H); 7,03-7,25 (6H); 7,43 (2H); 7,60 (2H) ppm.

### Bsp. 31: Herstellung von 6-(1,1-Dimethyl-2-morpholin-4-yl-ethyl)-4-(4-phenoxyphenyl)]-1H-pyrazolo[3,4b]pyridin-3-ylamin

### Bsp. 31a) Herstellung von 6-(1,1-Dimethyl-2-morpholin-4-yl-ethyl)-2-oxo-4-(4-phenoxyphenyl)-1,2-dihydro-pyridin-3-carbonitril

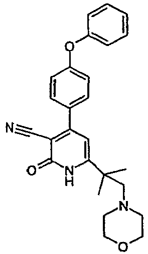

Analog zu Beispiel 13 werden aus 145 mg der unter Beispiel 30b) beschriebenen Verbindung, 180 µl Morpholin und 100 mg Natriumtriacetoxyborhydrid 122 mg Produkt erhalten.
¹H-NMR (d6-DMSO): δ = 1,28 (6H); 2,40 (4H); 2,59 (2H); 3,53 (4H); 6,27 (1H); 7,12 (4H); 7,22 (1H); 7,48 (2H); 7,68 (2H); 12,42 (1H) ppm.

### Bsp. 31 b) Herstellung von 3-Cyano-6-(1,1-dimethyl-2-morpholin-4-yl-ethyl)-4-(4-phenoxyphenyl)-2-trifluormethansulfonylpyridin

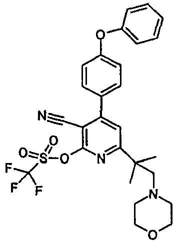

Analog zu Beispiel 3b) werden aus 118 mg der unter Beispiel 31a) beschriebenen Substanz und 102 µl Trifluormethansulfonsäureanhydrid in Pyridin 139 mg Produkt erhalten.
¹H-NMR (CDCl₃): δ = 1,37 (6H); 2,30 (4H); 2,65 (2H); 3,53 (4H); 7,18-7,30 (5H); 7,41 (2H); 7,49 (1H); 7,61 (2H)

### Bsp. 31c) Herstellung von 6-(1,1-Dimethyl-2-morpholin-4-yl-ethyl)-4-(4-phenoxyphenyl)]-1H-pyrazolo[3,4b]pyridin-3-ylamin

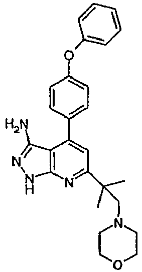

Analog zu Beispiel 1c) werden aus 135 mg der unter Beispiel 31 b) beschriebenen Verbindung mit 50 µl Hydrazinhydrat-Lösung (80%ig) in Propanol 89 mg Produkt hergestellt.
¹H-NMR (CDCl₃): δ = 1,45 (6H); 2,30 (4H); 2,70 (2H); 3,53 (4H); 3,95 (2H); 7,05 (1H); 7,08-7,23 (5H); 7,40 (2H); 7,55 (2H) ppm.

### Bsp. 32 Herstellung von 3-(3-Amino-6-pyridin-4-yl-1H-pyrazolo[3,4-b]pyridin-4-yl)-phenol

### Bsp. 32a) Herstellung von 4-(3-Hydroxyphenyl)-6-oxo-1,6-dihydro-[2,4']bipyridinyl-5-carbonitril

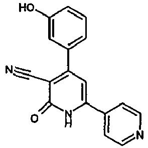

Analog zu Beispiel 1a werden aus 7,57 g Ammoniumacetat, 1,31 ml Cyanessigsäureethylester, 1,36 ml 4-Acetylpyridin und 1,5 g 3-Hydroxybenzaldehyd 1,82 g Produkt erhalten.
¹H-NMR (d6-DMSO): δ = 6,96 (1 H); 7,00-7,20 (3H); 7,48 (1H); 7,90 (2H); 8,72 (2H); 9,88 (1H); 12,97 (1H) ppm.

### Bsp. 32b) Herstellung von Essigsäure 3-(5-cyano-6-oxo-1,6-dihydro-[2,4']bipyridinyl-4-yl)-phenylester

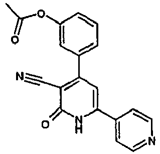

580 mg der unter Bsp. 32a beschriebenen Verbindung werden in 6 ml Pyridin gelöst. Man addiert 0,5 ml Essigsäureanhydrid und lässt 2 Stunden bei 80°C nachrühren. Nach dem abkühlen werden 20 ml Wasser addiert. Man rührt eine Stunde nach und saugt dann den Niederschlag ab. Es werden 564 mg Produkt erhalten.
¹H-NMR (d6-DMSO): δ = 2,31 (3H); 7,11 (1H); 7,37 (1H); 7,55 (1H); 7,64 (2H); 7,92 (2H); 8,76 (2H); 13,02 (1H) ppm.

### Bsp. 32c) Herstellung von Essigsäure 3-(5-cyano-6-trifluoromethansulfonyloxy-[2,4']bipyridinyl-4-yl)-phenyl ester

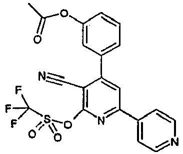

Analog zu Beispiel 3b werden aus 200 mg der unter Beispiel 32b beschriebenen Substanz und 305 µl Trifluormethansulfonsäure in Pyridin 202 mg Produkt erhalten.
¹H-NMR (CDCl₃): δ = 2,38 (3H); 7,36 (1H); 7,41 (1H); 7,55-7,68 (2H); 7,91 (1H); 8,00 (1H); 8,86 (1 H) ppm.

### Bsp. 32d) Herstellung von 3-(3-Amino-6-pyridin-4-yl-1 H-pyrazolo[3,4-b]pyridin-4-yl)-phenol

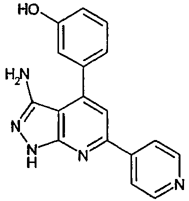

Analog zu Beispiel 1c werden aus 197 mg der unter Beispiel 32c beschriebenen Substanz mit 80 µl Hydrazinhydrat-Lösung (80%ig) in Propanol 65 mg Produkt erhalten.
¹H-NMR (d6-DMSO): δ = 6,93 (1H); 7,05 (2H); 7,36 (1H); 7,42 (1H); 8,20 (1H); 8,48 (1H); 8,71 (2H); 9,82 (1H) ppm.

### Bsp. 33 Herstellung von 3-(3-Amino-6-pyridin-4-yl-1H-pyrazolo[3,4-b]pyridin-4-yl)-5-methoxyphenol

### Bsp. 33a) Herstellung von 2-(3,5-Dimethoxyphenyl)-[1,3]dioxolan

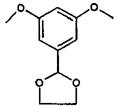

Zu einer Lösung von 5 g 3,5 Dimetoxybenzaldehyd in 100 ml Dichlormethan werden 4,2 ml Ethylenglykol, 4,1 ml Trimethylorthoformiat und 5 mg p-Toluolsulfonsäure addiert. Man lässt 20 Stunden bei 25°C nachrühren. Danach wird mit gesättigter Natriumhydrogencarbonatlösung und gesättigter Natriumchloridlösung gewaschen, über Natriumsulfat getrocknet und im Vakuum eingeengt. Das Rohprodukt wird durch Säulenchromatographie gereinigt. Man erhält 5,67 g Produkt.
¹H-NMR (CDCl₃): δ = 3,80 (6H); 4,00-4,15 (4H); 5,77 (1H); 6,45 (1H); 6,64 (2H) ppm.

### Bsp. 33b) Herstellung von 3-[1,3]Dioxolan-2-yl-5-methoxyphenol

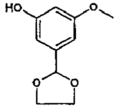

5,67 g der unter Beispiel 33a beschriebenen Substanz werden in 100 ml Dimethylformamid gelöst. Man addiert 7,6 g Natriummethanthiolat und kocht 4 Stunden unter Rückfluß. Anschließend wird das Reaktionsgemisch auf Eiswasser gegossen. Man lässt eine Stunde nachrühren und extrahiert dann mit Ethylacetat. Die organische Phase wird mit gesättigter Natriumchloridlösung gewaschen, über Natriumsulfat getrocknet und im Vakuum eingeengt. Das Rohprodukt wird durch Säulenchromatographie gereinigt. Man erhält 2,84 g Produkt.
¹H-NMR (CDCl₃): δ = 3,80 (3H); 4,00-4,15 (4H); 5,18 (1H); 5,73 (1H); 6,40 (1H); 6,55 (1H); 6,61 (1H) ppm.

### Bsp. 33c) Herstellung von 3-Hydroxy-5-methoxy-benzaldehyd

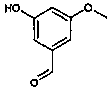

Zu einer Lösung von 450 mg der unter Beispiel 33b beschriebenen Substanz in 10 ml Aceton werden 300 ml 2 normale Salzsäure addiert. Man lässt 30 Minuten bei 25°C rühren, verdünnt dann mit Dichlormethan. Die organische Phase wird mit gesättigter Natriumchloridlösung, über Natriumsulfat getrocknet und im Vakuum eingeengt. Das Rohprodukt wird durch Säulenchromatographie gereinigt. Man erhält 207 mg Produkt.
¹H-NMR (d6-DMSO): δ = 3,79 (3H); 6,65 (1H); 6,90 (2H); 9,85 (1H); 10,00 (1H) ppm.

### Bsp. 33d) Herstellung von 4-(3-Hydroxy-5-methoxyphenyl)-6-oxo-1,6-dihydro-[2,4']bipyridinyl-5-carbonitril

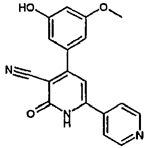

Analog zu Beispiel 1a werden aus 450 mg der unter Beispiel 33c beschriebenen Verbindung, 1,84 g Ammoniumacetat, 320 µl Cyanessigsäureethylester und 330 µl 4-Acetylpyridin 477 mg Produkt erhalten.
¹H-NMR (d6-DMSO): δ = 3,77 (3H); 6,51 (1H); 6,70 (2H); 7,05 (1H); 7,90 (2H); 8,72 (2H); 9,11 (1 H) ppm.

### Bsp. 33e) Herstellung von Essigsäure 3-(5-cyano-6-oxo-1,6-dihydro-[2,4']bipyridinyl-4-yl)-5-methoxyphenyl ester

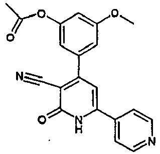

Analog zu Beispiel 32b werden aus 200 mg der unter Beispiel 33d beschriebenen Verbindung und 160 µl Essigsäureanhydrid in Pyridin 204 mg Produkt erhalten.
¹H-NMR (d6-DMSO): δ = 2,29 (3H); 3,34 (3H); 6,99 (1H); 7,11 (2H); 7,22 (1H); 7,93 (2H); 8,75 (2H) ppm.

### Bsp. 33f) Herstellung von Essigsäure 3-(5-cyano-6-trifluoromethan-sulfonyloxy-[2,4']bipyridinyl-4-yl)-5-methoxyphenyl ester

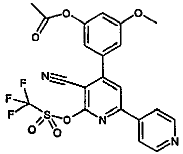

Analog zu Beispiel 3b werden aus 200 mg der unter Beispiel 33e beschriebenen Substanz und 280 µl Trifluormethansulfonsäure in Pyridin 186 mg Produkt erhalten.
¹H-NMR (CDCl₃): δ = 2,33 (3H); 3,90 (3H); 6,89 (1H); 7,00 (1H); 7,06 (1H); 7,91 (2H); 7,99 (1H); 8,85 (2H) ppm.

### Bsp. 33g) Herstellung von 3-(3-Amino-6-pyridin-4-yl-1H-pyrazolo[3,4-b]pyridin-4-yl)-5-methoxyphenol

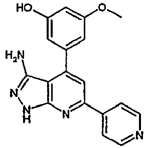

Analog zu Beispiel 1c werden aus 181 mg der unter Beispiel 33f beschriebenen Substanz mit 70 µl Hydrazinhydrat-Lösung (80%ig) in Propanol 67 mg Produkt erhalten.¹H-NMR (d6-DMSO): δ = 3,78 (3H); 6,50 (1H); 6,62 (2H); 7,43 (1H); 8,20 (2H); 8,45 (1H); 8,70 (2H); 9,85 (1H) ppm.

### Bsp. 34 Herstellung von 4-(3,5-Dimethoxy-phenyl)-6-pyridin-4-yl-1H-pyrazolo[3,4-b]pyridin-3-ylamin

### Bsp. 34a) Herstellung von 4-(3,5-Dimethoxyphenyl)-6-oxo-1,6-dihydro-[2,4']bipyridinyl-5-carbonitril

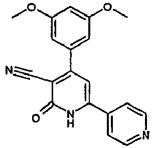

Analog zu Beispiel 1a werden aus 5,57 g Ammoniumacetat, 960 µl Cyanessigsäureethylester, 1 ml 4-Acetylpyridin und 1,5 g 3,5-Dimetoxybenzaldehyd 1,49 g Produkt erhalten.
¹H-NMR (d6-DMSO): δ = 3,80 (6H); 6,60 (1 H); 6,76 (2H); 6,87 (1 H); 7,95 (2H); 8,65 (2H) ppm.

### Bsp. 34b) Herstellung von Trifluoromethanesulfonsäure-5-cyano-4-(3,5-dimethoxy-phenyl)-[2,4']bipyridinyl-6-yl ester

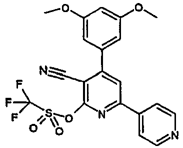

Analog zu Beispiel 3b werden aus 240 mg der unter Beispiel 34a beschriebenen Substanz und 360 µl Trifluormethansulfonsäure in Pyridin 302 mg Produkt erhalten.
¹H-NMR (d6-DMSO): δ = 3,85 (6H); 6,79 (1H); 7,04 (2H); 8,17 (2H); 8,61 (1H); 8,85 (1H) ppm.

### Bsp. 34c) Herstellung von 4-(3,5-Dimethoxy-phenyl)-6-pyridin-4-yl-1H-pyrazolo[3,4-b]pyridin-3-ylamin

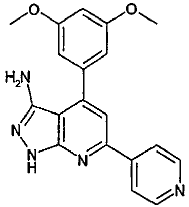

Analog zu Beispiel 1c werden aus 297 mg der unter Beispiel 34b beschriebenen Substanz mit 120 µl Hydrazinhydrat-Lösung (80%ig) in Propanol 76 mg Produkt erhalten.
¹H-NMR (d6-DMSO): δ = 3,85 (6H); 4,73 (2H); 6,68 (1H); 6,84 (2H); 7,65 (1H); 8,15 (2H); 8,71 (2H); 12,51 (1H) ppm.

### Bsp. 35 Herstellung von 3-(3-Amino-6-pyridin-4-yl-1H-pyrazolo[3,4-b]pyridin-4-yl)-4-methyl-phenol

### Bsp. 35a) Herstellung von 2-Methyl-5-nitro-benzoesäuremethylester

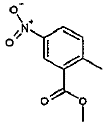

Zu einer Suspension von 2 g 2-Methyl-5-nitrobenzoesäure in 7 ml Methanol werden 300 µl konzentrierte Schwefelsäure gegeben. Man kocht 6 Stunden unter Rückfluß. Anschließend wird das Reaktionsgemisch mit Ethylacetat verdünnt und auf gesättigte Natriumhydrogencarbonatlösung gegossen. Man trennt die Phasen und extrahiert wäßrige Phase mit Ethylacetat. Die vereinigten organischen Phasen werden dann mit gesättigter Natriumchloridlösung gewaschen, über Natriumsulfat getrochnet und im Vakuum eingeengt. Das erhaltene Rohprodukt wird ohne Aufreinigung in die Folgestufe eingesetzt.
¹H-NMR (CDCl₃): δ = 2,70 (3H); 3,95 (3H); 7,42 (1 H); 8,23 (1 H); 8,78 (1 H) ppm.

### Bsp. 35b) Herstellung von 5-Amino-2-methylbenzoesäure-methylester

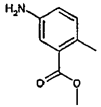

3,17 g der unter Beispiel 35a beschriebenen Verbindung werden in einem Gemisch aus 20 ml Tetrahydrofuran und 5 ml Ethanol mit 625 mg Palladium/Kohle (10%) unter Wasserstoff hydriert. Nach kompletter Umsetzung wird über Celite filtriert und im Vakuum eingeent. Das erhaltene Rohprodukt wird durch Säulenchromatographie an Kieselgel mit einem Gemisch aus Ethylacetat/Hexan gereinigt. Man erhält 2,65 g Produkt.
¹H-NMR (CDCl₃): δ = 2,47 (3H); 3,87 (3H); 6,75 (1 H); 7,01 (1 H); 7,25 (1 H) ppm.

### Bsp. 35c) Herstellung von 5-Hydroxy-2-methyl-benzoesäure-methylester

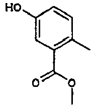

3,17 g der unter Beispiel 35b beschriebenen Verbindung werden in 18 ml 50%iger Schwefelsäure suspendiert. Man kühlt auf -5°C und addeirt 6,4 ml einer 2,5 molaren wäßrigen Natriumnitritlösung mit einer solchen Geschwindigkeit, dass die Innentemperatur nicht über 5°C steigt. Danach wird 1 Stunde bei 25°C und weitere 30 Minuten bei 80°C nachgerührt. Anschließend wird zu der Reaktionslösung langsam gesättigte Natriumhydrogencarbonatlösung addiert. Man extrahiert mit Ethylacetat, wäscht die organische Phase mit gesättigter Natriumchloridlösung, trocknet über Natriumsulfat und engt im Vakuum ein. Das erhaltene Rohprodukt wird durch Säulenchromatographie an Kieselgel mit einem Gemisch aus Ethylacetat/Hexan gereinigt. Man erhält 1,6 g Produkt.
¹H-NMR (CDCl₃): δ = 2,50 (3H); 3,88 (3H); 5,08 (1 H); 6,90 (1 H); 7,10 (1 H); 7,40 (1 H) ppm.

### Bsp. 35d) Herstellung von 5-(tert-Butyldimethylsilanyloxy)-2-methylbenzoesäuremethylester

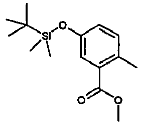

Zu einer Lösung von 770 mg der unter Beispiel 35c beschriebenen Verbindung und 760 mg Imidazol in 20 ml N,N-Dimethylformamid wird 1 g *tert*Butyldimetyhsilylchlorid addiert. Man lässt 2 Stunden bei 25°C nachrühren und gießt dann das Reaktionsgemisch auf gesättigte Natriumhydrogencarbonatlösung. Man extrahiert mit Ethylacetat, wäscht die organische Phase mit gesättigter Natriumchloridlösung, trocknet über Natriumsulfat und engt im Vakuum ein. Das erhaltene Rohprodukt wird durch Säulenchromatographie an Kieselgel mit einem Gemisch aus Ethylacetat/Hexan gereinigt. Man erhält 1,12 g Produkt.
¹H-NMR (CDCl₃): δ = 0,18 (6H); 0,98 (9H); 2,50 (3H); 3,89 (3H); 6,88 (1H); 7,08 (1H); 7,37 (1H) ppm.

### Bsp. 35e) Herstellung von [5-(tert-Butyldimethylsilanyloxy)-2-methyl-phenyl]-methanol

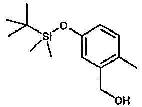

Zu einer Lösung von 250 mg der unter Beispiel 35d beschriebenen Verbindung in 7 ml Toluol werden bei -30°C 2,3 ml einer 1,2 molaren Lösung von Diisobutylaluminiumhydrid in Toluol addiert. Man lässt 1 Stunde bei 0°C nachrühren und addiert dann 1 ml 2-Propanol. Man lässt weitere 10 Minuten nachrühren und addiert 1,3 ml Wasser. Man lässt eine weitere Stunde nachrühren und filtriert dann über Celite. Anschließend wird im Vakuum eingeengt. Das erhaltene Rohprodukt (223 mg) wird ohne Aufreinigung in die Folgestufe eingesetzt.
¹H-NMR (CDCl₃): δ = 0,18 (6H); 0,98 (9H); 2,26 (3H); 4,64 (2H); 6,68 (1H); 6,88 (1H); 7,01 (1H) ppm.

### Bsp. 35f) Herstellung von 5-(tert-Butyldimethylsilanyloxy)-2-methyl-benzaldehyde

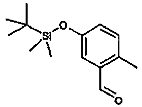

Analog zu der unter Beispiel 25f) beschriebenen Oxidation werden aus 2,2 g der unter Beispiel 35e) beschriebenen Verbindung 2,03 g Produkt erhalten.
¹H-NMR (CDCl₃): δ= 0,19 (6H); 0,99 (9H); 2,58 (3H); 6,97 (1H); 7,11 (1H); 7,26 (1H); 10,21 (1H) ppm.

### Bsp. 35g) Herstellung von 4-[5-(tert-Butyldimethylsilanyloxy)-2-methylphenyl]-6-oxo-1,6-dihydro-[2,4']bipyridinyl-5-carbonitril

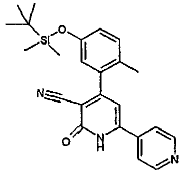

Analog zu Beispiel 1a werden aus 600 mg Ammoniumacetat, 105 µl Cyanessigsäureethylester, 110 µl 4-Acetylpyridin und 240 mg der unter Beispiel 35f beschriebenen Substanz 170 mg Produkt erhalten.
¹H-NMR (CDCl₃): δ = 0,19 (6H); 0,97 (9H); 2,25 (3H); 6,72 (2H); 6,89 (1 H); 7,20 (1 H); 7,79 (2H); 8,86 (2H) ppm.

### Bsp. 35h) Herstellung von Trifluoromethanesulfonsäure-4-[5 -(tert-butyldimethyl-silanyloxy)-2-methyl-phenyl]-5-cyano-[2,4']bipyridinyl-6-yl ester

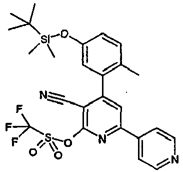

Analog zu Beispiel 3b werden aus 107 mg der unter Beispiel 35g beschriebenen Substanz und 130 µl Trifluormethansulfonsäure in Pyridin 85 mg Produkt erhalten.
¹H-NMR (CDCl₃): δ = 0,21 (6H); 0,98 (9H); 2,19 (3H); 6,73 (1H); 6,92 (1H); 7,23 (1H); 7,86 (1H); 7,91 (2H); 8,82 (2H) ppm.

### Bsp. 35i) Herstellung von 4-[5-(tert-Butyldimethylsilanyloxy)-2-methyl-phenyl]-6-pyridin-4-yl-1H-pyrazolo[3,4-b]pyridin-3-ylamine

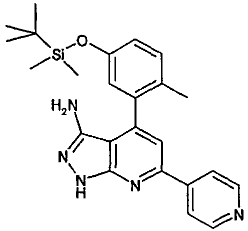

Analog zu Beispiel 1c werden aus 50 mg der unter Beispiel 35h beschriebenen Substanz mit 20 µl Hydrazinhydrat-Lösung (80%ig) in Propanol 27 mg Produkt erhalten.
¹H-NMR (d6-DMSO): δ = 0,21 (6H); 0,93 (9H); 2,07 (3H); 4,38 (2H); 6,79 (1H); 6,93 (1H); 7,30 (1H); 7,52 (1H); 8,13 (2H); 8,70 (2H); 12,45 (1H) ppm.

### Bsp. 35j) Herstellung von 3-(3-Amino-6-pyridin-4-yl-1H-pyrazolo[3,4-b]pyridin-4-yl)-4-methylphenol

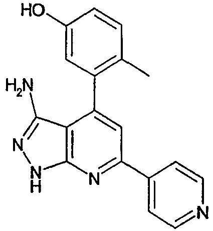

Zu einer Lösung von 24 mg der unter Beispiel 35i beschriebenen Substanz in 5 ml Tetrahydrofuran werden bei 0°C 70 µl einer 1 molaren Lösung von Tetrabutylammoniumfluorid in Tetrahydrofuran addiert. Man lässt eine Stunde nachrühren und gießt dann auf gesättigte Natriumhydrogencarbonatlösung. Man extrahiert mit Ethylacetat, wäscht die organische Phase mit gesättigter Natriumchloridlösung, trocknet über Natriumsulfat und engt im Vakuum ein. Das erhaltene Rohprodukt wird durch Säulenchromatographie an Kieselgel mit einem Gemisch aus Ethylacetat/Hexan gereinigt. Man erhält 9,8 mg Produkt.
¹H-NMR (d6-DMSO): δ = 1,85 (3H); 4,22 (2H); 6,52 (1H); 6,67 (1H); 7,02 (1H); 7,35 (1H); 7,95 (2H); 8,50 (2H); 9,36 (1H); 12,27 (1H) ppm.

### Bsp. 36 Herstellung von 3-(3-Amino-6-tert-butyl-1H-pyrazolo[3,4-b]pyridin-4-yl)-5-methoxy-phenol

### Bsp. 36a) Herstellung von 6-tert-Butyl-4-(3-hydroxy-5-methoxy -phenyl)-2-oxo-1,2-dihydro-pyridin-3-carbonitril

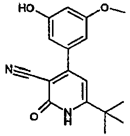

Analog zu Beispiel 1a werden aus 820 mg Ammoniumacetat, 140 µl Cyanessigsäureethylester, 165 µl 3,3-Dimethyl-2-butanon und 202 mg der unter Beispiel 33c beschriebenen Verbindung 164 mg Produkt erhalten.
¹H-NMR (d6-DMSO): δ = 1,29 (9H); 3,77 (3H); 6,19 (3H); 6,49 (1H); 6,60 (2H); 9,86 (1H); 12,25 (1H) ppm.

### Bsp. 36b) Herstellung von Essigsäure-3-(6-tert-butyl-3-cyano -2-oxo-1,2-dihydro-pyridin-4-yl)-5-methoxy phenyl ester

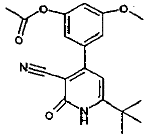

Analog zu Beispiel 32b werden aus 150 mg der unter Beispiel 36a beschriebenen Verbindung und 130 µl Essigsäureanhydrid in Pyridin 122 mg Produkt erhalten.
¹H-NMR (CDCl₃): δ = 1,42 (9H); 2,31 (3H); 3,88 (3H); 6,29 (1 H); 6,80 (1 H); 6,91 (1 H); 7,02 (1 H); 12,12 (1 H) ppm.

### Bsp. 36c) Herstellung von Essigsäure 3-(6-tert-butyl-3-cyano -2-trifluoromethanesulfonyloxy-pyridin-4-yl)-5-methoxy-phenyl ester

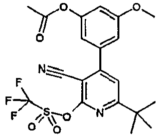

Analog zu Beispiel 3b werden aus 120 mg der unter Beispiel 36b beschriebenen Substanz und 175 µl Trifluormethansulfonsäure in Pyridin 133 mg Produkt erhalten.
¹H-NMR (CDCl₃): δ = 1,39 (9H); 2,35 (3H); 3,88 (3H); 6,84 (1H); 6,90 (1H); 7,01 (1H); 7,46 (1H) ppm.

### Bsp. 36d) Herstellung von 3-(3-Amino-6-tert-butyl-1H-pyrazolo[3,4-b]pyridin-4-yl)-5-methoxyphenol

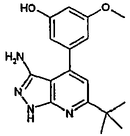

Analog zu Beispiel 1c werden aus 130 mg der unter Beispiel 36c beschriebenen Substanz mit 55 µl Hydrazinhydrat-Lösung (80%ig) in Propanol 49 mg Produkt erhalten.
¹H-NMR (d6-DMSO): δ = 1,38 (9H); 3,77 (3H); 4,57 (2H); 6,46 (1H); 6,53 (2H); 6,95 (1H); 9,79 (1H); 12,12 (1H) ppm.

### Bsp. 37 Herstellung von 3-(3-Amino-6-tert-butyl-1H-pyrazolo[3,4-b]pyridin-4-yl)-phenol

### Bsp. 37a)Herstellung von 6-tert-Butyl-4-(3-hydroxyphenyl)-2 -oxo-1,2-dihydro-pyridine-3-carbonitril

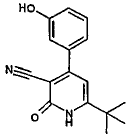

Analog zu Beispiel 1a werden aus 5 g Ammoniumacetat, 875 µl Cyanessigsäureethylester, 1,01 ml 3,3-Dimethyl-2-butanon und 1 g 3-Hydroxybenzaldehyd 814 mg Produkt erhalten.
¹H-NMR (d6-DMSO): δ = 1,28 (9H); 6,20 (1H); 6,93 (1H); 6,98-7,08 (2H); 7,34 (1H); 9,84 (1H); 12,25 (1H) ppm.

### Bsp. 37b) Herstellung von Essigsäure 3-(6-tert-butyl-3-cyano -2-oxo-1,2-dihydro-pyridin-4-yl)-phenyl ester

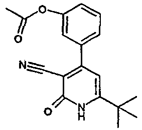

Analog zu Beispiel 32b werden aus 396 mg der unter Beispiel 37a beschriebenen Verbindung und 370 µl Essigsäureanhydrid in Pyridin 376 mg Produkt erhalten.
¹H-NMR (d6-DMSO): δ = 1,31 (9H); 2,29 (3H); 6,25 (1H); 7,34 (1H); 7,42 (1H); 7,52-7,65 (2H); 12,35 (1H) ppm.

### Bsp. 37c) Herstellung von Essigsäure 3-(6-tert-butyl-3-cyano -2-trifluoromethanesulfonyloxy-pyridin-4-yl)-phenyl ester

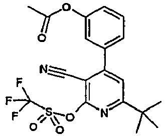

Analog zu Beispiel 3b werden aus 371 mg der unter Beispiel 37a beschriebenen Substanz und 605 µl Trifluormethansulfonsäure in Pyridin 480 mg Produkt erhalten.
¹H-NMR (CDCl₃): δ = 1,40 (9H); 2,35 (3H); 7,28-7,37 (2H); 7,45-7,53 (2H); 7,59 (1H) ppm.

### Bsp. 37d) Herstellung von 3-(3-Amino-6-tert-butyl-1H-pyrazolo[3,4-b]pyridin-4-yl)-phenol

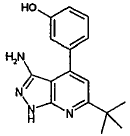

Analog zu Beispiel 1c werden aus 477 mg der unter Beispiel 37c beschriebenen Substanz mit 200 µl Hydrazinhydrat-Lösung (80%ig) in Propanol 147 mg Produkt erhalten.
¹H-NMR (d6-DMSO): δ = 1,38 (9H); 4,52 (2H); 6,37-7,00 (4H); 7,36 (1H); 9,75 (1H); 12,14 (1H) ppm.

### Bsp. 38 Herstellung von 3-(3-Amino-6-tert-butyl-1H-pyrazolo [3,4-b]pyridin-4-yl)-4-methyl-phenol

### Bsp. 38a) Herstellung von 6-tert-Butyl-4-[5-(tert-butyl-dimethyl-silanyloxy)-2-methyl-phenyl]-2-oxo-1,2-dihydro-pyridine-3-carbonitril

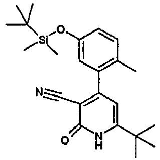

Analog zu Beispiel 1a werden aus 5 g Ammoniumacetat, 865 µl Cyanessigsäureethylester, 1 ml 3,3-Dimethyl-2-butanon und 2,03 g der unter Beispiel 35f beschriebenen Substanz 1,13 g Produkt erhalten.

### Bsp. 38b) Herstellung von Trifluoromethanesulfonsäure 6-tert-butyl-4-[5-(tert-butyl-dimethyl-silanyloxy)-2-methyl-phenyl]-3-cyano-pyridin-2-yl ester

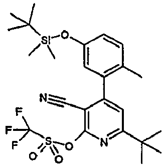

Analog zu Beispiel 3b werden aus 330 mg der unter Beispiel 38a beschriebenen Substanz und 200 µl Trifluormethansulfonsäure in Pyridin 352 mg Produkt erhalten.

### Bsp. 38c) Herstellung von 6-tert-Butyl-4-[5-(tert-butyl-dimethyl-silanyloxy)-2-methyl-phenyl]-1H-pyrazolo[3,4-b]pyridin-3-ylamin

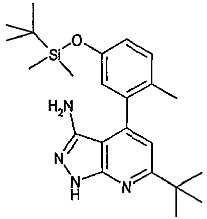

Analog zu Beispiel 1c werden aus 367 mg der unter Beispiel 38b beschriebenen Substanz mit 150 µl Hydrazinhydrat-Lösung (80%ig) in Propanol 189 mg Produkt erhalten.

### Bsp. 38d) Herstellung von 3-(3-Amino-6-tert-butyl-1H-pyrazolo [3,4-b]pyridin-4-yl)-4-methyl-phenol

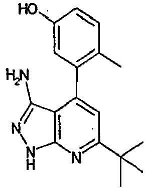

Analog zu Beispiel 35j werden aus 150 mg der unter Beispiel 38c beschriebenen Substanz 64 mg Produkt erhalten.
¹H-NMR (d6-DMSO): δ = 1,34 (9H); 1,96 (3H); 4,23 (2H); 6,62 (1H); 6,75-6,86 (2H); 7,17 (1H); 9,48 (1H); 12,08 (1H) ppm.

### Bsp. 39 Herstellung von 4-(3,5-Dimethoxy-phenyl)-6-pyridin-4-yl-1H-pyrazolo[3,4-b]pyridin-3-ylamin

### Bsp. 39a) Herstellung von 6-tert-Butyl-4-(3,5-dimethoxyphenyl)-2-oxo-1,2-dihydro-pyridine-3-carbonitril

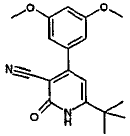

Analog zu Beispiel 1a werden aus 3,71 g Ammoniumacetat, 645 µl Cyanessigsäureethylester, 745 µl 3,3-Dimethyl-2-butanon und 1 g 3,5-Dimetoxybenzaldehyd 600 mg Produkt erhalten.
¹H-NMR (d6-DMSO): δ = 1,30 (9H); 3,80 (6H); 6,27 (1 H); 6,68 (1 H); 6,77 (2H) ppm.

### Bsp. 39b) Herstellung von Trifluormethanesulfonsäure-6-tert-butyl-3-cyano-4-(3,5-dimethoxyphenyl)pyridin-2-yl ester

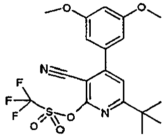

Analog zu Beispiel 3b werden aus 290 mg der unter Beispiel 39a beschriebenen Substanz und 470 µl Trifluormethansulfonsäure in Pyridin 382 mg Produkt erhalten.
¹H-NMR (CDCl₃): δ = 1,38 (9H); 3,86 (6H); 6,61 (1H); 6,69 (2H); 7,46 (1H) ppm.

### Bsp. 39c) Herstellung von 6-tert-Butyl-4-(3,5-dimethoxyphenyl)-1H-pyrazolo[3,4-b]pyridin-3-ylamin

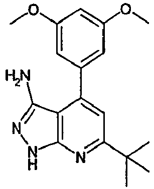

Analog zu Beispiel 1c werden aus 377 mg der unter Beispiel 39b beschriebenen Substanz mit 155 µl Hydrazinhydrat-Lösung (80%ig) in Propanol 126 mg Produkt erhalten.
¹H-NMR (d6-DMSO): δ = 1,35 (9H); 3,80 (6H); 4,53 (2H); 6,62 (1H); 6,70 (2H); 7,00 (1H); 12,15 (1H) ppm.

### Bsp. 40 Herstellung von 3-(3-Amino-6-tert-butyl-1H-pyrazolo3,4-b]pyridin-4-yl)-5-isopropoxyphenol

### Bsp. 40a) Herstellung von 2-(3-Isopropoxy-5-methoxy-phenyl)-[1,3]dioxolan

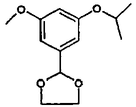

Zu einer Lösung von 2,85 g der unter Beispiel 33b beschriebenen Verbindung, 5,7 g Triphenylphosphin und 1,7 ml 2-Propanol in 20 ml Tetrahydrofuran werden bei 0°C langsam 3,42 ml Azodicarbonsäurediethylester getropft. Man lässt 20 Minuten bei 23°C nachrühren und gießt dann das Reaktionsgemisch auf 10 ml Hexan. Anschließend wird 15 Minuten nachgerührt und dann über Celite filtriert. Das Rohprodukt wird durch Säulenchromatographie gereinigt. Man erhält 2,94 g Produkt.
¹H-NMR (CDCl₃): δ = 1,30 (6H); 3,78 (3H); 4,00-4,15 (4H); 4,53 (1H); 5,76 (1 H); 6,42 (1H); 6,62 (2H) ppm.

### Bsp. 40b) Herstellung von 3-[1,3jDioxolan-2-yl-5-isopropoxyphenol

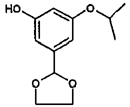

Analog zu Beispiel 33b werden aus 2,94 g der unter 40a beschriebenen Verbindung und 3,46 g Natriummethanthiolat in N,N-Dimethylformamid nach Säulenchromatographie 1,91 g Produkt erhalten.
¹H-NMR (CDCl₃): δ = 1,30 (6H); 3,98-4,15 (4H); 4,51 (1H); 5,20 (1H); 6,37 (1H); 6,51 (1H); 6,60 (1H) ppm.

### Bsp. 40c) Herstellung von 3-Hydroxy-5-isopropoxybenzaldehyd

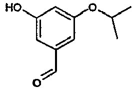

Analog zu Beispiel 33c werden aus 2,08 g der unter 40b beschriebenen Substanz mit 2 normaler Salzsäure in Aceton 1,46 g Produkt erhalten.
¹H-NMR (CDCl₃): δ = 1,35 (6H); 4,58 (1H); 5,61 (1H); 6,66 (1H); 6,91 (1H); 6,98(1H) 9,87 (1H) ppm.

### Bsp. 40d) Herstellung von 6-tert-Butyl-4-(3-hydroxy-5-isopropoxy-phenyl)-2-oxo-1,2-dihydro-pyridin-3-carbonitril

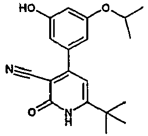

1,46 g der unter Beispiel 40c beschriebenen Verbindung, 5 g Ammoniumacetat, 865 µl Cyanessigsäureethylester und 1 ml 3,3-Dimethyl-2-butanon 1,12 g Produkt erhalten.
¹H-NMR (d6-DMSO): δ = 1,20-1,40 (15H); 4,60 (1H); 6,20 (1H); 6,47 (1H); 6,57 (2H); 9,82 (1H) ppm.

### Bsp. 40e) Herstellung von Essigsäure 3-(6-tert-butyl-3-cyano-2-oxo-1,2-dihydro-pyridin-4-yl)-5-isopropoxyphenyl ester

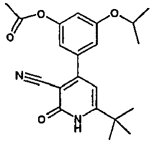

Analog zu Beispiel 32b werden aus 460 mg der unter Beispiel 40d beschriebenen Verbindung und 265 µl Essigsäureanhydrid in Pyridin 407 mg Produkt erhalten.
¹H-NMR (CDCl₃): δ= 1,36 (6H); 1,42 (9H); 2,31 (3H); 4,58 (1H); 6,30 (1H); 6,77 (1H); 6,89 (1H); 7,00 (1 H); 12,26 (1 H) ppm.

### Bsp. 40f) Herstellung von Essigsäure 3-(6-tert-butyl-3-cyano-2-trifluoromethan-sulfonyloxypyridin-4-yl)-5-isopropoxyphenyl ester

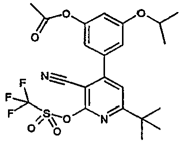

Analog zu Beispiel 3b werden aus 404 mg der unter Beispiel 40e beschriebenen Substanz und 555 µl Trifluormethansulfonsäure in Pyridin 490 mg Produkt erhalten.
¹H-NMR (CDCl₃): δ = 1,32-1,42 (15H); 2,32 (3H); 4,58 (1H); 6,80 (1 H); 6,88 (1H); 6,96 (1H); 7,46 (1H) ppm.

### Bsp. 40g) Herstellung von 3-(3-Amino-6-tert-butyl-1H-pyrazolo3,4-b]pyridin-4-yl)-5-isopropoxy-phenol

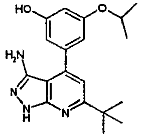

Analog zu Beispiel 1 c werden aus 486 mg der unter Beispiel 40f beschriebenen Substanz mit 180 µl Hydrazinhydrat-Lösung (80%ig) in Propanol 167 mg Produkt erhalten.
¹H-NMR (d6-DMSO): δ = 1,28 (6H); 1,37 (9H); 4,54-4,68 (3H); 6,42 (1H); 6,50 (2H); 6,95 (1H); 9,73 (1 H); 12,12 (1 H) ppm.

### Bsp. 41 Herstellung von 3-(3-Amino-6-tert-butyl-1H-pyrazolo [3,4b]pyridin-4-yl)-4-nitro-phenol

### Bsp. 41a) Herstellung von 6-tert-Butyl-4-(5-hydroxy-2-nitro-phenyl)-2-oxo-1,2-dihydro-pyridin-3-carbonitril

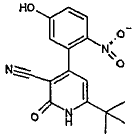

Analog zu Beispiel 1a werden aus 3,7 g Ammoniumacetat, 640 µl Cyanessigsäureethylester, 740 µl 3,3-Dimethyl-2-butanon und 1 g 5-Hydroxy-2-nitrobenzaldehyd 690 mg Produkt erhalten.
¹H-NMR (d6-DMSO): δ = 1,29 (9H); 6,25 (1 H); 6,81 (1 H); 7,06 (1 H); 8,21 (1H); 12,37 (1 H) ppm.

### Bsp. 41 b) Herstellung von Essigsäure-3-(6-tert-butyl-3-cyano-2-oxo-1,2-dihydropyridin-4-yl)-4-nitrophenyl ester

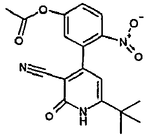

Analog zu Beispiel 33b werden aus 200 mg der unter Beispiel 41a beschriebenen Verbindung und 120 µl Essigsäureanhydrid in Pyridin 143 mg Produkt erhalten.
¹H-NMR (CDCl₃): δ = 1,35 (3H); 2,35 (3H); 6,10 (1H); 7,22 (1H); 7,42 (1 H); 8,29 (1 H) ppm.

### Bsp. 41c) Herstellung von Essigsäure 3-(6-tert-butyl-3-cyano-2-trifluormethan-sulfonyloxy-pyridin-4-yl)-4-nitro-phenyl ester

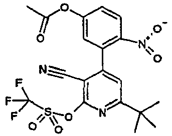

Analog zu Beispiel 3b werden aus 143 mg der unter Beispiel 41 b beschriebenen Substanz und 205 µl Trifluormethansulfonsäure in Pyridin 105 mg Produkt erhalten.
¹H-NMR (CDCl₃): δ = 1,38 (9H); 1,55 (3H); 7,32 (1H); 7,39 (1H); 7,68 (1H); 8,43 (1H) ppm.

### Bsp. 41 d) Herstellung von 3-(3-Amino-6-tert-butyl-1H-pyrazolo [3,4b]pyridin-4-yl)-4-nitro-phenol

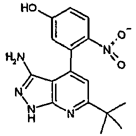

Analog zu Beispiel 1c werden aus 477 mg der unter Beispiel 41 beschriebenen Substanz mit 200 µl Hydrazinhydrat-Lösung (80%ig) in Propanol 147 mg Produkt erhalten.
¹H-NMR (d6-DMSO): δ = 1,35 (9H); 4,30 (2H); 6,72 (1H); 6,88 (1H); 7,00 (1H); 8,16 (1H); 12,10 (1H) ppm.

### Bsp. 42 Herstellung von 5-(3-Amino-6-tert-butyl-1H-pyrazolo[3,4-b]pyridin-4-yl)-2,3-dimethoxyphenol

### Bsp. 42a) Herstellung von 6-tert-Butyl-4-(3-hydroxy-4,5-dimethoxy-phenyl)-2-oxo-1,2-dihydro-pyridin-3-carbonitril

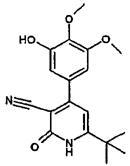

Analog zu Beispiel 1a werden aus 1,7 g Ammoniumacetat, 300 µl Cyanessigsäureethylester, 340 µl 3,3-Dimethyl-2-butanon und 500 mg 3,4-Dimethoxy-5-hydroxybenzaldehyd 340 mg Produkt erhalten.
¹H-NMR (d6-DMSO): δ = 1,30 (9H); 3,72 (3H); 3,82 (3H); 6,22 (1H); 6,76 (2H); 9,62 (1H); 12,21 (1H) ppm.

### Bsp. 42b) Herstellung von Essigsäure 5-(6-tert-butyl-3-cyano-2-oxo-1,2-dihydro-pyridin-4-yl)-2,3-dimethoxyphenyl ester

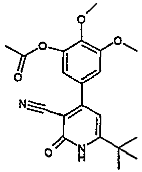

Analog zu Beispiel 33b werden aus 160 mg der unter Beispiel 42a beschriebenen Verbindung und 100 µl Essigsäureanhydrid in Pyridin 180 mg Produkt erhalten.
¹H-NMR (CDCl₃): δ = 1,42 (9H); 2,36 (3H); 3,91 (3H); 3,95 (3H); 6,29 (1H); 6,90 (1H); 7,18 (1H) ppm.

### Bsp. 42c) Herstellung von Essigsäure 5-(6-tert-butyl-3-cyano-2-trifluor-methanesulfonyloxypyridin-4-yl)-2,3-dimethoxyphenyl ester

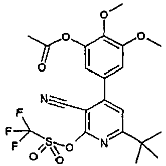

Analog zu Beispiel 3b werden aus 180 mg der unter Beispiel 42b beschriebenen Substanz und 250 µl Trifluormethansulfonsäure in Pyridin 215 mg Produkt erhalten.
¹H-NMR (CDCl₃): δ = 1,40 (9H); 2,36 (3H); 3,92 (3H); 3,97 (3H); 6,90 (1H); 7,11 (1H); 7,43 (1H) ppm.

### Bsp. 42d) Herstellung von 5-(3-Amino-6-tert-butyl-1H-pyrazolo[3,4b]pyridin-4-yl)-2,3-dimethoxyphenol

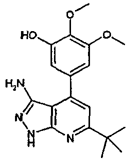

Analog zu Beispiel 1c werden aus 210 mg der unter Beispiel 42c beschriebenen Substanz mit 80 µl Hydrazinhydrat-Lösung (80%ig) in Propanol 90 mg Produkt erhalten.
¹H-NMR (d6-DMSO): δ = 1,38 (9H); 3,73 (3H); 3,82 (3H); 4,59 (2H); 6,68 (2H); 6,96 (1H); 9,51 (1 H); 12,12 (1 H) ppm.

### Bsp. 43: Herstellung von 6-Cyclopropyl-4-(3,4-dichlorphenyl)-1H-pyrazolo-[3,4-b]pyridin-3-ylamin

### Bsp. 43a) Herstellung von 6-Cyclopropyl-4-(3,4-dichlorphenyl)-2-oxo-1,2-dihydropyridin-3-carbonitril

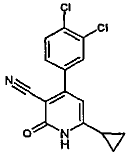

Unter Rühren versetzt man 300 ml Ethanol mit 14 g Acetylcyclopropan, 18 g Cyanessigsäureethylester, 28 g 3,4-Dichlorbenzaldehyd sowie 100 g Ammoniumacetat und erhitzt in der Folge sechs Stunden bei 80-90 °C Badtemperatur zum Sieden. Aus der Reaktionslösung fällt nach einiger Zeit bereits ein Teil des Produktes aus. Zur Vervollständigung der Ausfällung lässt man dann unter Rühren auf Raumtemperatur abkühlen. Der Niederschlag wird abgesaugt, mit kaltem Ethanol und Wasser acetatfrei gewaschen und abschließend getrocknet. Es fallen 15 g Produkt an, Schmelzpunkt 290-295 °C, unter Zersetzung.

### Bsp. 43b) Herstellung von 2-Chlor-6-cyclopropyl-4-(3,4-dichlorphenyl)-nicotinonitril

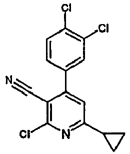

In 75 ml Phenylphosphonsäuredichlorid trägt man 16 g des Pyridons (obiges Beispiel) vorsichtig ein und rührt das Gemisch anschließend vier Stunden unter Feuchtigkeitsausschluss bei 150-160 °C. Das Pyridon geht unter diesen Bedingungen alsbald in Lösung. Zur Aufarbeitung lässt man auf Raumtemperatur abkühlen und rührt dann vorsichtig in Wasser ein. Der entstandene Niederschlag wird abgesaugt, mit Wasser gewaschen und an der Luft getrocknet. Ausbeute 11 g, Schmelzpunkt 158-160 °C (Propanol).

### Bsp. 43c) Herstellung von 6-Cyclopropyl-4-(3,4-dichlorophenyl)-1H-pyrazolo[ 3,4-b]pyridin-3-ylamin

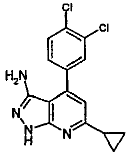

Man erhitzt eine eine Lösung von 0,971 g des vorstehenden Chlorcyanpyridins in 10 ml Propanol und 1 ml Hydrazinhydrat (80%ig) vier Stunden bei 100°C. Nach Abkühlen des Reaktiongemisches saugt man das ausgefallene Produkt ab und kristallisiert aus Essigsäure um. Ausbeute 250 mg, Schmelzpunkt 256-258°C.

### Bsp. 44 Herstellung von 3-(3-Amino-6-cyclopropyl-1H-pyrazolo-[3,4-b]pyridin-4-yl)-phenol

### Bsp. 44a) Herstellung von 6-Cyclopropyl-4-(3-hydroxy-phenyl)-2-oxo-1,2-dihydropyridin-3-carbonitril

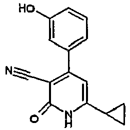

Analog zu Beispiel 1a werden aus 5 g Ammoniumacetat, 875 µl Cyanessigsäureethylester, 815 µl Cyclopropylmethylketon und 1 g 3-Hydroxybenzaldehyd 750 mg Produkt erhalten.
¹H-NMR (d6-DMSO): δ = 1,00-1.25 (4H); 1,97 (1H); 5,99 (1H); 6,86-7,05 (3H); 7,30 (1H); 9,80 (1 H); 12,58 (1 H) ppm.

### Bsp. 44b) Herstellung von Essigsäure 3-(3-cyano-6-cyclopropyl-2-oxo-1,2-dihydro-pyridin-4-yl)-phenyl ester

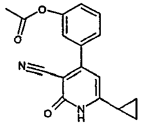

Analog zu Beispiel 33b werden aus 750 mg der unter Beispiel 44a beschriebenen Verbindung und 5500 µl Essigsäureanhydrid in Pyridin 570 mg Produkt erhalten.
¹H-NMR (CDCl₃): δ = 1,12 (2H); 1,31 (2H); 2,07 (1H); 2,33 (3H); 5,96 (1H); 7,20-7,33 (2H); 7,50 (2H); 13,46(1H) ppm.

### Bsp. 44c) Herstellung von Essigsäure 3-(3-cyano-6-cyclopropyl-2-trifluoromethan-sulfonyloxypyridin-4-yl)-phenyl ester

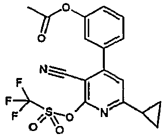

Analog zu Beispiel 3b werden aus 350 mg der unter Beispiel 44b beschriebenen Substanz und 600 µl Trifluormethansulfonsäure in Pyridin 458 mg Produkt erhalten.
¹H-NMR (CDCl₃): δ = 1,22 (4H); 2,10 (1H); 2,33 (3H); 7,27-7,38 (2H); 7,39 (1H); 7,49 (1H); 7,56 (1H) ppm.

### Bsp. 44d) Herstellung von 3-(3-Amino-6-cyclopropyl-1H-pyrazolo-[3,4-b]pyridin-4-yl)-phenol

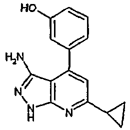

Analog zu Beispiel 1c werden aus 453 mg der unter Beispiel 44c beschriebenen Substanz mit 200 µl Hydrazinhydrat-Lösung (80%ig) in Propanol 208 mg Produkt erhalten.
¹H-NMR (d6-DMSO): δ = 0.93-1,14 (4H); 2,19 (1H); 4,49 (2H); 6,83 (1H); 6,88-7,00 (3H); 7,34 (1H); 9,78 (1H); 12,01 (1 H) ppm.

### Bsp. 45 Herstellung von 3-(3-Amino-6-cyclohexyl-1H-pyrazolo-[3,4-b]pyridin-4-yl)-phenol

### Bsp. 45a) Herstellung von 6-Cyclohexyl-4-(3-hydroxy-phenyl)-2-oxo-1,2-dihydro-pyridine-3-carbonitril

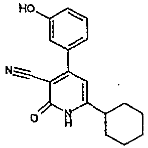

Analog zu Beispiel 1a werden aus 5 g Ammoniumacetat, 875 µl Cyanessigsäureethylester, 1,13 ml 3,3-Dimethyl-2-butanon und 1 g 3-hydroxybenzaldehyd 750 mg Produkt erhalten.
¹H-NMR (d6-DMSO): δ = 1,10-1,40 (4H); 1,40-1,60 (2H); 1,62-1,91 (4H); 2,53 (1H); 6,23 (1H); 6,88-7,06 (3H); 7,32 (1H); 9,81 (1H); 12,49 (1H) ppm.

### Bsp. 45b) Herstellung von Essigsäure 3-(3-cyano-6-cyclohexyl-2-oxo-1,2-dihydro-pyridin-4-yl)-phenyl ester

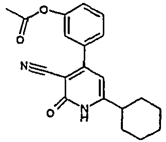

Analog zu Beispiel 33b werden aus 743 mg der unter Beispiel 45a beschriebenen Verbindung und 470 µl Essigsäureanhydrid in Pyridin 720 mg Produkt erhalten.
¹H-NMR (d6-DMSO): δ = 1,10-1,38 (4H); 1,40-1,60 (2H); 1,60-1,90 (4H); 2,30 (3H); 2,53 (1H); 6,29 (1 H); 7,32 (1 H); 7,40 (1 H); 7,50-7,65 (2H); 12,56 (1 H) ppm.

### Bsp. 45c) Herstellung von Essigsäure 3-(3-cyano-6-cyclohexyl-2-trifluoromethan-sulfonyloxy-pyridin-4-yl)phenyl ester

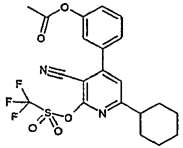

Analog zu Beispiel 3b werden aus 365 mg der unter Beispiel 45b beschriebenen Substanz und 550 µl Trifluormethansulfonsäure, in Pyridin 385 mg Produkt erhalten.
¹H-NMR (CDCl₃): δ = 1,20-1,62 (5H); 1,77 (1H); 1,82-2,02 (4H); 2,33 (3H); 2,78 (1H); 7,23-7,37 (3H); 7,49 (1 H); 7,57 (1 H) ppm.

### Bsp. 45d) Herstellung von 3-(3-Amino-6-cyclohexyl-1H-pyrazolo-[3,4-b]pyridin-4-yl)-phenol

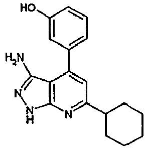

Analog zu Beispiel 1c werden aus 380 mg der unter Beispiel 45c beschriebenen Substanz mit 150 µl Hydrazinhydrat-Lösung (80%ig) in Propanol 165 mg Produkt erhalten.
¹H-NMR (d6-DMSO): δ = 1,15-1,50 (4H); 1,50-1,97 (6H); 2,75 (1H); 4,51 (2H); 6,78 (1H); 6,85-7,00 (3H); 7,33 (1 H); 9,75 (1 H); 12,10 (1 H) ppm.

### Bsp. 46 Herstellung von 5-(3-Amino-6-tert-butyl-1H-pyrazolo[3,4-b]pyridin-4-yl)-2-methoxyphenol

### Bsp. 46a) Herstellung von 6-tert-Butyl-4-(3-hydroxy-4-methoxyphenyl)-2-oxo-1,2-dihydro-pyridin-3-carbonitril

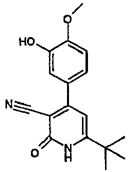

Analog zu Beispiel 1a werden aus 4,1 g Ammoniumacetat, 700 µl Cyanessigsäureethylester, 815 µl 3,3-Dimethyl-2-butanon und 1 g 3-Hydroxy-4-methoxybenzaldehyd 660 mg Produkt erhalten.
¹H-NMR (d6-DMSO): δ = 1,28 (9H); 3,84 (3H); 6,19 (1H); 7,08 (3H); 9,41 (1H); 12,13 (1 H) ppm.

### 46b) Herstellung von Essigsäure 5-(6-tert-butyl-3-cyano-2-oxo-1,2-dihydro-pyridin-4-yl)-2-methoxyphenyl ester

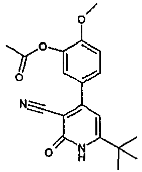

Analog zu Beispiel 33b werden aus 435 mg der unter Beispiel 46a beschriebenen Verbindung und 270 µl Essigsäureanhydrid in Pyridin 413 mg Produkt erhalten.
¹H-NMR (CDCl₃): δ = 1,42 (9H); 2,34 (3H); 3,91 (3H); 6,29 (1H); 7,10 (1H); 7,31 (1H); 7,60 (1H) 12,18 (1 H) ppm.

### Bsp. 46c) Herstellung von Essigsäure 5-(6-tert-butyl-3-cyano-2-trifluoromethan-sulfonyloxy-pyridin-4-yl)- 2-methoxy-phenyl ester

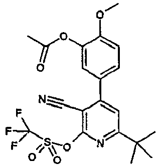

Analog zu Beispiel 3b werden aus 408 mg der unter Beispiel 46b beschriebenen Substanz und 605 µl Trifluormethansulfonsäure in Pyridin 501 mg Produkt erhalten.
¹H-NMR (CDCl₃): δ = 1,38 (9H); 2,35 (3H); 3,91 (3H); 7,12 (1H); 7,31 (1H); 7,42 (1H); 7,56 (1H) ppm.

### Bsp. 46d) Herstellung von 5-(3-Amino-6-tert-butyl-1H-pyrazolo[3,4-b]pyridin-4-yl)-2-methoxyphenol

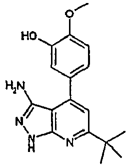

Analog zu Beispiel 1c werden aus 496 mg der unter Beispiel 46c beschriebenen Substanz mit 190 µl Hydrazinhydrat-Lösung (80%ig) in Propanol 145 mg Produkt erhalten.
¹H-NMR (d6-DMSO): δ = 1,35 (9H); 3,83 (3H); 4,56 (2H); 6,90 (1H); 6,99 (2H); 7,10 (1H); 9,31 (1H); 12,10 (1H) ppm.

### Bsp. 47 Herstellung von 5-(3-Amino-6-tert-butyl-1H-pyrazolo[3,4-b]pyridin-4-yl)-benzol-1,3-diol

### Bsp. 47a) Herstellung 6-tert-Butyl-4-(3,5-dihydroxyphenyl)-2-oxo-1,2-dihydropyridine-3-carbonitril

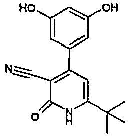

Analog zu Beispiel 1a werden aus 6,46 g Ammoniumacetat, 770 µl Cyanessigsäureethylester, 895 µl 3,3-Dimethyl-2-butanon und 1 g 3,5-Dihydroxybenzaldehyd 850 mg Produkt erhalten. ¹H-NMR (d6-DMSO): δ = 1,30 (9H); 6,15 (2H); 6,36 (1H); 6,43 (1H); 9,67 (2H); 12,23 (1H) ppm.

### Bsp. 47b) Herstellung von Essigsäure 3-acetoxy-5-(6-tert-butyl-3-cyano-2-oxo-1,2-dihydro-pyridin-4-yl)-phenyl ester

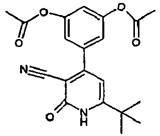

Analog zu Beispiel 33b werden aus 200 mg der unter Beispiel 47a beschriebenen Verbindung und 350 µl Essigsäureanhydrid in Pyridin 210 mg Produkt erhalten.
¹H-NMR (CDCl₃): δ = 1,40 (9H); 2,31 (6H); 6,38 (1H); 7,10 (1H); 7,28 (2H); 12,07 (1H) ppm.

### Bsp. 47c) Herstellung von Essigsäure 3-acetoxy-5-(6-tert-butyl-3-cyano-2-trifluoromethanesulfonyloxy-pyridin-4-yl)-phenyl ester

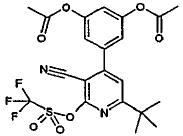

Analog zu Beispiel 3b werden aus 110 mg der unter Beispiel 47b beschriebenen Substanz und 150 µl Trifluormethansulfonsäure in Pyridin 67 mg Produkt erhalten.
¹H-NMR (CDCl₃): δ = 1,39 (9H); 2,32 (6H); 7,16 (1H); 7,26 (2H); 7,47 (1H) ppm.

### Bsp. 47d) Herstellung von 5-(3-Amino-6-tert-butyl-1H-pyrazolo[3,4-b]pyridin-4-yl)-benzol-1,3-diol

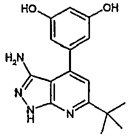

Analog zu Beispiel 1c werden aus 60 mg der unter Beispiel 47c beschriebenen Substanz mit 22 µl Hydrazinhydrat-Lösung (80%ig) in Propanol 25 mg Produkt erhalten.
¹H-NMR (d6-DMSO): δ = 1,37 (9H); 4,60 (2H); 6,35 (3H); 6,90 (1H); 9,59 (2H); 12,1 (1H) ppm.

### Bsp. 48: Herstellung von 4-Isopropyl-6-(3,4-methylendioxyphenyl)-1H-pyrazolo[3,4b]pyridin-3-ylamin

### Bsp. 48a) Herstellung von 4-Isopropyl-6-(3,4-methylendioxyphenyl)-2-oxo-1,2-dihydropyridin-3-carbonitril

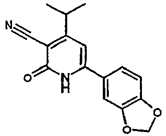

Zu einer Lösung von 12 g Isobutyraldehyd, 26 g 3,4-Methylendioxy-acetophenon und 18 g Cyanessigsäureethylester in 300 ml Ethanol gibt man 100 g Ammoniumacetat und erhitzt sodann sechs Stunden unter Rühren am Rückfluss (80-90°C). Nach dem Abkühlen wird der Niederschlag abgesaugt, zunächst mit kaltem Ethanol, dann mit Wasser gewaschen und an der Luft getrocknet. Ausbeute 17 g, Schmelzpunkt 266-268°C.

### Bsp. 48b) Herstellung von 2-Chlor-4-isopropyl-6-(3,4-methylendioxyphenyl)-nicotinonitril

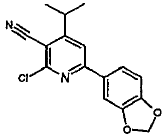

Man erhitzt eine Mischung von 15 g 2-Pyridon (vorstehendes Beispiel) und 75 ml Phenylphosphonsäuredichlorid vier Stunden lang in einer Stickstoff-atmosphäre unter Feuchtigkeitsausschluss auf 150-160 °C. Nach Abkühlen wird das Reaktionsgemisch vorsichtig in Eiswasser eingerührt, der Niederschlag wird abgesaugt, sorgfältig mit Wasser gewaschen und an der Luft getrocknet. Ausbeute 16 g, Schmelzpunkt 171-173 °C.

### Bsp. 48c) Herstellung von 4-Isopropyl-6-(3,4-methylendioxyphenyl)-1H-pyrazolo[3,4-b]pyridin-3-ylamin

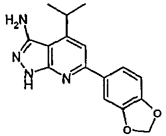

300 mg Chlornicotinonitril (vorstehendes Beispiel) versetzt man in 3 ml Propanol mit 1ml Hydrazinhydrat und erhitzt vier Stunden unter Rühren bei 100 °C. Nach der Hälfte der Reaktionszeit gibt man eine zweite Portion Hydrazinhydrat ( 0,5 ml) zur Reaktionslösung. Zur Aufarbeitung wird das Produkt durch Zugabe von Wasser gefällt. Der Niederschlag wird abgesaugt, mit Wasser, kaltem Isopropanol und Diethylether gewaschen und an der Luft getrocknet. Ausbeute 200 mg, Schmelzpunkt 184-186 °C.

### Bsp. 49: Herstellung von 6-(3-Hydroxyphenyl)-4-(4-pyridyl)-1H-pyrazolo[3,4b]pyridin-3-ylamin

### Bsp. 49a) Herstellung von 6-(3-Hydroxyphenyl)-2-oxo-4-(4-pyridyl)-1,2-dihydropyridin-3-carbonitril

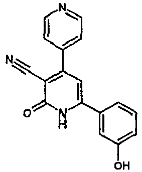

Nach dem allgemeinen Herstellungsprocedere für 2-Pyridone fallen aus einem Ansatz. mit 50 g Ammoniumacetat, 11 g 3-Hydroxyacetophenon, 9 g Cyan-essigsäureethylester und 8 g 4-Pyridinaldehyd in 300 ml Ethanol nach sechs Stunden Rektionszeit bei 80°C insgesamt 8 g der Titelverbindung an.

### Bsp. 49b) Herstellung von 6-(3-Acetyloxyphenyl)-2-oxo-4-(4-pyridyl)-1,2-dihydropyridin-3-carbonitril

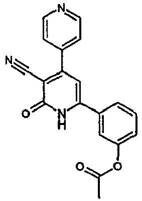

Man trägt 8 g Pyridon (vorstehendes Beispiel) in 80 ml Pyridin ein, versetzt unter Rühren zügig mit 6 ml Essigsäureanhydrid und hält das Reaktionsgemisch anschließend zwei Stunden bei 80 °C. Zur Aufarbeitung lässt man abkühlen und rührt in Eiswasser ein. Der Niederschlag wird abgesaugt, mit Wasser gewaschen und an der Luft getrocknet. Ausbeute 7 g, Schmelzpunkt 284-286 °C.

### Bsp. 49c) Herstellung von 6-(3-Acetyloxyphenyl)-4-(4-pyridyl)-2-trifluormethylsulfonyl-oxynicotinonitril

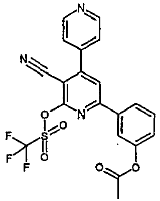

Zu einer Suspension von 1g des acetylierten Pyridons (vorstehendes Beispiel) in 10 ml Pyridin tropft man unter Feuchtigkeitsausschluss bei Eisbadkühlung 1 ml Trifluormethansulfonsäureanhydrid. Anschließend entfernt man das Eisbad und lässt zwei Stunden bei Raumtemperatur rühren. Zur Aufarbeitung wird die homogene, dunkelgefärbte Reaktionslösung in Eiswasser eingetragen, der Niederschlag abgesaugt, mit Wasser gewaschen und getrocknet. Das Rohprodukt (1g) kann aus Diethylether umkristallisiert werden. Ausbeute 720 mg.

### Bsp. 49d) Herstellung von 6-(3-Hydroxyphenyl)-4-(4-pyridyl)-1 H-pyrazolo[3,4-b]pyridin-3-ylamin

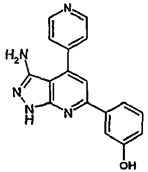

Eine Suspension von 980 mg Triflat (vorstehendes Beispiel) in 25 ml Propanol versetzt man 1ml Hydrazinhydrat (80 %ig) und rührt das Gemisch 6 Stunden bei 100°C Badtemperatur. Nach einiger Zeit wird die Reaktionslösung homogen. Zur Vervollständigung der Umsetzung gibt man nach drei Stunden weitere 0,4 ml Hydrazinhydrat Lösung zu. Das Produkt fällt beim Abkühlen der Reaktionslösung aus. Der Niederschlag wird abgesaugt, mit Propanol und Ether gewaschen und abschließend getrocknet. Ausbeute 420 mg.
¹H-NMR (DMSO-d₆) δ= 4,68(s, 2H); 6,86(dd, 1 H); 7,29(t, 1 H); 7,45(s, 1 H); 7,59(m, 2H); 7,70(d, 2H); 8,76(d, 2H); 9,55(brs, 1 H); 12,42(s, 1 H) ppm.

Analog zu den oben beschriebenen Beispielen werden folgende Verbindungen hergestellt:

### Bsp. 50: 4-(4-Chlorphenyl)-6-phenyl-1H-pyrazolo[3,4-b]pyridin-3-ylamin

### Bsp. 51: 6-Cyclopropyl-4-(3,4-dichlorphenyl)-1H-pyrazolo[3,4-b]pyridin-3-ylamin

### Bsp. 52: 5-[3-Amino-6-(4-fluorphenyl)-1H-pyrazolo[3,4-b]pyridin-4-yl]-2-methoxyphenol

### Bsp. 53: 6-Pyridin-3-yl-4-quinolin-3-yl-1H-pyrazolo[3,4-b]pyridin-3-ylamin

### Bsp. 54: 6-(3-Chlorphenyl)-4-(1H-indol-3-yl)-1H-pyrazolo[3,4-b]pyridin-3-ylamin

### Bsp. 55: 4-[3-Amino-6-(3-methoxyphenyl)-1H-pyrazolo[3,4-b]pyridin-4-yl]-benzonitril

### Bsp. 56: 4-(4-Chlorphenyl)-6-cyclopropyl-1H-pyrazolo[3,4-b]pyridin-3-ylamin

### Bsp. 57: 6-Cyclopropyl-4-(3-nitrophenyl)-1H-pyrazolo[3,4-b]pyridin-3-ylamin

### Bsp. 58: 6-(4-Morpholin-4-yl-phenyl)-4-pyridin-4-yl-1H-pyrazolo[3,4-b]pyridin-3-ylamin

### Bsp. 59: 4-[4-(3-Dimethylaminopropoxy)phenyl]-6-(4-fluorophenyl)-1H-pyrazolo[3,4-b]pyridin-3-ylamin

### Bsp. 60: 6-Cyclopropyl-4-(4-trifluoromethylphenyl)-1H-pyrazolo[3,4-b]pyridin-3-ylamin

### Bsp. 61: 4-(3-Amino-6-cyclopropyl-1H-pyrazolo[3,4-b]pyridin-4-yl)-benzonitril

### Bsp. 62: 4-(1H-Imidazol-2-yl)-6-(4-phenoxy-phenyl)-1H-pyrazolo[3,4-b]pyridin-3-ylamine

### Bsp. 63: Herstellung von 6-tert-Butyl-4-(4-nitro-phenyl)-1H-pyrazolo[3,4b]pyridin-3-ylamin

### Bsp. 63a) Herstellung von 6-tert-Butyl-4-(4-nitro-phenyl)-2-oxo-1,2-dihydro-pyridin-3-carbonitril

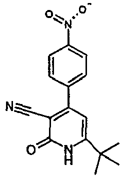

Analog zu Beispiel 1a) werden aus 10 g Ammoniumacetat, 1,8 ml Cyanessigsäureethylester, 2,1 ml 3,3-Dimethyl-2-butanon und 3 g 4-Nitrobenzaldehyd 1,55 g Produkt erhalten.
¹H-NMR (d6-DMSO): δ = 1,33 (9H); 6,30 (1H); 7,93 (2H); 8,38 (2H); 12,46 (1H) ppm.

### Bsp. 63b) Herstellung von 6-tert-Butyl-3-cyano-4-(4-nitro-phenyl)-2-trifluormethanesulfonylpyridin

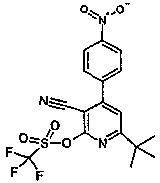

Analog zu Beispiel 3b) werden aus 1,55 g der unter Beispiel 63a) beschriebenen Substanz und 2,63 ml Trifluormethansulfonsäureanhydrid in Pyridin 1,85 g Produkt erhalten.
¹H-NMR (CDCl₃): δ = 1,41 (9H); 7,48 (1H); 7,78 (2H); 8,43 (2H) ppm.

### Bsp. 63c) Herstellung von 6-tert-Butyl-4-(4-nitro-phenyl)-1H-pyrazolo[3,4b]pyridin-3-ylamin

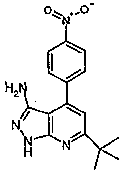

Analog zu Beispiel 1c) werden aus 1,85g der unter Beispiel 63b) beschriebenen Verbindung mit 800 µl Hydrazinhydrat-Lösung (80%ig) in Propanol 969 mg Produkt hergestellt.
¹H-NMR (d6-DMSO): δ = 1,39 (9H); 4,58 (2H); 7,87 (2H); 8,38 (2H); 12,30 (1H) ppm.

### Biologische Testung der Verbindungen

### Testsystem für EphB4

Eine Mischung aus 20 ng/ml rekombinanter EphB4-Kinase (bezogen von ProQinase GmbH, Freiburg, Deutschland), 2.67 µg/ml polyGluAlaTyr, 2 µM ATP, 25 mM HEPES (pH 7.3), 5 mM MgCl₂, 1 mM MnCl₂, 2 mM DTT, 0.1 mM NaVO₄, 1% (v/v) Glycerin, 0.02% NP40, EDTA-freie Protease Inhibitoren (Complete Fa. Roche, 1 Tablette in 50 ml) wird bei 20°C für 10 min inkubiert. Testsubstanzen werden in 100% DMSO gelöst und in 0.017 fachem Volumen vor dem Start der Reaktion vorgelegt. 60 Minuten nach Zugabe von 1.7 fachem Volumen einer Lösung 50mM Hepes pH 7.0, 0.2 % BSA, 0.14 µg/ml PT66-Europium, 3.84 µg/ml SA-XL665, 75 mM EDTA wird der Ansatz in einem Discovery HTRF-Messgerät der Firma PerkinElmer vermessen.

Unter anderen hemmen folgende Verbindungen die EphB4-Kinase mit einer IC₅₀, die kleiner als 10 µM ist: Beispiele 6, 12, 14, 16, 33, 35, 39, 40, 41, 51, etc. Die IC₅₀ der Verbindung 6 ist beispielsweise 6,1 µM.

Dies verdeutlicht, dass die erfindungsgemäßen Substanzen Proteintyrosinkinasen, insbesondere Eph-Rezeptoren und hier insbesondere EphB4 inhibieren.

## Patentansprüche

1. Verbindungen der allgemeinen Formel (I), 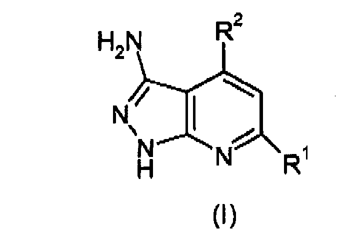 in der,
R¹ für gegebenenfalls ein- oder mehrfach, gleich oder verschieden mit K substituiertes C₁-C₆-Alkyl, C₂-C₆-Alkenyl, C₂-C₆-Alkinyl, C₃-C₁₀-Cycloalkyl, C₃-C₁₀-Heterocycloalkyl, Aryl oder Heteroaryl steht, wobei das C₃-C₁₀-Heterocycloalkyl und/oder das Heteroaryl selbst durch mindestens eins der folgenden Atome Stickstoff, Sauerstoff und/oder Schwefel im Ring unterbrochen ist und das C₃-C₁₀-Heterocycloalkyl und/oder Heteroaryl nur über ein Kohlenstoffringatom mit dem Pyrazolopyridin verknüpft sein kann, steht und C₃-C₁₀-Cycloalkyl und/oder C₃-C₁₀-Heterocycloalkyl gegebenenfalls durch ein oder mehrere, gleich oder verschledene -(CO)-, -SO- oder -SO₂- Gruppen im Ring unterbrochen sein kann und gegebenenfalls ein oder mehrere Doppelbindungen Im Ring enthalten sein können, oder für ein mit der Gruppe -O-(CH₂)ₙ-O-substitulertes C₃-C₁₀-Cycloalkyl, Aryl, C₃-C₁₀-Heterocycloalkyl oder Heteroaryl steht, wobei die endständigen Sauerstoffatome der -O-(CH₂)ₙ-O- Gruppe mit einem gleichen oder direkt benachbarten C₃-C₁₀-Cycloalkylring-, Arylring-, C₃-C₁₀-Heterocycloalkyring- oder Heteroarylring-Kohlenstoffatom verknüpft sind,
K für Halogen, Hydroxy oder die Gruppe -OR³, COR⁴ oder -NR⁵R⁶ steht oder für gegebenenfalls ein- oder mehrfach, gleich oder verschleden mit L substituiertes C₃-C₁₀-Cycloalkyl, C₃-C₁₀-Heterocycloalkyl, Aryl oder Heteroaryl steht, wobei das C₃-C₁₀-Heterocycloalkyl und/oder das Heteroaryl selbst durch mindestens eins der folgenden Atome Stickstoff, Sauerstoff und/oder Schwefel im Ring unterbrochen ist und C₃-C₁₀-Cycloalkyl und/oder C₃-C₁₀-Heterocycloalkyl gegebenenfalls durch ein oder mehrere, gleich oder verschiedene -(CO)-, -SO-oder -SO₂- Gruppen im Ring unterbrochen sein kann und gegebenenfalls ein oder mehrere Doppelbindungen im Ring enthalten sein können,
L für C₁-C₆-Alkyl oder die Gruppe -COR⁴, -OR³ oder -NR⁵R⁶ steht,
R² für gegebenenfalls ein- oder mehrfach, gleich oder verschieden mit M substituiertes C₁-C₆-Alkyl, C₃-C₁₀-Cycloalkyl, Aryl oder Heteroaryl steht,
R³ für gegebenenfalls ein- oder mehrfach, gleich oder verschieden mit der Gruppe-NR⁵R⁶ substituiertes C₁-C₆-Alkyl, Aryl oder -(CH₂)ₙ-Aryl steht,
R⁴ für Wasserstoff, Hydroxy, C₁-C₆-Alkyl oder C₁-C₆-Alkoxy steht,
M für Amino, Cyano, Halogen, Hydroxy, Nitro oder für gegebenenfalls ein- oder mehrfach, gleich oder verschieden mit Amino, Cyano, Halogen, Hydroxy, Nitro, C₁-C₆-Alkoxy substituiertes C₁-C₆-Alkyl, C₂-C₆-Alkenyl, C₂-C₆-Alkinyl steht oder für die Gruppe -O-R³, -COR⁴, oder -CO-N-R⁷ steht,
R⁵ und R⁶ unabhängig voneinander für Wasserstoff, C₁-C₆-Alkyl oder für die Gruppe-COR⁴ stehen und
R⁷ Wasserstoff oder NH₂ steht,
n für 1 bis 4 steht,
mit der Vorgabe, dass wenn
R¹ für Methyl steht, dann darf R² nicht gleichzeitig für -CH₂-O-CH₃, Chlorphenyl, mit Hydroxy und/oder Methoxy substituiertes Benzofuranyl, -CF₃ oder Furanyl stehen oder wenn
R¹ für -CH₂-O-CH₃ steht, dann darf R² nicht gleichzeitig für Methyl stehen oder wenn
R¹ für -CH=CH-Phenyl steht, dann darf R² nicht gleichzeitig für Phenyl stehen oder wenn
R¹ für -CH=CH-Chlorphenyl steht, dann darf R² nicht gleichzeitig für Phenyl oder Chlorphenyl stehen oder wenn
R¹ für -CH=CH-Methoxyphenyl steht, dann darf R² nicht gleichzeitig für Phenyl oder Methoxyphenyl stehen oder wenn
R¹ für Phenyl steht, dann darf R² nicht gleichzeitig für -CF₃, p-Methylphenyl oder Methoxyphenyl stehen oder wenn
R¹ für Methoxyphenyl steht, dann darf R² nicht gleichzeitig für -CF₃ stehen oder wenn
R¹ für Methylphenyl steht, dann darf R² nicht gleichzeitig -CF₃ stehen oder wenn
R¹ für Chlorphenyl steht, dann darf R² nicht gleichzeitig für Chlorphenyl oder -CF₃ stehen oder wenn
R¹ für Dichlorphenyl steht, dann darf R² nicht gleichzeitig für Trimethoxyphenyl stehen oder wenn
R¹ für Bromphenyl steht, dann darf R² nicht gleichzeitig nicht für Trimethoxyphenyl stehen oder wenn
R¹ für Alkyl, Alkenyl, Aryl, Aralkyl, Cycloalkyl, für ein mit Phenyl substitulertes Alkyl oder p-Methoxyphenyl steht, dann darf R² nicht gleichzeitig auch für Alkyl, Alkenyl, Aryl, Aralkyl, heterocyclischer Rest oder Cycloalkyl stehen oder wenn
R¹ für Hydroxyphenyl steht, dann darf R² nicht gleichzeitig für Heterocycyl oder mit-COO-*tert*-Butyl substitulertes Heterocycloalkyl stehen oder wenn
R¹ für Benzyloxyphenyl steht, dann darf R² nicht gleichzeitig für mit-COO-*tert*-Butyl substituiertes Heterocycloalkyl stehen, oder wenn
R¹ für p-Methylphenyl steht, dann darf R² nicht gleichzeitig für Phenyl stehen, oder wenn
R¹ für Cyclopropyl steht, dann darf R² nicht gleichzeitig für p-Methoxyphenyl stehen ;
und mit der Vorgabe, dass wenn :
R¹ für unsubstituiertes C₁-C₆-Alkyl, Aryl, C₃-C₁₀-Cycloalkyl steht, oder für einen mit K substitulertes C₁-C₆-Alkyl steht und K für Aryl steht, dann steht
R² für ein- oder mehrfach, gleich oder verschieden mit M substituiertes C₁-C₆-Alkyl, C₃-C₁₀-Cycloalkyl, Aryl oder Heteroaryl ;
oder wenn :
R² für unsubstituiertes C₁-C₆-Alkyl, C₃-C₁₀-Cycloalkyl oder Aryl steht, dann steht
R¹ für ein- oder mehrfach, gleich oder verschieden mit M substituiertes C₁-C₆-Alkyl, C₃-C₁₀-Cycloalkyl, Aryl oder Heteroaryl ;
bedeuten, sowie deren Solvate, Hydrate, Stereoisomere, Diastereomere, Enantiomere und Salze.

2. Verbindungen der allgemeinen Formel I, gemäß Anspruch 1, in der
R¹ für gegebenenfalls ein- oder mehrfach, gleich oder verschieden mit K substituiertes C₁-C₆ Alkyl, C₃-C₁₀-Cycloalkyl, wobei das C₃-C₁₀-Cycloalkyl gegebenenfalls durch ein oder mehrere -(CO)-, -SO- oder -SO₂-Gruppen im Ring unterbrochen ist und gegenenfalls ein oder mehrere Doppelbindungen im Ring enthalten sein können, steht, oder für gegebenenfalls ein- oder mehrfach, gleich oder verschieden mit K substituiertes Aryl, C₃-C₁₀-Heterocycloalkyl oder Heteroaryl steht, wobei das C₃-C₁₀-Heterocycloalkyl und/oder das Heteroaryl selbst durch mindestens ein Stickstoff, Sauerstoff und/oder Schwefel unterbrochen ist und das C₃-C₁₀-Heterocycloalkyl und/oder Heteroaryl nur über ein Kohlenstoffringatom mit dem Pyrazolopyridin verknüpft sein kann, steht, oder für ein mit der Gruppe -O-(CH₂)ₙ-O- substituiertes C₃-C₁₀-Cycloalkyl, Aryl, C₃-C₁₀-Heterocycloalkyl oder Heteroaryl steht, wobei die endständigen Sauerstoffatome der -O-(CH₂)ₙ-_{O}- Gruppe mit einem gleichen oder direkt benachbarten C₃-C₁₀-Cycloalkylring-, Arylring-, C₃-C₁₀-Heterocycloalkylring-, oder Heteroarylring-Kohlenstoffatom verknüpft sind,
K für Halogen, Hydroxy oder die Gruppe -OR³ oder -COR⁴ steht oder für gegebenenfalls ein- oder mehrfach, gleich oder verschieden mit L substituiertes C₃-C₁₀-Cycloalkyl, C₃-C₁₀-Heterocycloalkyl, Aryl oder Heteroaryl steht, wobei das C₃-C₁₀-Heterocycloalkyl und/oder das Heteroaryl selbst durch mindestens eins der folgenden Atome Stickstoff, Sauerstoff und/oder Schwefel im Ring unterbrochen ist und C₃-C₁₀-Cycloalkyl und/oder C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Rind enthalten sein können,
R² für gegebenenfalls ein- oder mehrfach, gleich oder verschieden mit M substitulertes C₁-C₆-Alkyl, C₃-C₁₀-Cycloalkyl, Aryl oder Heteroaryl steht, wobei das Heteroaryl durch mindestens einen Stickstoff, Sauerstoff und/oder Schwefel unterbrochen ist, steht,
R³ für C₁-C₆-Alkyl, Aryl oder -(CH₂)ₙ-Aryl steht,
M für Amino, Cyano, Halogen, Hydroxy, Nitro oder für gegebenenfalls ein- oder mehrfach, gleich oder verschieden mit Amino, Cyano, Halogen, Hydroxy, Nitro, C₁-C₆-Alkoxy substituiertes C₁-C₆-Alkyl steht oder für die Gruppe -O-C₁-C₆-Alkyl, -O-(CH₂)ₙ-NR⁵R⁶, -COR⁴, -O-Phenyl, -O-(CH₂)ₙ-Phenyl oder -CO-N-R⁷ steht,
bedeuten, sowie deren Solvate, Hydrate, Stereoisomere, Diastereomere, Enantiomere und Salze.

3. Verbindungen der allgemeinen Formel I, gemäß Anspruch 2, in der
R¹ für gegebenenfalls ein- oder mehrfach, gleich oder verschieden mit K substituiertes C₁-C₆ Alkyl, C₃-C₁₀-Cycloalkyl, wobei das C₃-C₁₀-Cycloalkyl gegebenenfalls durch ein oder mehrere -(CO)-, -SO- oder -SO₂-Gruppen im Ring unterbrochen ist und gegenenfalls ein oder mehrere Doppelbindungen im Ring enthalten sein können, steht, oder für gegebenenfalls ein- oder mehrfach, gleich oder verschieden mit K substituiertes Aryl oder Heteroaryl steht, wobei das Heteroaryl selbst durch mindestens ein Stickstoff, Sauerstoff und/oder Schwefel unterbrochen ist und das Heteroaryl nur über ein Kohlenstoffringatom mit dem Pyrazolopyridin verknüpft sein kann, steht, oder für ein mit der Gruppe -O-(CH₂)ₙ-O- substituiertes C₃-C₁₀-Cycloalkyl, Aryl oder Heteroaryl steht, wobei die endständigen Sauerstoffatome der -O-(CH₂)ₙ-O- Gruppe mit einem gleichen oder direkt benachbarten C₃-C₁₀-Cycloalkylring-, Arylring- oder Heteroarylring-Kohlenstoffatom verknüpft sind,
K für Halogen, Hydroxy oder die Gruppe -OR³ oder COR⁴ steht oder für gegebenenfalls ein- oder mehrfach, gleich oder verschieden mit L substituiertes C₃-C₁₀-Cycloalkyl,oder C₃-C₁₀-Heterocycloalkyl, wobei das C₃-C₁₀-Heterocycloalkyl selbst durch mindestens eins der folgenden Atome Stickstoff, Sauerstoff und/oder Schwefel im Ring unterbrochen ist und C₃-C₁₀-Cycloalkyl und/oder C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthalten sein können,
L für C₁-C₆-Alkyl oder die Gruppe -COR⁴ oder -OR³ steht,
R³ für C₁-C₆-Alkyl oder Aryl steht,
M für Amino, Cyano, Halogen, Hydroxy, Nitro oder für gegebenenfalls ein- oder mehrfach, gleich oder verschieden mit Amino, Cyano, Halogen, Hydroxy, Nitro, C₁-C₆-Alkoxy substituiertes C₁-C₆-Alkyl steht oder für die Gruppe -O-C₁-C₆-Alkyl, -O-(CH₂)ₙ-NR⁵R⁶-CO-C₁-C₆-Alkyl, -O-Phenyl, -O-(CH₂)ₙ-Phenyl oder -CO-N-R⁷ steht,
bedeuten, sowie deren Solvate, Hydrate, Stereoisomere, Diastereomere, Enantiomere und Salze.

4. Verbindungen der allgemeinen Formel I, gemäß Anspruch 3, in der
R¹ für gegebenenfalls ein- oder mehrfach, gleich oder verschieden mit K substituiertes C₁-C₆ Alkyl, C₃-C₁₀-Cycloalkyl, wobei das C₃-C₁₀-Cycloalkyl gegebenenfalls durch ein oder mehrere -(CO)-Gruppen im Ring unterbrochen ist, steht, oder für gegebenenfalls ein- oder mehrfach, gleich oder verschieden mit K substituiertes Aryl oder Heteroaryl steht, wobei das Heteroaryl seibst durch mindestens ein Stickstoff unterbrochen ist und das Heteroaryl nur über ein Kohlenstoffringatom mit dem Pyrazolopyridin verknüpft sein kann, steht, oder für ein mit der Gruppe -O-(CH₂)ₙ-O- substituiertes C₃-C₁₀-Cycloalkyl, Aryl oder Heteroaryl steht, wobei die endständigen Sauerstoffatome der -O-(CH₂)ₙ-O-Gruppe mit einem gleichen oder direkt benachbarten C₃-C₁₀-Cycloalkylring-, Arylring- oder Heteroarylring-Kohlenstoffatom verknüpft sind,
K für Halogen, Hydroxy oder für die Gruppe -OR³ steht, oder gegebenenfalls ein-oder mehrfach, gleich oder verschieden mit L substituiertes C₃-C₁₀-Heterocycloalkyl, wobei das C₃-C₁₀-Heterocycloalkyl selbst durch mindestens eins der folgenden Atome Stickstoff und/oder Sauerstoff im Ring unterbrochen ist, steht,
L für C₁-C₆-Alkyl oder -COO-C₁-C₆-Alkyl steht,
R² für gegebenenfalls ein- oder mehrfach, gleich oder verschieden mit M substituiertes C₁-C₆ Alkyl, C₃-C₆-Cycloalkyl, Aryl oder Heteroaryl, wobei das Heteroaryl durch mindestens einen Stickstoff unterbrochen ist, steht,
M für Amino, Cyano, Halogen, Hydroxy, Nitro oder für gegebenenfalls ein- oder mehrfach, gleich verschieden mit Amino, Cyano, Halogen, Hydroxy, Nitro oder C₁-C₆-Alkoxy substituiertes C₁-C₆-Alkyl steht oder für die Gruppe -O-C₁-C₆-Alkyl, -O-(CH₂)ₙ-N(C₁-C₆-Alkyl)₂, -CO-C₁-C₆-Alkyl, Phenoxy, Benzyloxy oder -CO-N-NH₂ steht und
bedeuten, sowie deren Solvate, Hydrate, Stereoisomere, Diastereomere, Enantiomere und Salze.

5. Verbindungen der allgemeinen Formel I, gemäß einem der Ansprüche 1 bis 4, in der
M für Amino, Cyano, Halogen, Hydroxy, Nitro oder für gegebenenfalls ein- oder mehrfach, gleich verschieden mit Amino, Cyano, Halogen, Hydroxy, Nitro oder C₁-C₆-Alkoxy substituiertes C₁-C₆-Alkyl steht oder für die Gruppe -O-C₁-C₆-Alkyl, -O-(CH₂)ₙ-N(C₁-C₆-Alkyl)₂ oder -CO-C₁-C₆-Alkyl steht und
bedeuten, sowie deren Solvate, Hydrate, Stereoisomere, Diastereomere, Enantiomere und Salze.

6. Verbindungen der allgemeinen Formel I, gemäß Anspruch 4, in der
R¹ für gegebenenfalls ein- oder mehrfach, gleich oder verschieden mit K substituiertes C₁-C₆-Alkyl, C₃-C₆-Cycloalkyl, Cyclohexanon, 1,4-Dioxaspiro[4.5]dec-8-yl, Phenyl, 1,3-Benzodioxolyl oder Pyridinyl steht,
K für Halogen, Hydroxy, C₁-C₆-Alkoxy oder gegebenenfalls ein- oder mehrfach, gleich oder verschieden mit L substituiertes Morpholinyl, Piperazinyl, Piperidinyl, oder Phenoxy steht,
R² für gegebenenfalls ein- oder mehrfach, gleich oder verschieden mit M substituiertes C₁-C₆-Alkyl, Phenyl oder Chinolinyl, Imidazolyl, Indolyl oder Pyridinyl steht,
M für Amino, Cyano, Halogen, Hydroxy, Nitro, C₁-C₆-Alkyl, -CF₃, oder für C₁-C₃-Alkoxy steht oder für die Gruppe -CO-C₁-C₆-Alkyl, -O-(CH₂)ₙ-N(C₁-C₆-Alkyl)₂, Phenoxy oder Benzyloxy,
bedeuten, sowie deren Solvate, Hydrate, Stereoisomere, Diastereomere, Enantiomere und Salze.

7. Verbindungen der allgemeinen Formel I, gemäß Anspruch 6, in der
R¹ für gegebenenfalls ein- oder mehrfach, gleich oder verschieden mit K substituiertes C₃-C₆Alkyl, Cyclopropyl, Cyclohexyl, Cyclohexanon, 1,4-Dioxaspiro[4.5]dec-8-yl, Phenyl, 1,3-Benzodioxolyl oder Pyridinyl steht,
K für Halogen, Hydroxy, Methoxy oder gegebenenfalls ein- oder mehrfach, gleich oder verschieden mit L substituiertes Morpholinyl, Piperazinyl, Piperidinyl oder Phenoxy steht,
L für C₁-C₃-Alkyl oder -COO-C₃-C₅-Alkyl steht,
R² für gegebenenfalls ein- oder mehrfach, gleich oder verschieden mit M substituiertes Isopropyl, Phenyl, Chinolinyl, Imidazolyl, Indolyl oder Pyridinyl steht und
M für Cyano, Halogen, Hydroxy, Nitro, Methyl, -CF₃ oder für Methoxy steht oder für die Gruppe -CO-C₁-C₃-Alkyl, oder -O-(CH₂)₃-N(Methyl)₂ steht
bedeuten, sowie deren Solvate, Hydrate, Stereoisomere, Diastereomere, Enantiomere und Salze.

8. Verbindungen der allgemeinen Formel I, gemäß Anspruch 7, in der
R¹ für gegebenenfalls ein- oder mehrfach, gleich oder verschieden mit K substituiertes *tert*-Butyl, Cyclopropyl, Cyclohexyl, Cyclohexanon, 1,4-Dioxaspiro[4.5]dec-8-yl, Phenyl, 1,3-Benzodioxolyl oder Pyridinyl steht,
L für Methyl oder -COO-*tert*-Butyl steht,
R² für gegebenenfalls ein- oder mehrfach, gleich oder verschieden mit M substituiertes Isopropyl, Phenyl, Chinolinyl, Imidazolyl, Indolyl oder Pyridinyl steht und
M für Cyano, Halogen, Hydroxy, Nitro, Methyl, -CF₃ oder für Methoxy steht oder für die Gruppe -CO-Methyl oder -O-(CH₂)₃-N(Methyl)₂ steht,
bedeuten, sowie deren Solvate, Hydrate, Stereoisomere, Diastereomere, Enantiomere und Salze.

9. Verwendung der Verbindungen der allgemeinen Formel I, gemäß einem der Ansprüche 1 bis 8, wobei wenn
R¹ für Alkyl, Alkenyl, Aryl, Aralkyl, Cycloalkyl, für ein mit Phenyl substituiertes Alkyl oder p-Methoxyphenyl steht, dann darf R² gleichzeitig auch für Alkyl, Alkenyl, Aryl, Aralkyl, Cyano, heterocyclischer Rest oder Cycloalkyl stehen,
R¹ für einen C₁-C₆-Alkylrest, Alkoxy oder Aryloxy steht, dann darf R² auch gleichzeitig für ein C₁-C₆-Alkylrest stehen,
zur Herstellung eines Arzneimittels.

10. Verwendung der Verbindungen der allgemeinen Formel I, gemäß einem der Ansprüche 1 bis 8 zur Herstellung eines Arzneimittels.

11. Verwendung der Verbindungen der allgemeinen Formel I, gemäß einem der Ansprüche 1 bis 8, in der :
R¹ für gegebenenfalls ein- oder mehrfach, gleich oder verschieden mit K substituiertes C₁-C₆-Alkyl, C₂-C₆-Alkenyl, C₂-C₆-Alkinyl, C₃-C₁₀-Cycloalkyl, C₃-C₁₀-Heterocycloalkyl, Aryl oder Heteroaryl steht, wobei das C₃-C₁₀-Heterocycloalkyl und/oder das Heteroaryl selbst durch mindestens eins der folgenden Atome Stickstoff, Sauerstoff und/oder Schwefel im Ring unterbrochen ist und das C₃-C₁₀-Heterocycloalkyl und/oder Heteroaryl nur über ein Kohlenstoffringatom mit dem Pyrazolopyridin verknüpft sein kann, steht und C₃-C₁₀-Cycloalkyl und/oder C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthalten sein können, oder für ein mit der Gruppe -O-(CH₂)ₙ-O-substituiertes C₃-C₁₀-Cycloalkyl, Aryl, C₃-C₁₀-Heterocycloalkyl oder Heteroaryl steht, wobei die endständigen Sauerstoffatome der -O-(CH₂)ₙ-O- Gruppe mit einem gleichen oder direkt benachbarten C₃-C₁₀-Cycloalkylring-, Arylring-, C₃-C₁₀-Heterocycloalkyring- oder Heteroarylring-Kohlenstoffatom verknüpft sind,
K für Halogen, Hydroxy oder die Gruppe -OR³, COR⁴ oder -NR⁵R⁶ steht oder für gegebenenfalls ein- oder mehrfach, gleich oder verschieden mit L substituiertes C₃-C₁₀-Cycloalkyl, C₃-C₁₀-Heterocycloalkyl, Aryl oder Heteroaryl steht, wobei das C₃-C₁₀-Heterocycloalkyl und/oder das Heteroaryl seibst durch mindestens eins der folgenden Atome Stickstoff, Sauerstoff und/oder Schwefel im Ring unterbrochen ist und C₃-C₁₀-Cycloalkyl und/oder C₃-C₁₀-Heterocycloalkyl gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthalten sein können,
L für C₁-C₆-Alkyl oder die Gruppe -COR⁴, -OR³ oder -NR⁵R⁶ steht,
R² für gegebenenfalls ein- oder mehrfach, gleich oder verschieden mit M substituiertes C₁-C₆-Alkyl, C₃-C₁₀-Cycloalkyl, Aryl oder Heteroaryl steht,
R³ für gegebenenfalls ein- oder mehrfach, gleich oder verschieden mit der Gruppe-NR⁵R⁶ substituiertes C₁-C₆-Alkyl, Aryl oder -(CH₂)ₙ-Aryl steht,
R⁴ für Wasserstoff, Hydroxy, C₁-C₆-Alkyl oder C₁-C₆-Alkoxy steht,
M für Amino, Cyano, Halogen, Hydroxy, Nitro oder für gegebenenfalls ein- oder mehrfach, gleich oder verschieden mit Amino, Cyano, Halogen, Hydroxy, Nitro, C₁-C₆-Alkoxy substituiertes C₁-C₆-Alkyl, C₂-C₆-Alkenyl, C₂-C₆-Alkinyl steht oder für die Gruppe -O-R³, -COR⁴ oder -CO-N-R⁷ steht,
R⁵ und R⁶ unabhängig voneinander für Wasserstoff, C₁-C₆-Alkyl oder für die Gruppe-COR⁴ stehen und
R⁷ Wasserstoff oder NH₂ steht,
n für 1 bis 4 steht,
wobei wenn :
R¹ für Methyl steht, dann darf R² gleichzeitig auch für Methyl, -CH₂-O-CH₃, Phenyl, Chlorphenyl, mit Hydroxy und/oder Methoxy substituiertes Benzofuranyl, -CF₃ oder Furanyl stehen oder wenn
R¹ für -CH₂-O-CH₃ steht, dann darf R² gleichzeitig auch für Methyl stehen oder wenn
R¹ für -CH=CH-Phenyl steht, dann darf R² gleichzeitig auch für Phenyl stehen oder wenn
R¹ für -CH=CH-Chlorphenyl steht, dann darf R² gleichzeitig auch für Phenyl oder Chlorphenyl stehen oder wenn
R¹ für -CH=CH-Methoxyphenyl steht, dann darf R² gleichzeitig auch für Phenyl oder Methoxyphenyl stehen oder wenn
R¹ für Phenyl steht, dann darf R² gleichzeitig auch für -CF₃ , p-Methylphenyl, Methyl, Methoxyphenyl oder Phenyl stehen oder wenn
R¹ für Methoxyphenyl steht, dann darf R² gleichzeitig auch für -CF₃ stehen oder wenn
R¹ für Methylphenyl steht, dann darf R² gleichzeitig auch -CF₃ stehen oder wenn
R¹ für Chlorphenyl steht, dann darf R² gleichzeitig auch für Chlorphenyl oder -CF₃ stehen oder wenn
R¹ für Dichlorphenyl steht, dann darf R² gleichzeitig auch für Trimethoxyphenyl stehen oder wenn
R¹ für Bromphenyl steht, dann darf R² gleichzeitig auch für Trimethoxyphenyl stehen oder wenn
R¹ für Alkyl, Alkenyl, Aryl, Aralkyl, Cycloalkyl, für ein mit Phenyl substituiertes Alkyl oder p-Methoxyphenyl steht, dann darf R² gleichzeitig auch für Alkyl, Alkenyl, Aryl, Aralkyl, heterocyclischer Rest oder Cycloalkyl stehen oder wenn
R¹ für einen C₁-C₆-Alkylrest steht, dann darf R² gleichzeitig auch für ein C₁-C₆-Alkylrest stehen oder wenn
R¹ für Hydroxyphenyl steht, dann darf R² gleichzeitig auch für Heterocyclyl oder mit-COO-*tert*-Butyl substituiertes Heterocycloalkyl stehen oder wenn
R¹ für Benzyloxyphenyl steht, dann darf R² gleichzeitig auch für mit -COO-*tert*-Butyl substitulertes Heterocycloalkyl stehen oder wenn
R¹ für p-Methylphenylsteht, dann darf R² gleichzeitig für Phenyl stehen, oder wenn
R¹ für Cyclopropyl steht, dann darf R² gleichzeitig für p-Methoxyphenyl stehen, oder wenn
R¹ für ein C₃-C₁₀-Heterocycloalkyl oder ein Heteroaryl steht, dann darf das C₃-C₁₀-Heterocycloalkyl und/oder das Heteroaryl über ein Kohlenstoffringatom mit dem Pyrazolopyridin verknüpft sein,
zur Herstellung eines Arzneimittels zur Behandlung von Erkankungen, In denen die Angiogenese, Lymphangiogenese oder Vaskulogenese eine Rolle spielen, Erkrankungen der Blutgefäße, Erkankungen, die durch eine Hyperproliferation von Körperzellen hervorgerufen werden, sowie chronische oder akute neurodegenerative Erkrankungen.

12. Verwendung der Verbindungen der allgemeinen Formel 1, gemäß Anspruch 11, zur Herstellung eines Arzneimittels zur Behandlung von Erkankungen, in denen die Angiogenese, Lymphangiogenese oder Vaskulogenese eine Rolle spielen, Erkankungen, die durch eine Hyperproliferation von Körperzellen hervorgerufen werden, sowie chronische oder akute neurodegenerative Erkrankungen.

13. Verwendung der Verbindungen der allgemeinen Formel I, gemäß einem der Ansprüche 1 bis 8 zur Herstellung eines Arzneimittels zur Behandlung von Erkankungen, in denen die Angiogenese, Lymphangiogenese oder Vaskulogenese eine Rolle spielen, Erkrankungen der Blutgefäße, Erkankungen, die durch eine Hyperproliferation von Körperzellen hervorgerufen werden, sowie chronische oder akute neurodegenerative Erkrankungen.

14. Verwendung der Verbindungen der allgemeinen Formel I, gemäß einem der Ansprüche 1 bis 8, für diagnostische Zwecke in vitro oder in vivo zur Identifizierung von Rezeptoren in Geweben mittels Autoradiographie oder PET.

15. Verwendung der Verbindungen der allgemeinen Formeln (II) und/oder (III) 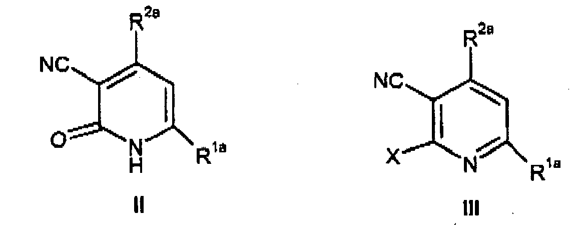 in denen
X für Halogen oder -O-SO₂-CₘF₂ₘ₊₁, steht,
m für 1 bis 4 steht, und
R^{1a} und R^{2a} die gleiche Bedeutung wie R¹ und R² gemäß einem der Ansprüche 1 bis 8 haben, wobei K jedoch zusätzlich auch für die Gruppe -COR⁴ und R³ zusätzlich auch für die Gruppe Trimethylsilyl (TMS), *tert*-Butyl-dimethylsilyl (TBDMS), *tert-*Butyl-diphenylsilyl (TBDPS), Triethylsilyl (TES), C₁-C₂-Alkyl, C₃-C₆-Allyl, Benzyl oder für die Gruppe -COR^{4a} stehen kann,
bedeuten, sowie deren Solvate, Hydrate, Stereoisomere, Diastereomere, Enantiomere und Salze als Zwischenprodukte zur Herstellung von Verbindungen der allgemeinen Formel (I).

16. Arzneimittel, die mindestens eine Verbindungen der allgemeinen Formel I gemäß einem der Ansprüche 1 bis 8 enthalten, wobei wenn
R¹ für Alkyl, Alkenyl, Aryl, Aralkyl, Cycloalkyl, für ein mit Phenyl substituiertes Alkyl oder p-Methoxyphenyl steht, dann darf R² auch gleichzeitig auch für Alkyl, Alkenyl, Aryl, Aralkyl, Cyano, heterocyclischer Rest oder Cycloalkyl stehen,
R¹ für einen C₁-C₆-Alkylrest steht, dann darf R² auch gleichzeitig für ein C₁-C₆-Alkylrest stehen.

17. Arzneimittel, die mindestens eine Verbindungen der allgemeinen Formel I gemäß einem der Ansprüche 1 bis 8 enthalten.

18. Verbindungen gemäß den Ansprüchen 1 bis 8 oder Arzneimittel gemäß den Ansprüchen 16 oder 17 mit geeigneten Formulierungs- und Trägerstoffen.

## Claims

1. Compounds of the general formula (I), 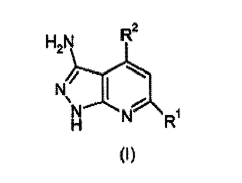 in which,
R¹ represents C₁-C₆-alkyl, C₂-C₆-alkenyl, C₂-C₆-alkinyl, C₃-C₁₀-cycloalkyl, C₃-C₁₀-heterocycloalkyl, aryl or heteroaryl which are optionally mono- or polysubstituted by identical or different K, where the C₃-C₁₀-heterocycloalkyl and/or the heteroaryl for its part is interrupted in the ring by at least one of the following atoms nitrogen, oxygen and/or sulphur and the C₃-C₁₀-heterocycloalkyl and/or heteroaryl may only be attached via a carbon ring atom to the pyrazolopyridine, and C₃-C₁₀-cycloalkyl and/or C₃-C₁₀-heterocycloalkyl may optionally be interrupted in the ring by one or more identical or different -(CO)-, -SO- or -SO₂-groups and may optionally contain one or more double bonds in the ring, or represents C₃-C₁₀-cycloalkyl, aryl, C₃-C₁₀-heterocycloalkyl or heteroaryl which are substituted by the group -O-(CH₂)ₙ-O-, where the terminal oxygen atoms of the-O-(CH₂)ₙ-O- group are attached to an identical or directly adjacent C₃-C₁₀-cycloalkyl ring, aryl ring, C₃-C₁₀-heterocycloalkyl ring or heteroaryl ring carbon atom,
K represents halogen, hydroxy or the group -OR³, COR⁴ or -NR⁵ or represents C₃-C₁₀-cycloalkyl, C₃-C₁₀-heterocycloalkyl, aryl or heteroaryl which are optionally mono- or polysubstituted by identical or different L, where the C₃-C₁₀-heterocycloalkyl and/or the heteroaryl for its part is interrupted in the ring by at least one of the following atoms nitrogen, oxygen and/or sulphur and C₃-C₁₀-cycloalkyl and/or C₃-C₁₀-heterocycloalkyl may optionally be interrupted in the ring by one or more identical different -(CO)-, -SO- or -SO₂-groups and one or more double bonds may optionally be present in the ring,
L represents C₁-C₆-alkyl or the group -COR⁴, -OR³ or -NR⁵R⁶,
R² represents C₁-C₆-alkyl, C₃-C₁₀-cycloalkyl, aryl or heteroaryl which are optionally mono- or polysubstituted by identical or different M,
R³ represents C₁-C₆-alkyl, aryl or -(CH₂)ₙ-aryl which are optionally mono- or polysubstituted by identical or different groups -NR⁵R⁶,
R⁴ represents hydrogen, hydroxy, C₁-C₆-alkyl, or C₁-C₆-alkoxy,
M represents amino, cyano, halogen, hydroxy, nitro or represents C₁-C₆-alkyl, C₂-C₆-alkenyl, C₂-C₆-alkinyl which are optionally mono- or polysubstituted by identical or different substituents from the group consisting of amino, cyano, halogen, hydroxy, nitro, C₁-C₆-alkoxy, or represents the group -O-R³, -COR⁴, or -CO-N-R⁷,
R⁵ and R⁶ independently of one another represent hydrogen, C₁-C₆-alkyl or represent the group -COR⁴ and
R⁷ represents hydrogen or NH₂,
n represents 1 to 4,
with the proviso that, if
R¹ represents methyl, R² may not simultaneously represent -CH₂-O-CH₃, chlorophenyl, hydroxy- and/or methoxy-substituted benzofuranyl, -CF₃ or furanyl, or if
R¹ represents -CH₂-O-CH₃, R² may not simultaneously represent methyl, or if
R¹ represents -CH=CH-phenyl, R² may not simultaneously represent phenyl, or if
R¹ represents -CH=CH-chlorophenyl, R² may not simultaneously represent phenyl or chlorophenyl, or if
R¹ represents -CH=CH-methoxyphenyl, R² may not simultaneously represent phenyl or methoxyphenyl, or if
R¹ represents phenyl, R² may not simultaneously represent -CF₃, p-methylphenyl or methoxyphenyl, or if
R¹ represents methoxyphenyl, R² may not simultaneously represent -CF₃, or if
R¹ represents methylphenyl, R² may not simultaneously represent -CF₃, or if
R¹ represents chlorophenyl, R² may not simultaneously represent chlorophenyl or -CF₃, or if
R¹ represents dichlorophenyl, R² may not simultaneously represent trimethoxyphenyl, or if
R¹ represents bromophenyl, R² may not simultaneously represent trimethoxyphenyl, or if
R¹ represents alkyl, alkenyl, aryl, aralkyl, cycloalkyl, phenyl-substituted alkyl or p-methoxyphenyl, R² may not simultaneously also represent alkyl, alkenyl, aryl, aralkyl, a heterocyclic radical or cycloalkyl, or if
R¹ represents hydroxyphenyl, R² may not simultaneously represent heterocycyl or -COO-*tert*-butyl-substituted heterocycloalkyl, or if
R¹ represents benzyloxyphenyl, R² may not simultaneously represent -COO-*tert-*-butyl-substituted heterocycloalkyl, or if
R¹ represents p-methylphenyl, R² may not simultaneously represent phenyl, or if
R¹ represents cyclopropyl, R² may not simultaneously represent p-methoxyphenyl;
and with the proviso that, if
R¹ represents unsubsituted C₁-C₆-alkyl, aryl, C₃-C₁₀-cycloalkyl or K-substituted C₁-C₆-alkyl and K represents aryl, then
R² represents C₁-C₆-alkyl, C₃-C₁₀-cycloalkyl, aryl or heteroaryl which are mono- or polysubstituted by identical or different M;
or if:
R² represents unsubstituted C₁-C₆-alkyl, C₃-C₁₀-cycloalkyl or aryl, then
R¹ represents C₁-C₆-alkyl, C₃-C₁₀-cycloalkyl, aryl or heteroaryl which are mono- or polysubstituted by identical or different M;
and their solvates, hydrates, stereoisomers, diastereomers, enantiomers and salts.

2. Compounds of the general formula I according to Claim 1 in which,
R¹ represents C₁-C₆-alkyl, C₃-C₁₀-cycloalkyl which are optionally mono- or polysubstituted by identical or different K, where C₃-C₁₀-cycloalkyl is optionally interrupted in the ring by one or more -(CO)-, -SO- or -SO₂-groups and optionally one or more double bonds may be present in the ring, or represents aryl, C₃-C₁₀-heterocycloalkyl or heteroaryl which are optionally mono- or polysubstituted by identical or different K, where C₃-C₁₀-heterocycloalkyl and/or the heteroaryl for its part is interrupted by at least one nitrogen, oxygen and/or sulphur and the C₃-C₁₀-heterocycloalkyl and/or heteroaryl may only be attached via a carbon ring atom to the pyrazolopyridine, or represents C₃-C₁₀-cycloalkyl, aryl, C₃-C₁₀-heterocycloalkyl or heteroaryl which are substituted by the group -O-(CH₂)ₙ-O-, where the terminal oxygen atoms of the -O-(CH₂)ₙ-O-group are attached to an identical or directly adjacent C₃-C₁₀-cycloalkyl ring, aryl ring, C₃-C₁₀-heterocycloalkyl ring or heteroaryl ring carbon atom,
K represents halogen, hydroxy or the group -OR³ or -COR⁴ or represents C₃-C₁₀-cycloalkyl, C₃-C₁₀-heterocycloalkyl, aryl or heteroaryl which are optionally mono- or polysubstituted by identical or different L, where the C₃-C₁₀-heterocycloalkyl and/or the heteroaryl for its part is interrupted in the ring by at least one of the following atoms nitrogen, oxygen and/or sulphur and C₃-C₁₀-cycloalkyl and/or C₃-C₁₀-heterocycloalkyl may optionally be interrupted in the ring by one or more identical or different -(CO)-, -SO- or -SO₂-groups and one or more double bonds may optionally be present in the ring,
R² represents C₁-C₆-alkyl, C₃-C₁₀-cycloalkyl, aryl or heteroaryl which are optionally mono- or polysubstituted by identical or different M, where the heteroaryl is interrupted by at least one nitrogen, oxygen and/or sulphur,
R³ represents C₁-C₆-alkyl, aryl or -(CH₂)ₙ-aryl,
M represents amino, cyano, halogen, hydroxy, nitro or represents C₁-C₆-alkyl which is optionally mono- or polysubstituted by identical or different substituents from the group consisting of amino, cyano, halogen, hydroxy, nitro, C₁-C₆-alkoxy, or represents the group -O-C₁-C₆-alkyl-O-(CH₂)n-NR⁵R⁶, -COR⁴, -O-phenyl, -O-(CH₂)ₙ-phenyl or -CO-N-R⁷,
and their solvates, hydrates, stereoisomers, diastereomers, enantiomers and salts.

3. Compounds of the general formula I according to Claim 2 in which
R¹ represents C₁-C₆-alkyl, C₃-C₁₀-cycloalkyl which are optionally mono- or polysubstituted by identical or different K, where the C₃-C₁₀-cycloalkyl is optionally interrupted in the ring by one or more -(CO)-, -SO- or -SO₂- groups and one or more double bonds may optionally be present in the ring, or represents aryl or heteroaryl which are optionally mono- or polysubstituted by identical or different K, where the heteroaryl for its part is interrupted by at least one nitrogen, oxygen and/or sulphur, and the heteroaryl may only be attached via a carbon ring atom to the pyrazolopyridine, or represents C₃-C₁₀-cycloalkyl, aryl or heteroaryl which are substituted by the group -O-(CH₂)ₙ-O-, where the terminal oxygen atoms of the -O-(CH₂)ₙ-O- group are attached to an identical or directly adjacent C₃-C₁₀-cycloalkyl ring, aryl ring or heteroaryl ring carbon atom,
K represents halogen, hydroxy or the group -OR³ or COR⁴ or represents C₃-C₁₀-cycloalkyl or C₃-C₁₀-heterocycloalkyl which are optionally mono- or polysubstituted by identical or different L, where the C₃-C₁₀-heterocycloalkyl for its part is interrupted in the ring by at least one of the following atoms nitrogen, oxygen and/or sulphur and C₃-C₁₀-cycloalkyl and/or C₃-C₁₀-heterocycloalkyl may optionally be interrupted in the ring by one or more identical or different -(CO)-, -SO- or -SO₂-groups and one or more double bonds may be optionally present in the ring,
L represents C₁-C₆-alkyl or the group -COR⁴ or -OR³,
R³ represents C₁-C₆-alkyl or aryl,
M represents amino, cyano, halogen, hydroxy, nitro or represents C₁-C₆-alkyl which is optionally mono- or polysubstituted by identical or different substituents from the group consisting of amino, cyano, halogen, hydroxy, nitro, C₁-C₆-alkoxy, or represents the group -O-C₁-C₆-alkyl, -O-(CH₂)ₙ-NR⁵R⁶ -CO-C₁-C₆-alkyl, -O-phenyl, -O-(CH₂)ₙ-phenyl or -CO-N-R⁷,
and their solvates, hydrates, stereoisomers, diastereomers, enantiomers and salts.

4. Compounds of the general formula I according to Claim 3 in which
R¹ represents C₁-C₆-alkyl, C₃-C₁₀-cycloalkyl which are optionally mono- or polysubstituted by identical or different K, where the C₃-C₁₀-cycloalkyl is optionally interrupted in the ring by one or more -(CO)- groups, or represents aryl or heteroaryl which are optionally mono- or polysubstituted by identical or different K, where the heteroaryl for its part is interrupted by at least one nitrogen and the heteroaryl may only be attached via a carbon ring atom to the pyrazolopyridine, or represents C₃-C₁₀-cycloalkyl, aryl or heteroaryl which are substituted by the group -O-(CH₂)ₙ-O-, where the terminal oxygen atoms of the -O-(CH₂)ₙ-O- group are attached to an identical or directly adjacent C₃-C₁₀-cycloalkyl ring, aryl ring or heteroaryl ring carbon atom,
K represents halogen, hydroxy or represents the group -OR³, or C₃-C₁₀-heterocycloalkyl which is optionally mono- or polysubstituted by identical or different L, where the C₃-C₁₀heterocycloalkyl for its part is interrupted in the ring by at least one of the following atoms nitrogen and/or oxygen,
L represents C₁-C₆-alkyl or -COO-C₁-C₆-alkyl,
R² represents C₁-C₆-alkyl, C₃-C₁₀-cycloalkyl, aryl or heteroaryl which are optionally mono- or polysubstituted by identical or different M, where the heteroaryl is interrupted by at least one nitrogen,
M represents amino, cyano, halogen, hydroxy, nitro or represents C₁-C₆-alkyl which is optionally mono- or polysubstituted by identical or different substituents from the group consisting of amino, cyano, halogen, hydroxy, nitro and C₁-C₆-alkoxy, or represents the group -O-C₁-C₆-alkyl, -O-(CH₂)ₙ-N(C₁-C₆-alkyl)₂, -CO-C₁-C₆-alkyl, phenoxy, benzyloxy or -CO-N-NH₂,
and their solvates, hydrates, stereoisomers, diastereomers, enantiomers and salts.

5. Compounds of the general formula I according to any of Claims 1 to 4 in which
M represents amino, cyano, halogen, hydroxy, nitro or represents C₁-C₆-alkyl which is optionally mono- or polysubstituted by identical or different substituents from the group consisting of amino, cyano, halogen, hydroxy, nitro and C₁-C₆-alkoxy, or represents the group -O-C₁-C₆-alkyl,-O-(CH₂)ₙ-N(C₁-C₆-alkyl)₂ or -CO-C₁-C₆-alkyl,
and their solvates, hydrates, stereoisomers, diastereomers, enantiomers and salts.

6. Compounds of the general formula I according to Claim 4 in which
R¹ represents C₁-C₆-alkyl, C₃-C₆-cycloalkyl, cyclohexanone, 1, 4-dioxaspiro[4.5]dec-8-yl, phenyl, 1,3-benzodioxolyl or pyridinyl which are optionally mono- or polysubstituted by identical or different K,
K represents halogen, hydroxy, C₁-C₆-alkoxy or represents morpholinyl, piperazinyl, piperindinyl, or phenoxy which are optionally mono- or polysubstituted by identical or different L,
R² represents C₁-C₆-alkyl, phenyl or quinolinyl, imidazolyl, indolyl or pyridinyl which are optionally mono- or polysubstituted by identical or different M,
M represents amino, cyano, halogen, hydroxy, nitro, C₁-C₆-alkyl, -CF₃ or represents C₁-C₃-alkoxy or represents the group -CO-C₁-C₆-alkyl, -O-(CH₂)ₙ-N(C₁-C₆-alkyl)₂, phenoxy or benzyloxy,
and their solvates, hydrates, stereoisomers, diastereomers, enantiomers and salts.

7. Compounds of the general formula I according to Claim 6 in which
R¹ represents C₃-C₆-alkyl, cyclopropyl, cyclohexyl, cyclohexanone, 1,4-dioxaspiro[4.5]dec-8-yl, phenyl, 1,3-benzodioxolyl or pyridinyl which are optionally mono- or polysubstituted by identical or different K,
K represents halogen, hydroxy, methoxy or represents morpholinyl, piperazinyl, piperidinyl or phenoxy which are optionally mono- or polysubstituted by identical or different L,
L represents C₁-C₃-alkyl or -COO-C₃-C₅-alkyl,
R² represents isopropyl, phenyl, quinolinyl, imidazolyl, indolyl or pyridinyl which are optionally mono- or polysubstituted by identical or different M and
M represents cyano, halogen, hydroxy, nitro, methyl, -CF₃ or represents methoxy or represents the group -CO-C₁-C₃-alkyl or -O-(CH₂)₃-N (methyl)₂,
and their solvates, hydrates, stereoisomers, diastereometers, enantiomers and salts.

8. Compounds of the general formula I according to Claim 7 in which
R¹ represents *tert*-butyl, cyclopropyl, cyclohexyl, cyclohexanone, 1,4-dioxaspiro[4.5]dec-8-yl, phenyl, 1,3-benzodioxolyl or pyridinyl, which are optionally mono- or polysubstituted by identical or different K,
L represents methyl or -COO-*tert-*butyl,
R² represents isopropyl, phenyl, quinolinyl, imidazolyl, indolyl or pyridinyl which are optionally mono- or polysubstituted by identical or different M, and
M represents cyano, halogen, hydroxy, nitro, methyl, -CF₃ or represents methoxy or represents the group -CO-methyl or -O-(CH₂)₃-N(methyl)₂,
and their solvates, hydrates, stereoisomers, diastereomers, enantiomers and salts.

9. Use of the compounds of the general formula I according to any of Claims 1 to 8 where, if
R¹ represents alkyl, alkenyl, aryl, aralkyl, cycloalkyl, phenyl-substituted alkyl or p-methoxyphenyl, R² may simultaneously also represent alkyl, alkenyl, aryl, aralkyl, cyano, a heterocyclic radical or cycloalkyl,
R¹ represents a C₁-C₆-alkyl radical, alkoxy or aryloxy, R² may also simultaneously represent a C₁-C₆-alkyl radical,
for preparing a medicament.

10. Use of the compounds of the general formula I according to any of Claims 1 to 8 for preparing a medicament.

11. Use of the compounds of the general formula I according to any of Claims 1 to 8 in which:
R¹ represents C₁-C₆-alkyl, C₂-C₆-alkenyl, C₂-C₆-alkinyl, C₃-C₁₀-cycloalkyl, C₃-C₁₀-heterocycloalkyl, aryl or heteroaryl which are optionally mono- or polysubstituted by identical or different K, where the C₃-C₁₀-heterocycloalkyl and/or the heteroaryl for its part is interrupted in the ring by at least one of the following atoms nitrogen, oxygen and/or sulphur and the C₃-C₁₀-heterocycloalkyl and/or heteroaryl may only be attached via a carbon ring atom to the pyrazolopyridine, and C₃-C₁₀-cycloalkyl and/or C₃-C₁₀heterocycloalkyl may optionally be interrupted in the ring by one or more identical or different -(CO)-, -SO- or -SO₂-groups and optionally one or more double bonds may be present in the ring, or represents C₃-C₁₀-cycloalkyl, aryl, C₃-C₁₀-heterocycloalkyl or heteroaryl which are substituted by the group -O-(CH₂)ₙ-O-, where the terminal oxygen atoms of the -O-(CH₂)ₙ-O- group are attached to an identical or directly adjacent C₃-C₁₀-cycloalkyl ring, aryl ring, C₃-C₁₀-heterocycloalkyl ring or heteroaryl ring carbon atom,
K represents halogen, hydroxy or the group -OR³, COR⁴ or -NR⁵R⁶ or represents C₃-C₁₀-cycloalkyl, C₃-C₁₀-heterocycloalkyl, aryl or heteroaryl which are optionally mono- or polysubstituted by identical or different L, where the C₃-C₁₀-heterocycloalkyl and/or the heteroaryl for its part is interrupted in the ring by at least one of the following atoms nitrogen, oxygen and/or sulphur and C₃-C₁₀-cycloalkyl and/or C₃-C₁₀-heterocycloalkyl may optionally be interrupted in the ring by one or more identical or different -(CO)-, -SO- or -SO₂-groups and one or more double bonds may optionally be present in the ring,
L represents C₁-C₆-alkyl or the group -COR⁴, -OR³ or -NR⁵R⁶,
R² represents C₁-C₆-alkyl, C₃-C₁₀-cycloalkyl, aryl or heteroaryl which are optionally mono- or polysubstituted by identical or different M,
R³ represents C₁-C₆-alkyl, aryl or -(CH₂)ₙ-aryl which are optionally mono- or polysubstituted by identical or different groups -NR⁵R⁶,
R⁴ represents hydrogen, hydroxy, C₁-C₆-alkyl, or C₁-C₆-alkoxy,
M represents amino, cyano, halogen, hydroxy, nitro or represents C₁-C₆-alkyl, C₂-C₆-alkenyl, C₂-C₆-alkinyl which are optionally mono- or polysubstituted by identical or different substituents from the group consisting of amino, cyano, halogen, hydroxy, nitro, C₁-C₆-alkoxy, or represents the group -O-R³, -COR⁴, or -CO-N-R⁷,
R⁵ and R⁶ independently of one another represent hydrogen, C₁-C₆-alkyl or represent the group -COR⁴ and
R⁷ represents hydrogen or NH₂,
n represents 1 to 4,
where, if:
R¹ represents methyl, R² may simultaneously also represent methyl, -CH₂-O-CH₃, phenyl, chlorophenyl, hydroxy-and/or methoxy-substituted benzofuranyl, -CF₃ or furanyl, or if
R¹ represents -CH₂-O-CH₃, R² may simultaneously also represent methyl, or if
R¹ represents -CH=CH-phenyl, R² may simultaneously also represent phenyl, or if
R¹ represents -CH=CH-chlorophenyl, R² may simultaneously also represent phenyl or chlorophenyl, or if
R¹ represents -CH=CH-methoxyphenyl, R² may simultaneously also represent phenyl or methoxyphenyl, or if
R¹ represents phenyl, R² may simultaneously also represent -CF₃, p-methylphenyl, methyl, methoxyphenyl or phenyl, or if
R¹ represents methoxyphenyl, R² may simultaneously also represent -CF₃, or if
R¹ represents methylphenyl, R² may simultaneously also represent -CF₃, or if
R¹ represents chlorophenyl, R² may simultaneously also represent chlorophenyl or -CF₃, or if
R¹ represents dichlorophenyl, R² may simultaneously also represent trimethoxyphenyl, or if
R¹ represents bromophenyl, R² may simultaneously also represent trimethoxyphenyl, or if
R¹ represents alkyl, alkenyl, aryl, aralkyl, cycloalkyl, represents phenyl substituted alkyl or p-methoxyphenyl, R² may simultaneously also represent alkyl, alkenyl, aryl, aralkyl, a heterocyclic radical or cycloalkyl, or if
R¹ represents a C₁-C₆-alkyl radical, R² may simultaneously also represent a C₁-C₆-alkyl radical, or if
R¹ represents hydroxyphenyl, R² may simultaneously also represent heterocyclyl or -COO-*tert-*butyl-substituted heterocycloalkyl, or if
R¹ represents benzyloxyphenyl, R² may simultaneously also represent -COO-*tert-*butyl-substituted heterocycloalkyl, or if
R¹ represents p-methylphenyl, R² may simultaneously also represent phenyl, or if
R¹ represents cyclopropyl, R² may simultaneously also represent p-methoxyphenyl, or if
R¹ represents C₃-C₁₀-heterocycloalkyl or heteroaryl, the C₃-C₁₀-heterocycloalkyl and/or the heteroaryl may be attached via a carbon ring atom to the pyrazolopyridinee,
for preparing a medicament for the treatment of disorders involving angiogenesis, lymphangiogenesis or vasculogenesis, disorders of the blood vessels, disorders caused by hyperproliferation of body cells and chronic or acute neurodegenerative disorders.

12. Use of the compounds of the general formula I according to Claim 11 for preparing a medicament for the treatment of disorders involving angiogenesis, lymphangiogenesis or vasculogenesis, disorders caused by hyperproliferation of body cells and chronic or acute neurodegenerative disorders.

13. Use of the compounds of the general formula I according to any of Claims 1 to 8 for preparing a medicament for the treatment of disorders involving angiogenesis, lymphangiogenesis or vasculogenesis, disorders of the blood vessels, disorders caused by hyperproliferation of body cells and chronic or acute neurodegenerative disorders.

14. Use of the compounds of the general formula I according to any of Claims 1 to 8 for diagnostic purposes in vitro or in vivo for identifying receptors in vessels by autoradiography or PET.

15. Use of the compounds of the general formulae (II) and/or (III) 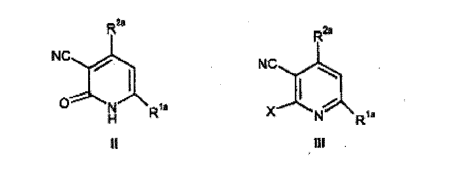 in which
X represents halogen or -O-SO₂-CₘF₂ₘ₊₁,
m represents 1 to 4 and
R^{1a} and R^{2a} have the same meaning as R¹ and R² according to any of Claims 1 to 8, but where K may additionally also represent the group -COR⁴ and R³ may additionally also represent the group trimethylsilyl (TMS), *tert-*butyldimethylsilyl (TBDMS), *tert-*butyldiphenylsilyl (TBDPS), triethylsilyl (TES), C₁-C₂-alkyl, C₃-C₆-allyl, benzyl or the group -COR^{4a},
and their solvates, hydrates, stereoisomers, diastereomers, enantiomers and salts as intermediates for preparing compounds of the general formula (I).

16. Medicaments comprising at least one compound of the general formula I according to any of Claims 1 to 8 where, if
R¹ represents alkyl, alkenyl, aryl, aralkyl, cycloalkyl, phenyl-substituted alkyl or p-methoxyphenyl, R² may simultaneously also represent alkyl, alkenyl, aryl, aralkyl, cyano, a heterocyclic radical or cycloalkyl,
R¹ represents a C₁-C₆-alkyl radical, R² may simultaneously also represent a C₁-C₆-alkyl radical.

17. Medicaments comprising at least one compound of the general formula I according to any of Claims 1 to 8.

18. Compounds according to any of Claims 1 to 8 or medicaments according to Claims 16 or 17 with suitable formulation auxiliaries and carriers.

## Revendications

1. Composés de formule générale (I), 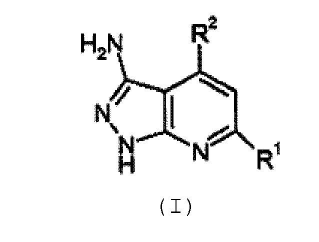 dans laquelle
R¹ représente un groupe alkyle en C₁-C₆, alcényle en C₂-C₆, alcynyle en C₂-C₆, cycloalkyle en C₃-C₁₀, hétérocycloalkyle en C₃-C₁₀, aryle ou hétéroaryle, éventuellement portant un ou plusieurs substituants K identiques ou différents, le groupe hétérocycloalkyle en C₃-C₁₀ et/ou le groupe hétéroaryle pouvant lui-même/eux-mêmes être interrompu(s) dans le cycle par au moins l'un des atomes suivants azote, oxygène et/ou soufre et le groupe hétérocycloalkyle en C₃-C₁₀ et/ou le groupe hétéroaryle ne pouvant être relié(s) à la pyrazolopyridine que par un atome de carbone formant le cycle, et le groupe cycloalkyle en C₃-C₁₀ et/ou le groupe hétérocycloalkyle en C₃-C₁₀ pouvant éventuellement être interrompu(s) dans le cycle par un ou plusieurs groupes -(CO)-, -SO- ou -SO₂-identiques ou différents et une ou plusieurs doubles liaisons pouvant éventuellement être contenues dans le cycle, ou représente un radical cycloalkyle en C₃-C₁₀, aryle, hétérocycloalkyle en C₃-C₁₀ ou hétéroaryle, substitué par le groupe -O-(CH₂)ₙ-O-, les atomes d'oxygène en bout de chaîne du groupe -O-(CH₂)ₙ-O- étant liés à un même atome de carbone ou à deux atomes de carbone contigus du cycle cycloalkyle en C₃-C₁₀, cycle aryle, cycle hétérocycloalkyle en C₃-C₁₀ ou cycle hétéroaryle,
K représente un atome d'halogène, un groupe hydroxy ou le groupe -OR³, COR⁴ ou -NR⁵R⁶ ou représente un groupe cycloalkyle en C₃-C₁₀, hétérocycloalkyle en C₃-C₁₀, aryle ou hétéroaryle, éventuellement portant un ou plusieurs substituants L identiques ou différents, le groupe hétérocycloalkyle en C₃-C₁₀ et/ou le groupe hétéroaryle pouvant lui-même/eux-mêmes être interrompu(s) dans le cycle par au moins l'un des atomes suivants azote, oxygène et/ou soufre et le groupe cycloalkyle en C₃-C₁₀ et/ou le groupe hétérocycloalkyle en C₃-C₁₀ pouvant éventuellement être interrompus(s) dans le cycle par un ou plusieurs groupes -(CO)-, -SO- ou -SO₂- identiques ou différents et une ou plusieurs doubles liaisons pouvant éventuellement être contenues dans le cycle,
L représente un groupe alkyle en C₁-C₆ ou le groupe -COR⁴, -OR³, ou -NR⁵R⁶,
R² représente un groupe alkyle en C₁-C₆, cycloalkyle en C₃-C₁₀, aryle ou hétéroaryle éventuellement portant un ou plusieurs substituants M identiques ou différents,
R³ représente un groupe alkyle en C₁-C₆, aryle ou -(CH₂)ₙ-aryle, éventuellement portant un ou plusieurs substituants NR⁵R⁶ identiques ou
R⁴ différents, représente un atome d'hydrogène, un groupe hydroxy, alkyle en C₁-C₆ ou alcoxy en C₁-C₆,
M représente un atome d'halogène ou un groupe amino, cyano, hydroxy, nitro ou représente un groupe alkyle en C₁-C₆, alcényle en C₂-C₆, alcynyle en C₂-C₆, éventuellement portant un ou plusieurs substituants, identiques ou différents, amino, cyano, halogéno, hydroxy, nitro, alcoxy en C₁-C₆ ou représente le groupe -O-R³, -COR⁴ ou -CO-N-R⁷,
R⁵ et R⁶ représentent indépendamment l'un de l'autre un atome d'hydrogène, un groupe alkyle en C₁-C₆ ou le groupe -COR⁴ et
R⁷ représente un atome d'hydrogène ou NH₂,
n représente 1 à 4,
étant entendu que lorsque
R¹ représente le groupe méthyle, R² ne peut pas représenter en même temps -CH₂-O-CH₃, chlorophényle, benzofuranyle substitué par hydroxy et/ou méthoxy, ni -CF₃ ou furanyle ou lorsque
R¹ représente -CH₂-O-CH₃, R² ne peut pas représenter en même temps méthyle ou lorsque
R¹ représente -CH=CH-phényle, R² ne peut pas représenter en même temps phényle ou lorsque
R¹ représente -CH=CH-chlorophényle, R² ne peut pas représenter en même temps phényle ou chlorophényle ou lorsque
R¹ représente -CH=CH-méthoxyphényle, R² ne peut pas représenter en même temps phényle ou méthoxyphényle ou lorsque
R¹ représente phényle, R² ne peut pas représenter en même temps -CF₃, p-méthylphényle ou méthoxyphényle ou lorsque
R¹ représente méthoxyphényle, R² ne peut pas représenter en même temps -CF₃ ou lorsque
R¹ représente méthylphényle, R² ne peut pas représenter en même temps -CF₃ ou lorsque
R¹ représente chlorophényle, R² ne peut pas représenter en même temps chlorophényle ou -CF₃ ou lorsque
R¹ représente dichlorophényle, R² ne peut pas représenter en même temps triméthoxyphényle ou lorsque
R¹ représente bromophényle, R² ne peut pas représenter en même temps triméthoxyphényle ou lorsque
R¹ représente alkyle, alcényle, aryle, aralkyle, cycloalkyle, représente un groupe p-méthoxy-phényle ou alkyle substitué par phényle, R² ne peut pas représenter en même temps également alkyle, alcényle, aryle, aralkyle, un radical hétérocyclique ou cycloalkyle ou lorsque
R¹ représente hydroxyphényle, R² ne peut pas représenter en même temps hétérocyclyle ni hétérocycloalkyle substitué par COO-tert-butyle ou lorsque
R¹ représente benzyloxyphényle, R² ne peut pas représenter en même temps hétérocycloalkyle substitué par -COO-tert-butyle, ou lorsque
R¹ représente p-méthylphényle, R² ne peut pas représenter en même temps phényle, ou lorsque
R¹ représente cyclopropyle, R² ne peut pas représenter en même temps p-méthoxyphényle ;
et étant entendu que lorsque :
R¹ représente un groupe alkyle en C₁-C₆, aryle, cycloalkyle en C₃-C₁₀, non substitué, ou représente un groupe alkyle en C₁-C₆ substitué par K et K représente un groupe aryle, alors
R² représente un groupe alkyle en C₁-C₆, cycloalkyle en C₃-C₁₀, aryle ou hétéroaryle portant un ou plusieurs substituants M identiques ou différents ;
ou lorsque :
R² représente un groupe alkyle en C₁-C₆, cycloalkyle en C₃-C₁₀ ou aryle, non substitué, alors
R¹ représente un groupe alkyle en C₁-C₆, cycloalkyle en C₃-C₁₀, aryle ou hétéroaryle portant un ou plusieurs substituants M identiques ou différents ;
ainsi que leurs produits de solvatation, hydrates, stéréoisomères, diastéréoisomères, énantiomères et sels.

2. Composés de formule générale I, selon la revendication 1, dans lesquels
R¹ représente un groupe alkyle en C₁-C₆, cycloalkyle en C₃-C₁₀, éventuellement portant un ou plusieurs substituants K identiques ou différents, le groupe cycloalkyle en C₃-C₁₀ pouvant éventuellement être interrompu dans le cycle par un ou plusieurs groupes -(CO)-, -SO- ou -SO₂- et une ou plusieurs doubles liaisons pouvant éventuellement être contenues dans le cycle, ou représente un groupe aryle, hétérocycloalkyle en C₃-C₁₀ ou hétéroaryle éventuellement portant un ou plusieurs substituants K identiques ou différents, le groupe hétérocycloalkyle en C₃-C₁₀ et/ou le groupe hétéroaryle pouvant lui-même/eux-mêmes être interrompu(s) par au moins un atome d'azote, d'oxygène et/ou de soufre et le groupe hétérocycloalkyle en C₃-C₁₀ et/ou le groupe hétéroaryle ne pouvant être relié(s) à la pyrazolopyridine que par un atome de carbone formant le cycle, ou représente un radical cycloalkyle en C₃-C₁₀, aryle, hétérocycloalkyle en C₃-C₁₀ ou hétéroaryle, substitué par le groupe -O-(CH₂)ₙ-O-, les atomes d'oxygène en bout de chaîne du groupe -O-(CH₂)ₙ-O- étant liés à un même atome de carbone ou à deux atomes de carbone contigus du cycle cycloalkyle en C₃-C₁₀, cycle aryle, cycle hétérocycloalkyle en C₃-C₁₀ ou cycle hétéroaryle,
K représente un atome d'halogène, un groupe hydroxy ou le groupe -OR³ ou -COR⁴ ou représente un groupe cycloalkyle en C₃-C₁₀, hétérocycloalkyle en C₃-C₁₀, aryle ou hétéroaryle, éventuellement portant un ou plusieurs substituants L identiques ou différents, le groupe hétérocycloalkyle en C₃-C₁₀ et/ou le groupe hétéroaryle pouvant lui-même/eux-mêmes être interrompu(s) dans le cycle par au moins l'un des atomes suivants azote, oxygène et/ou soufre et le groupe cycloalkyle en C₃-C₁₀ et/ou le groupe hétérocycloalkyle en C₃-C₁₀ pouvant éventuellement être interrompus(s) dans le cycle par un ou plusieurs groupes -(CO)-, -SO- ou -SO₂- identiques ou différents et une ou plusieurs doubles liaisons pouvant éventuellement être contenues dans le cycle,
R² représente un groupe alkyle en C₁-C₆, cycloalkyle en C₃-C₁₀, aryle ou hétéroaryle éventuellement portant un ou plusieurs substituants M identiques ou différents, le groupe hétéroaryle étant interrompu par au moins un atome d'azote, d'oxygène et/ou de soufre,
R³ représente un groupe alkyle en C₁-C₆, aryle ou -(CH₂)ₙ-aryle,
M représente un atome d'halogène ou un groupe amino, cyano, hydroxy, nitro ou représente un groupe alkyle en C₁-C₆ éventuellement portant un ou plusieurs substituants, identiques ou différents, amino, cyano, halogéno, hydroxy, nitro, alcoxy en C₁-C₆ ou représente le groupe -O-alkyle (C₁-C₆), -0- (CH₂)ₙ-NR⁵R⁶, -COR⁴, -0-phényle, -O-(CH₂)ₙ-phényle ou -CO-N-R⁷,
ainsi que leurs produits de solvatation, hydrates, stéréoisomères, diastéréoisomères, énantiomères et sels.

3. Composés de formule générale I, selon la revendication 2, dans lesquels
R¹ représente un groupe alkyle en C₁-C₆, cycloalkyle en C₃-C₁₀, éventuellement portant un ou plusieurs substituants K identiques ou différents, le groupe cycloalkyle en C₃-C₁₀ pouvant éventuellement être interrompu dans le cycle par un ou plusieurs groupes -(CO)-, -SO- ou -SO₂- et une ou plusieurs doubles liaisons pouvant éventuellement être contenues dans le cycle, ou représente un groupe aryle ou hétéroaryle éventuellement portant un ou plusieurs substituants K identiques ou différents, le groupe hétéroaryle pouvant lui-même être interrompu par au moins un atome d'azote, d'oxygène et/ou de soufre et le groupe hétéroaryle ne pouvant être relié à la pyrazolopyridine que par un atome de carbone formant le cycle, ou représente un radical cycloalkyle en C₃-C₁₀, aryle ou hétéroaryle, substitué par le groupe -O-(CH₂)ₙ-O-, les atomes d'oxygène en bout de chaîne du groupe -O-(CH₂)ₙ-O- étant liés à un même atome de carbone ou à deux atomes de carbone contigus du cycle cycloalkyle en C₃-C₁₀, cycle aryle ou cycle hétéroaryle,
K représente un atome d'halogène, un groupe hydroxy ou le groupe -OR³ ou COR⁴ ou représente un groupe cycloalkyle en C₃-C₁₀ ou hétérocycloalkyle en C₃-C₁₀, éventuellement portant un ou plusieurs substituants L identiques ou différents, le groupe hétérocycloalkyle en C₃-C₁₀ pouvant lui-même être interrompu dans le cycle par au moins l'un des atomes suivants azote, oxygène et/ou soufre et le groupe cycloalkyle en C₃-C₁₀ et/ou le groupe hétérocycloalkyle en C₃-C₁₀ pouvant éventuellement être interrompus(s) dans le cycle par un ou plusieurs groupes -(CO)-, -SO- ou -SO₂- identiques ou différents et une ou plusieurs doubles liaisons pouvant éventuellement être contenues dans le cycle,
L représente un groupe alkyle en C₁-C₆ ou un groupe -COR⁴ ou -OR³,
R³ représente un groupe alkyle en C₁-C₆ ou aryle,
M représente un atome d'halogène ou un groupe amino, cyano, hydroxy, nitro ou représente un groupe alkyle en C₁-C₆ qui éventuellement porte un ou plusieurs substituants, identiques ou différents, amino, cyano, halogéno, hydroxy, nitro, alcoxy en C₁-C₆, ou représente le groupe -O-alkyle(C₁-C₆), -O-(CH₂)ₙ-NR⁵R⁶, -CO-alkyle(C₁-C₆), -0-phényle, -O-(CH₂)ₙ-phényle ou -CO-N-R⁷,
ainsi que leurs produits de solvatation, hydrates, stéréoisomères, diastéréoisomères, énantiomères et sels.

4. Composés de formule générale I, selon la revendication 3, dans lesquels
R¹ représente un groupe alkyle en C₁-C₆, cycloalkyle en C₃-C₁₀, éventuellement portant un ou plusieurs substituants K identiques ou différents, le groupe cycloalkyle en C₃-C₁₀ pouvant éventuellement être interrompu dans le cycle par un ou plusieurs groupes -(CO)-, ou représente un groupe aryle ou hétéroaryle éventuellement portant un ou plusieurs substituants K identiques ou différents, le groupe hétéroaryle pouvant lui-même être interrompu par au moins un atome d'azote et le groupe hétéroaryle ne pouvant être relié à la pyrazolopyridine que par un atome de carbone formant le cycle, ou représente un radical cycloalkyle en C₃-C₁₀, aryle ou hétéroaryle, substitué par le groupe -O-(CH₂)ₙ-O-, les atomes d'oxygène en bout de chaîne du groupe -O-(CH₂)ₙ-O- étant liés à un même atome de carbone ou à deux atomes de carbone contigus du cycle cycloalkyle en C₃-C₁₀, cycle aryle ou cycle hétéroaryle,
K représente un atome d'halogène, un groupe hydroxy ou le groupe -OR³ ou représente un groupe hétérocycloalkyle en C₃-C₁₀, éventuellement portant un ou plusieurs substituants L identiques ou différents, le groupe hétérocycloalkyle en C₃-C₁₀ pouvant lui-même être interrompu dans le cycle par au moins l'un des atomes suivants azote et/ou oxygène,
L représente un groupe alkyle en C₁-C₆ ou -COO-alkyle(C₁-C₆),
R² représente un groupe alkyle en C₁-C₆, cycloalkyle en C₃-C₆, aryle ou hétéroaryle éventuellement portant un ou plusieurs substituants M identiques ou différents, le groupe hétéroaryle étant interrompu par au moins un atome d'azote,
M représente un atome d'halogène ou un groupe amino, cyano, hydroxy, nitro ou représente un groupe alkyle en C₁-C₆ qui éventuellement porte un ou plusieurs substituants, identiques ou différents, amino, cyano, halogéno, hydroxy, nitro ou alcoxy en C₁-C₆, ou représente le groupe -O-alkyle(C₁-C₆), -O-(CH₂)ₙ-N(alkyle(C₁-C₆))₂, -CO-alkyle(C₁-C₆), phénoxy, benzyloxy ou -CO-N-NH₂,
ainsi que leurs produits de solvatation, hydrates, stéréoisomères, diastéréoisomères, énantiomères et sels.

5. Composés de formule générale I, selon l'une quelconque des revendications 1 à 4, dans lesquels
M représente un atome d'halogène ou un groupe amino, cyano, hydroxy, nitro ou représente un groupe alkyle en C₁-C₆ qui éventuellement porte un ou plusieurs substituants, identiques ou différents, amino, cyano, halogéno, hydroxy, nitro ou alcoxy en C₁-C₆, ou représente le groupe -O-alkyle(C₁-C₆), -O-(CH₂)ₙ-N(alkyle(C₁-C₆))₂ ou -CO-alkyle(C₁-C₆),
ainsi que leurs produits de solvatation, hydrates, stéréoisomères, diastéréoisomères, énantiomères et sels.

6. Composés de formule générale I, selon la revendication 4, dans lesquels
R¹ représente un groupe alkyle en C₁-C₆, cycloalkyle en C₃-C₆, cyclohexanone, 1,4-dioxaspiro[4.5]déc-8-yle, phényle, 1,3-benzodioxolyle ou pyridinyle, éventuellement portant un ou plusieurs substituants K identiques ou différents,
K représente un atome d'halogène, un groupe hydroxy, alcoxy en C₁-C₆ ou un groupe morpholinyle, pipérazinyle, pipéridinyle ou phénoxy, éventuellement portant un ou plusieurs substituants L identiques ou différents,
R² représente un groupe alkyle en C₁-C₆, phényle ou quinolinyle, imidazolyle, indolyle ou pyridinyle, éventuellement portant un ou plusieurs substituants M identiques ou différents,
M représente un atome d'halogène ou un groupe amino, cyano, hydroxy, nitro, alkyle en C₁-C₆, -CF₃ ou alcoxy en C₁-C₃, ou représente le groupe -CO-alkyle(C₁-C₆), -O-(CH₂)ₙ-N (alkyle(C₁-C₆))₂, phénoxy ou benzyloxy,
ainsi que leurs produits de solvatation, hydrates, stéréoisomères, diastéréoisomères, énantiomères et sels.

7. Composés de formule générale I, selon la revendication 6, dans lesquels
R¹ représente un groupe alkyle en C₃-C₆, cyclopropyle, cyclohexyle, cyclohexanone, 1,4-dioxaspiro[4.5]déc-8-yle, phényle, 1,3-benzodioxolyle ou pyridinyle, éventuellement portant un ou plusieurs substituants K identiques ou différents,
K représente un atome d'halogène, un groupe hydroxy, méthoxy ou un groupe morpholinyle, pipérazinyle, pipéridinyle ou phénoxy, éventuellement portant un ou plusieurs substituants L identiques ou différents,
L représente un groupe alkyle en C₁-C₃ ou -COO-alkyle(C₃-C₅),
R² représente un groupe isopropyle, phényle, quinolinyle, imidazolyle, indolyle ou pyridinyle, éventuellement portant un ou plusieurs substituants M identiques ou différents et
M représente un atome d'halogène ou un groupe cyano, hydroxy, nitro, méthyle, -CF₃ ou méthoxy ou représente le groupe -CO-alkyle(C₁-C₃) ou -O-(CH₂)₃-N(méthyle)₂,
ainsi que leurs produits de solvatation, hydrates, stéréoisomères, diastéréoisomères, énantiomères et sels.

8. Composés de formule générale I, selon la revendication 7, dans lesquels
R¹ représente un groupe tert-butyle, cyclopropyle, cyclohexyle, cyclohexanone, 1,4-dioxaspiro[4.5]déc-8-yle, phényle, 1,3-benzodioxolyle ou pyridinyle, éventuellement portant un ou plusieurs substituants K identiques ou différents,
L représente le groupe méthyle ou -COO-tert-butyle,
R² représente un groupe isopropyle, phényle, quinolinyle, imidazolyle, indolyle ou pyridinyle, éventuellement portant un ou plusieurs substituants M identiques ou différents et
M représente un atome d'halogène ou un groupe cyano, hydroxy, nitro, méthyle, -CF₃ ou méthoxy ou représente le groupe -CO-méthyle ou -O-(CH₂)₃-N(méthyle)₂,
ainsi que leurs produits de solvatation, hydrates, stéréoisomères, diastéréoisomères, énantiomères et sels.

9. Utilisation des composés de formule générale I, selon l'une quelconque des revendications 1 à 8, lorsque
R¹ représente un groupe alkyle, alcényle, aryle, aralkyle, cycloalkyle, représente un groupe p-méthoxyphényle ou alkyle substitué par phényle, R² pouvant alors en même temps représenter également un groupe alkyle, alcényle, aryle, aralkyle, cyano, un radical hétérocyclique ou cycloalkyle,
R¹ représente un radical alkyle en C₁-C₆, alcoxy ou aryloxy, R² pouvant alors en même temps représenter également un radical alkyle en C₁-C₆,
pour la fabrication d'un médicament.

10. Utilisation des composés de formule générale I, selon l'une quelconque des revendications 1 à 8, pour la fabrication d'un médicament.

11. Utilisation des composés de formule générale I, selon l'une quelconque des revendications 1 à 8, dans laquelle :
R¹ représente un groupe alkyle en C₁-C₆, alcényle en C₂-C₆, alcynyle en C₂-C₆, cycloalkyle en C₃-C₁₀, hétérocycloalkyle en C₃-C₁₀, aryle ou hétéroaryle, éventuellement portant un ou plusieurs substituants K identiques ou différents, le groupe hétérocycloalkyle en C₃-C₁₀ et/ou le groupe hétéroaryle pouvant lui-même/eux-mêmes être interrompu(s) dans le cycle par au moins l'un des atomes suivants azote, oxygène et/ou soufre et le groupe hétérocycloalkyle en C₃-C₁₀ et/ou le groupe hétéroaryle ne pouvant être relié(s) à la pyrazolopyridine que par un atome de carbone formant le cycle, et le groupe cycloalkyle en C₃-C₁₀ et/ou le groupe hétérocycloalkyle en C₃-C₁₀ pouvant éventuellement être interrompu(s) dans le cycle par un ou plusieurs groupes -(CO)-, -SO- ou -SO₂-identiques ou différents et une ou plusieurs doubles liaisons pouvant éventuellement être contenues dans le cycle, ou représente un radical cycloalkyle en C₃-C₁₀, aryle, hétérocycloalkyle en C₃-C₁₀ ou hétéroaryle, substitué par le groupe -O-(CH₂)ₙ-O-, les atomes d'oxygène en bout de chaîne du groupe -O-(CH₂)ₙ-O- étant liés à un même atome de carbone ou à deux atomes de carbone contigus du cycle cycloalkyle en C₃-C₁₀, cycle aryle, cycle hétérocycloalkyle en C₃-C₁₀ ou cycle hétéroaryle,
K représente un atome d'halogène, un groupe hydroxy ou le groupe -OR³, COR⁴ ou -NR⁵R⁶ ou représente un groupe cycloalkyle en C₃-C₁₀, hétérocycloalkyle en C₃-C₁₀, aryle ou hétéroaryle, éventuellement portant un ou plusieurs substituants L identiques ou différents, le groupe hétérocycloalkyle en C₃-C₁₀ et/ou le groupe hétéroaryle pouvant lui-même/eux-mêmes être interrompu(s) dans le cycle par au moins l'un des atomes suivants azote, oxygène et/ou soufre et le groupe cycloalkyle en C₃-C₁₀ et/ou le groupe hétérocycloalkyle en C₃-C₁₀ pouvant éventuellement être interrompus(s) dans le cycle par un ou plusieurs groupes -(CO)-, -SO- ou -SO₂- identiques ou différents et une ou plusieurs doubles liaisons pouvant éventuellement être contenues dans le cycle,
L représente un groupe alkyle en C₁-C₆ ou le groupe -COR⁴, -OR³, ou -NR⁵R⁶,
R² représente un groupe alkyle en C₁-C₆, cycloalkyle en C₃-C₁₀, aryle ou hétéroaryle éventuellement portant un ou plusieurs substituants M identiques ou différents,
R³ représente un groupe alkyle en C₁-C₆, aryle ou -(CH₂)ₙ-aryle, éventuellement portant un ou plusieurs substituants NR⁵R⁶ identiques ou différents,
R⁴ représente un atome d'hydrogène, un groupe hydroxy, alkyle en C₁-C₆ ou alcoxy en C₁-C₆,
M représente un atome d'halogène ou un groupe amino, cyano, hydroxy, nitro ou représente un groupe alkyle en C₁-C₆, alcényle en C₂-C₆, alcynyle en C₂-C₆, éventuellement portant un ou plusieurs substituants, identiques ou différents, amino, cyano, halogéno, hydroxy, nitro, alcoxy en C₁-C₆ ou représente le groupe -O-R³, -COR⁴ ou -CO-N-R⁷,
R⁵ et R⁶ représentent indépendamment l'un de l'autre un atome d'hydrogène, un groupe alkyle en C₁-C₆ ou le groupe -COR⁴ et
R⁷ représente un atome d'hydrogène ou NH₂,
n représente 1 à 4,
lorsque :
R¹ représente le groupe méthyle, R² pouvant alors représenter en même temps également méthyle, -CH₂-O-CH₃, phényle, chlorophényle, benzofuranyle substitué par hydroxy et/ou méthoxy, ou bien -CF₃ ou furanyle ou lorsque
R¹ représente -CH₂-O-CH₃, R² pouvant représenter en même temps également méthyle ou lorsque
R¹ représente -CH=CH-phényle, R² pouvant alors représenter en même temps également phényle ou lorsque
R¹ représente -CH=CH-chlorophényle, R² pouvant alors représenter en même temps également phényle ou chlorophényle ou lorsque
R¹ représente -CH=CH-méthoxyphényle, R² pouvant alors représenter en même temps également phényle ou méthoxyphényle ou lorsque
R¹ représente phényle, R² pouvant alors représenter en même temps également -CF₃, p-méthylphényle, méthyle, méthoxyphényle ou phényle ou lorsque
R¹ représente méthoxyphényle, R² pouvant alors représenter en même temps également -CF₃ ou lorsque
R¹ représente méthylphényle, R² pouvant alors représenter en même temps également -CF₃ ou lorsque
R¹ représente chlorophényle, R² pouvant alors représenter en même temps également chlorophényle ou -CF₃ ou lorsque
R¹ représente dichlorophényle, R² pouvant alors représenter en même temps également triméthoxyphényle ou lorsque
R¹ représente bromophényle, R² pouvant alors représenter en même temps également triméthoxyphényle ou lorsque
R¹ représente alkyle, alcényle, aryle, aralkyle, cycloalkyle, représente un groupe p-méthoxy-phényle ou alkyle substitué par phényle, R² pouvant alors représenter en même temps également alkyle, alcényle, aryle, aralkyle, un radical hétérocyclique ou cycloalkyle ou lorsque
R¹ représente un radical alkyle en C₁-C₆, R² pouvant alors représenter également un radical alkyle en C₁-C₆ ou lorsque
R¹ représente hydroxyphényle, R² pouvant alors représenter en même temps également hétérocyclyle ou bien hétérocycloalkyle substitué par COO-tert-butyle ou lorsque
R¹ représente benzyloxyphényle, R² pouvant alors représenter en même temps également hétérocycloalkyle substitué par -COO-tert-butyle, ou lorsque
R¹ représente le radical p-méthylphényle, R² pouvant alors représenter en même temps également phényle, ou lorsque
R¹ représente cyclopropyle, R² pouvant alors représenter en même temps également p-méthoxyphényle ou lorsque
R¹ représente un radical hétérocycloalkyle en C₃-C₁₀ ou un radical hétéroaryle, le radical hétérocycloalkyle en C₃-C₁₀ et/ou le radical hétéroaryle pouvant alors être reliés à la pyrazolopyridine par un atome de carbone formant le cycle,
pour la fabrication d'un médicament destiné au traitement de maladies dans lesquelles l'angiogenèse, la lymphangiogenèse ou la vasculogenèse jouent un rôle, de maladies des vaisseaux sanguins, de maladies qui sont provoquées par une hyperprolifération de cellules corporelles, ainsi que de maladies neurodégénératives chroniques ou aiguës.

12. Utilisation des composés de formule générale I, selon la revendication 11, pour la fabrication d'un médicament destiné au traitement de maladies dans lesquelles l'angiogenèse, la lymphangiogenèse ou la vasculogenèse jouent un rôle, de maladies qui sont provoquées par une hyperprolifération de cellules corporelles, ainsi que de maladies neurodégénératives chroniques ou aiguës.

13. Utilisation des composés de formule générale I, selon l'une quelconque des revendications 1 à 8, pour la fabrication d'un médicament destiné au traitement de maladies dans lesquelles l'angiogenèse, la lymphangiogenèse ou la vasculogenèse jouent un rôle, de maladies des vaisseaux sanguins, de maladies qui sont provoquées par une hyperprolifération de cellules corporelles, ainsi que de maladies neurodégénératives chroniques ou aiguës.

14. Utilisation des composés de formule générale I, selon l'une quelconque des revendications 1 à 8, à des fins de diagnostic in vitro ou in vivo pour l'identification de récepteurs dans des tissus par autoradiographie ou PET.

15. Utilisation des composés de formules générales (II) et/ou (III) 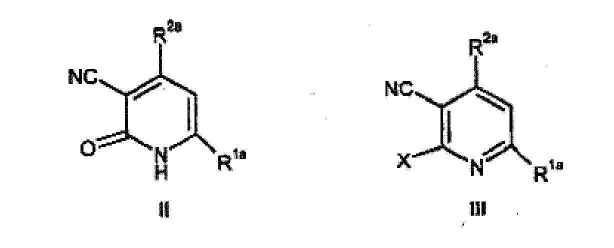 dans lesquelles
X représente un atome d'halogène ou -O-SO₂-CₘF₂ₘ₊₁,
m représente 1 à 4, et
R^{1a} et R^{1b} ont la même signification que R¹ et R² selon l'une quelconque des revendications 1 à 8, K pouvant toutefois également représenter le groupe -COR⁴ et R³ représenter en outre le groupe triméthylsilyle (TMS), tert-butyl-diméthylsilyle (TBDMS), tert-butyl-diphénylsilyle (TBDPS), triéthylsilyle (TES), alkyle en C₁-C₂, allyle en C₃-C₆, benzyle ou représenter le groupe -COR^{4a},
ainsi que de leurs produits de solvatation, hydrates, stéréoisomères, diastéréoisomères, énantiomères et sels, en tant que produits intermédiaires pour la préparation des composés de formule générale (I).

16. Médicaments, qui contiennent au moins un composé de formule générale I selon l'une quelconque des revendications 1 à 8, lorsque
R¹ représente un groupe alkyle, alcényle, aryle, aralkyle, cycloalkyle, représente un groupe p-méthoxyphényle ou alkyle substitué par phényle, R² pouvant alors en même temps représenter également un groupe alkyle, alcényle, aryle, aralkyle, cyano, un radical hétérocyclique ou cycloalkyle,
R¹ représente un radical alkyle en C₁-C₆, R² pouvant alors en même temps représenter également un radical alkyle en C₁-C₆.

17. Médicaments, qui contiennent au moins un composé de formule générale I selon l'une quelconque des revendications 1 à 8.

18. Composés selon les revendications 1 à 8 ou médicaments selon la revendication 16 ou 17, avec des véhicules et adjuvants de formulation appropriés
